# EUROPEAN PATENT APPLICATION

(11) **EP 4 656 628 A1**
(43) Date of publication of application: **03.12.2025**
(21) Application number: 24747366.3
(22) Date of filing: 26.01.2024
(51) Int. Cl.: C07C 271/12, A61K 9/127, A61K 31/713, A61K 31/7088, A61K 38/02, A61K 45/00, A61K 47/18, A61K 47/28, A61K 47/69, A61P 43/00, C07C 271/16, C07C 271/22

(54) **COMPOUND OR SALT THEREOF, LIPID COMPOSITION, PHARMACEUTICAL COMPOSITION, AND DELIVERY CARRIER**

(30) Priority: 27.01.2023 JP 2023010661
(71) Applicant: FUJIFILM Corporation, Tokyo 106-8620 (JP)
(72) Inventor: NITABARU, Tatsuya, Ashigarakami-gun, Kanagawa 258-8577 (JP); TANABE, Shintaro, Ashigarakami-gun, Kanagawa 258-8577 (JP); YAMAMOTO, Masahiko, Ashigarakami-gun, Kanagawa 258-8577 (JP); FUKUNAGA, Hirofumi, Ashigarakami-gun, Kanagawa 258-8577 (JP); NAKAMURA, Naoto, Ashigarakami-gun, Kanagawa 258-8577 (JP)
(74) Representative: Klunker IP Patentanwälte PartG mbB
(86) International application number: PCT/JP2024/002320
(87) International publication number: WO 2024/158042

(57) **Abstract**

An object of the present invention is to provide a compound or a salt thereof constituting lipid composition that can achieve a high nucleic acid encapsulation rate and excellent delivery of nucleic acids, and to provide a lipid composition, a pharmaceutical composition, and a delivery carrier, using the compound or a salt thereof. According to the present invention, a compound represented by Formula (1) or a salt thereof is provided.

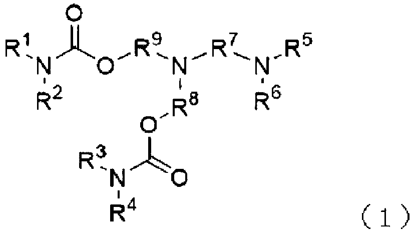

In the formula, R¹, R², R³, and R⁴ each independently represent a hydrogen atom or a hydrocarbon group having 1 to 24 carbon atoms, which may be substituted, R⁵ and R⁶ each independently represent a hydrocarbon group having 1 to 18 carbon atoms, which may be substituted, R⁷, R⁸, and R⁹ each independently represent a hydrocarbon group having 2 to 8 carbon atoms, and R⁵ and R⁶, or R⁵ and R⁷, may be combined to form a 4- to 7-membered ring.

## Description

### Technical Field

The present invention relates to a compound or a salt thereof, which facilitates the introduction of a nucleic acid into a cell or the like. The present invention further relates to a lipid composition, a pharmaceutical composition, and a delivery carrier, which contain the above-described compound or a salt thereof.

### Background Art

Nucleic acid drugs have a clear mechanism of action on diseases, have few side effects, and are regarded as promising next-generation medicines. For example, a nucleic acid drug using small interfering RNA (siRNA) can inhibit the expression of a target gene in a cell in a sequence-specific manner. As a result, the nucleic acid drug can relieve or treat the diseases and symptoms caused by the abnormal expression of a specific gene or gene group. To express the function of these nucleic acids, the nucleic acid drugs need to be delivered into cells.

One of the methods for efficiently delivering nucleic acids into cells is a method using a viral vector such as a retrovirus or an adenovirus. The method using a viral vector brings a high gene introduction efficiency. However, the size of genes to be introduced by this method is limited, and there is a concern over immunogenicity and safety of this method. On the other hand, in a case where a lipid composition is used for gene introduction, any gene can be introduced without limitation, and the above issues can be solved. Therefore, the lipid composition is being developed vigorously.

Patent Document 1 discloses a compound having an ester group as a linking group that links an aliphatic group and an amino group, as a compound to be contained in a lipid composition. Patent Document 1 describes delivering a nucleic acid to mouse cells using a lipid composition containing the above-described compound.

Patent Document 2 describes an amino lipid represented by a predetermined formula (1) or a salt thereof, and also describes a lipid composition containing the amino lipid or a salt thereof. Patent Document 2 describes delivering a nucleic acid to mouse cells using the above-described lipid composition.

Patent Document 3 describes a lipid composition containing an amino lipid represented by a predetermined formula (1) or a salt thereof, a nonionic lipid, a lipid having a nonionic hydrophilic polymer structure, and a nucleic acid, and containing or not containing a zwitterionic lipid. Patent Document 3 describes delivering a nucleic acid to mouse cells using the above-described lipid composition.

### Prior Art Documents

### Patent Documents

Patent Document 1: WO2010/054401A
Patent Document 2: WO2019/235635A
Patent Document 3: WO2020/246581A

### SUMMARY OF THE INVENTION

### Object to be solved by the invention

The lipid composition that can function as vectors and the compounds that constitute the lipid composition are further sought for, and there is a demand for the development of compounds that can achieve excellent delivery of nucleic acids.

The present invention has been made under such circumstances, and an object to be solved is to provide a compound or a salt thereof constituting lipid composition that can achieve a high nucleic acid encapsulation rate and excellent delivery of nucleic acids. Furthermore, an object to be solved of the present invention is to provide a lipid composition, a pharmaceutical composition, and a delivery carrier, which can achieve a high nucleic acid encapsulation rate and excellent delivery of nucleic acids, using the compound or a salt thereof. Means for solving the object

To achieve the above object, the inventors of the present invention have conducted intensive studies. As a result, the inventors have found that lipid composition prepared using a compound represented by Formula (1) or a salt thereof exhibits a high nucleic acid encapsulation rate and excellent delivery of nucleic acids. Based on the finding, the inventors have accomplished the present invention. According to the present invention, the following inventions are provided.
<1> A compound represented by Formula (1) or a salt thereof, 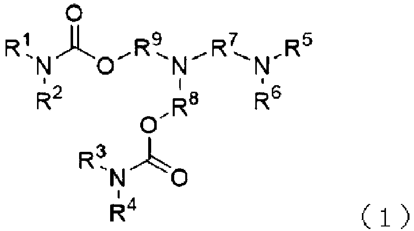
   in the formula, R¹, R², R³, and R⁴ each independently represent a hydrogen atom or a hydrocarbon group having 1 to 24 carbon atoms, which may be substituted,
   substituents on the hydrocarbon groups having 1 to 24 carbon atoms represented by R¹, R², R³, and R⁴, which may be substituted, each independently represent -C(O)O-R¹¹, - OC(O)-R¹², -O-R¹³, -CO-R¹⁴, -OC(O)O-R¹⁵, or -S-S-R¹⁶,
   R¹¹, R¹², R¹³, R¹⁴, R¹⁵, and R¹⁶ each independently represent a hydrocarbon group having 1 to 24 carbon atoms which may be substituted with -S-R¹⁷, where R¹⁷ represents a hydrocarbon group having 1 to 12 carbon atoms,
   R⁵ and R⁶ each independently represent a hydrocarbon group having 1 to 18 carbon atoms which may be substituted,
   substituents on the hydrocarbon groups having 1 to 18 carbon atoms represented by R⁵ and R⁶, which may be substituted, each independently represent -OH, -COOH, -NR²¹R²², - OC(O)O-R²³, -C(O)O-R²⁴, -OC(O)-R²⁵, -O-R²⁶, -C(O)NR²⁷R²⁸, -NR²⁹C(O)R³⁰, - N(R³¹)S(O)₂R³², -N(R³³)C(O)N(R³⁴)R³⁵, -N(R³⁶)C(S)N(R³⁷)R³⁸, -OC(O)N(R³⁹)R⁴⁰, or - N(R⁴¹)C(O)OR⁴²,
   R²¹ and R²² each independently represent a hydrogen atom or a hydrocarbon group having 1 to 8 carbon atoms,
   R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴² each independently represent a hydrogen atom or a hydrocarbon group having 1 to 24 carbon atoms, which may be substituted, and substituents on the hydrocarbon groups having 1 to 24 carbon atoms represented by R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴², which may be substituted, each independently represent an aryl group having 6 to 20 carbon atoms, a heterocyclic group, -OH, -COOH, or -NR⁵¹R⁵², where R⁵¹ and R⁵² each independently represent a hydrogen atom or a hydrocarbon group having 1 to 8 carbon atoms,
   R⁷, R⁸, and R⁹ each independently represent a hydrocarbon group having 2 to 8 carbon atoms, and
   R⁵ and R⁶, or R⁵ and R⁷, may be combined to form a 4- to 7-membered ring.
<2> The compound or a salt thereof according to <1>, in which R¹ represents -R^{1a}-L¹-R^{1b}, R^{1a} represents a hydrocarbon group having 1 to 18 carbon atoms, L¹ represents -C(O)O-, - OC(O)-, -OC(O)O-, or -S-S-, and R^{1b} represents a hydrocarbon group having 1 to 18 carbon atoms,
   R³ represents -R^{3a}-L³-R^{3b}, R^{3a} represents a hydrocarbon group having 1 to 18 carbon atoms, L³ represents -C(O)O-, -OC(O)-, -OC(O)O-, or -S-S-, and R^{3b} represents a hydrocarbon group having 1 to 18 carbon atoms,
   R² and R⁴ each independently represent a hydrocarbon group having 1 to 18 carbon atoms, which may be substituted, and substituents on the hydrocarbon groups having 1 to 18 carbon atoms represented by R² and R⁴, which may be substituted, each independently represent -C(O)O-R¹¹, -OC(O)-R¹², -O-R¹³, -CO-R¹⁴, -OC(O)O-R¹⁵, or -S-S-R¹⁶,
   R¹¹, R¹², R¹³, R¹⁴, R¹⁵, and R¹⁶ each independently represent a hydrocarbon group having 1 to 18 carbon atoms,
   R⁵ and R⁶ each independently represent a hydrocarbon group having 1 to 12 carbon atoms, which may be substituted,
   substituents on the hydrocarbon groups having 1 to 12 carbon atoms represented by R⁵ and R⁶, which may be substituted, each independently represent -OH, -O-R²⁶, -C(O)NR²¹R²¹ or -NR²⁹C(O)R³⁰,
   R²⁶, R²⁷, R²⁸, R²⁹, and R³⁰ each independently represent a hydrogen atom or a hydrocarbon group having 1 to 12 carbon atoms, which may be substituted, and substituents on the hydrocarbon groups having 1 to 12 carbon atoms represented by R²⁶, R²⁷, R²⁸, R²⁹, and R³⁰, which may be substituted, each independently represent an aryl group having 6 to 10 carbon atoms or a heterocyclic group, and
   R⁷, R⁸, and R⁹ each independently represent -(CH₂)ₙ-, and n represents an integer of 2 to 8.
<3> The compound or a salt thereof according to <1>, in which R¹ represents -R^{1a}-L¹-R^{1b}, R^{1a} represents a hydrocarbon group having 1 to 18 carbon atoms, L¹ represents -C(O)O- or -OC(O)-, and R^{1b} represents a hydrocarbon group having 1 to 18 carbon atoms,
   R³ represents -R^{3a}-L³-R^{3b}, R^{3a} represents a hydrocarbon group having 1 to 18 carbon atoms, L³ represents -C(O)O- or -OC(O)-, and R^{3b} represents a hydrocarbon group having 1 to 18 carbon atoms,
   R² and R⁴ each independently represent a hydrocarbon group having 1 to 10 carbon atoms,
   R⁵ and R⁶ each independently represent a hydrocarbon group having 1 to 6 carbon atoms, which may be substituted,
   substituents on the hydrocarbon groups having 1 to 6 carbon atoms represented by R⁵ and R⁶, which may be substituted, each independently represent -OH, -O-R²⁶, -C(O)NR²⁷R²⁸, or -NR²⁹C(O)R³⁰,
   R²⁶, R²⁷, R²⁸, R²⁹, and R³⁰ each independently represent a hydrogen atom or a hydrocarbon group having 1 to 12 carbon atoms, which may be substituted, and substituents on the hydrocarbon groups having 1 to 12 carbon atoms represented by R²⁶, R²⁷, R²⁸, R²⁹, and R³⁰, which may be substituted, each independently represent an aryl group having 6 to 10 carbon atoms, and
   R⁷, R⁸, and R⁹ each independently represent -(CH₂)ₙ-, and n represents an integer of 2 to 8.
<4> A compound or a salt thereof selected from the following compounds:
   ((2-(diethylamino)ethyl)azanediyl)bis(ethan-2,1-diyl)bis(dioctylcarbamate); 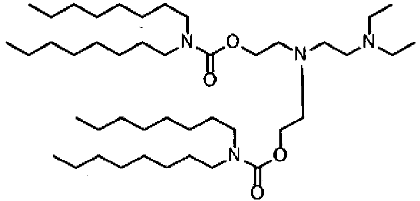
   bis(2-hexyloctyl) 11-(2-(diethylamino)ethyl)-6,16-dioctyl-7,15-dioxo-8,14-dioxa-6,11,16-triazahenicosanedioate; 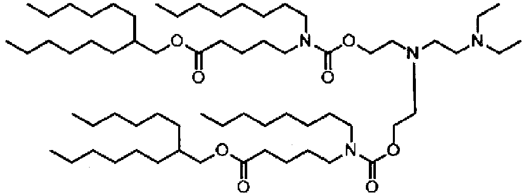
   bis(2-pentylheptyl) 11-(2-(diethylamino)ethyl)-6,16-dioctyl-7,15-dioxo-8,14-dioxa-6,11,16-triazahenicosanedioate; 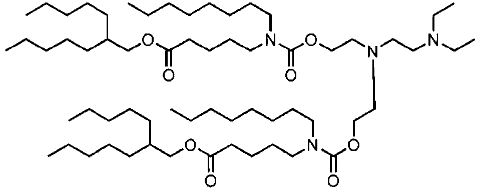
   bis(2-pentylheptyl) 11-(2-(diethylamino)ethyl)-6,16-dihexyl-7,15-dioxo-8,14-dioxa-6,11,16-triazahenicosanedioate; 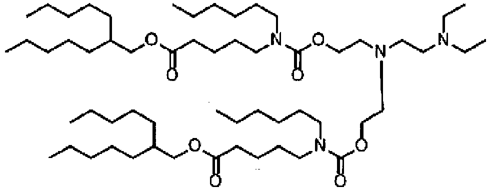
   bis(2-hexyloctyl) 11-(2-(diethylamino)ethyl)-6,16-dihexyl-7,15-dioxo-8,14-dioxa-6,11,16-triazahenicosanedioate; 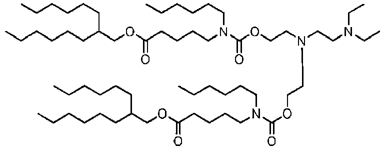
   bis(2-pentylheptyl) 4,14-dibutyl-9-(2-(diethylamino)ethyl)-5,13-dioxo-6,12-dioxa-4,9,14-triazaheptadecanedioate; 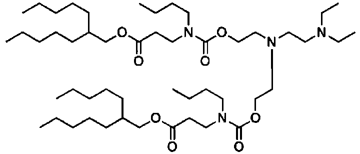
   bis(2-pentylheptyl) 4,14-dibutyl-9-(3-(diethylamino)propyl)-5,13-dioxo-6,12-dioxa-4,9,14-triazaheptadecanedioate; 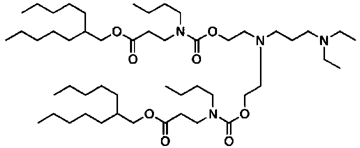
   bis(2-pentylheptyl) 9-(2-(diethylamino)ethyl)-5,13-dioxo-4,14-dipropyl-6,12-dioxa-4,9,14-triazaheptadecanedioate; 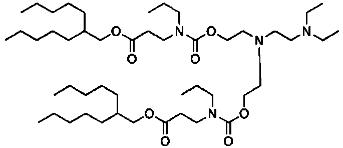
   bis(2-pentylheptyl) 9-(3-(diethylamino)propyl)-5,13-dioxo-4,14-dipropyl-6,12-dioxa-4,9,14-triazaheptadecanedioate; 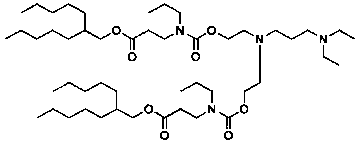
   bis(2-pentylheptyl) 8-(2-(diethylamino)ethyl)-4,12-dioxo-3,13-dipropyl-5,11-dioxa-3,8,13-triazapentadecanedioate; 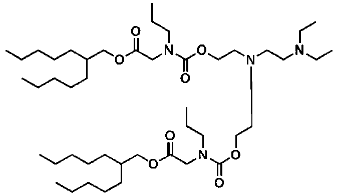
   bis(2-pentylheptyl) 8-(3-(diethylamino)propyl)-4,12-dioxo-3,13-dipropyl-5,11-dioxa-3,8,13-triazapentadecanedioate; 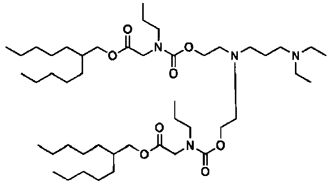
   2-(2-(2-(bis(2-decanoyloxyethyl)carbamoyloxy)ethyl-(2-(diethylamino)ethyl)amino)ethoxycarbonyl-(2-decanoyloxyethyl)amino)ethyl decanoate; 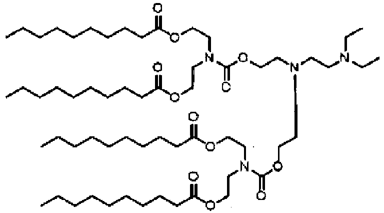
   bis(2-pentylheptyl) 11-(2-(diethylamino)ethyl)-6,16-diisopropyl-7,15-dioxo-8,14-dioxa-6,11,16-triazahenicosanedioate; 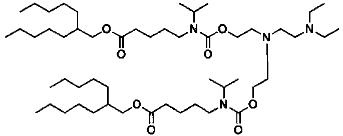
   bis(2-pentylheptyl) 11-(3-(diethylamino)propyl)-6,16-diisopropyl-7,15-dioxo-8,14-dioxa-6,11,16-triazahenicosanedioate; 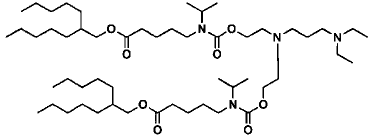
   bis(2-pentylheptyl) 11-(2-(diethylamino)ethyl)-7,15-dioxo-6,16-dipropyl-8,14-dioxa-6,11,16-triazahenicosanedioate; 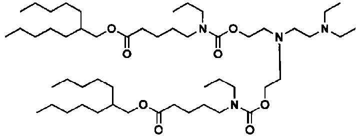
   bis(2-pentylheptyl) 11-(3-(diethylamino)propyl)-7,15-dioxo-6,16-dipropyl-8,14-dioxa-6,11,16-triazahenicosanedioate; 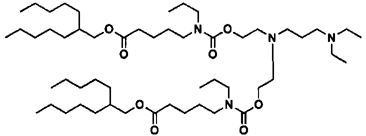
   bis(2-butyloctyl) 11-(2-(diethylamino)ethyl)-6,16-dihexyl-7,15-dioxo-8,14-dioxa-6,11,16-triazahenicosanedioate; 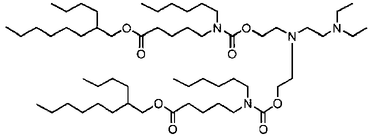
   bis(2-pentylheptyl) 11-(2-(diethylamino)ethyl)-6,16-diisobutyl-7,15-dioxo-8,14-dioxa-6,11,16-triazahenicosanedioate; 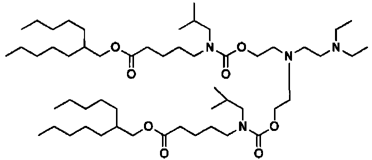
   bis(2-pentylheptyl) 11-(3-(diethylamino)propyl)-6,16-diisobutyl-7,15-dioxo-8,14-dioxa-6,11,16-triazahenicosanedioate; 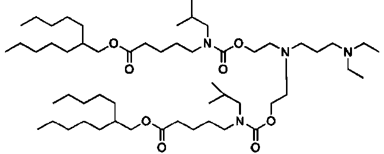
   2-(2-(2-(bis(2-dodecanoyloxyethyl)carbamoyloxy)ethyl-(2-(diethylamino)ethyl)amino)ethoxycarbonyl-(2-dodecanoyloxyethyl)amino)ethyl dodecanoate; 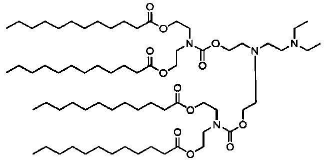
   bis(2-pentylheptyl) 6,16-dibutyl-11-(2-(diethylamino)ethyl)-7,15-dioxo-8,14-dioxa-6,11,16-triazahenicosanedioate; 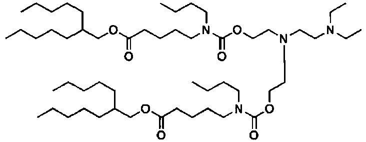
   bis(2-pentylheptyl) 6,16-dibutyl-11-(3-(diethylamino)propyl)-7,15-dioxo-8,14-dioxa-6,11,16-triazahenicosanedioate; 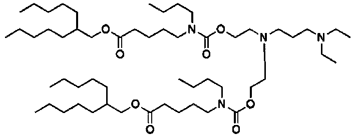
   bis(2-hexyloctyl) 6,16-dibutyl-11-(2-(diethylamino)ethyl)-7,15-dioxo-8,14-dioxa-6,11,16-triazahenicosanedioate; 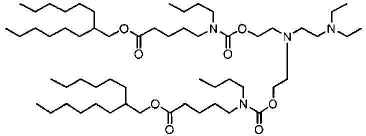
   bis(2-hexyloctyl) 6,16-dibutyl-11-(3-(diethylamino)propyl)-7,15-dioxo-8,14-dioxa-6,11,16-triazahenicosanedioate; 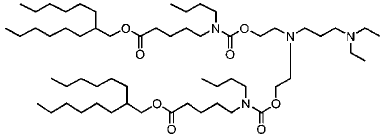
   bis(2-pentylheptyl) 12-(2-(diethylamino)ethyl)-7,17-dihexyl-8,16-dioxo-9,15-dioxa-7,12,17-triazatricosanedioate; 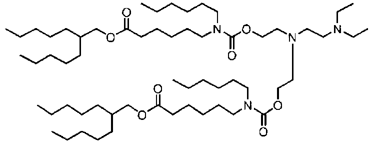
   bis(2-pentylheptyl) 12-(3-(diethylamino)propyl)-7,17-dihexyl-8,16-dioxo-9,15-dioxa-7,12,17-triazatricosanedioate; 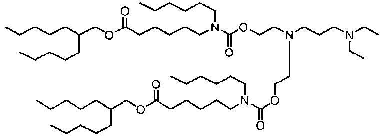
   decyl 2-(2-(2-(bis(2-decoxy-2-oxo-ethyl)carbamoyloxy)ethyl-(2-(diethylamino)ethyl)amino)ethoxycarbonyl-(2-decoxy-2-oxo-ethyl)amino)acetate; 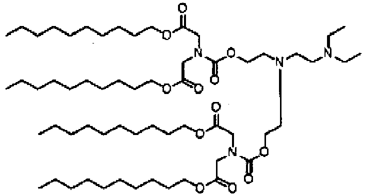
   bis(2-pentylheptyl) 11-(2-(diethylamino)ethyl)-7,15-dioxo-6,16-dipentyl-8,14-dioxa-6,11,16-triazahenicosanedioate; 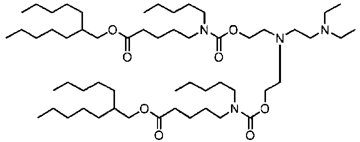
   bis(2-pentylheptyl) 11-(3-(diethylamino)propyl)-7,15-dioxo-6,16-dipentyl-8,14-dioxa-6,11,16-triazahenicosanedioate; 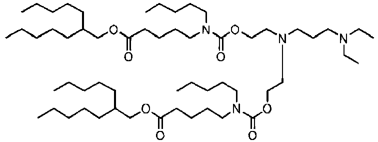
   bis(2-pentylheptyl) 11-(2-(diethylamino)ethyl)-6,16-diheptyl-7,15-dioxo-8,14-dioxa-6,11,16-triazahenicosanedioate; 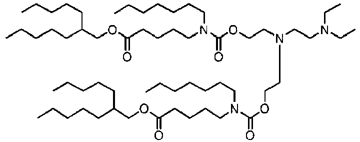
   bis(2-pentylheptyl) 11-(3-(diethylamino)propyl)-6,16-diheptyl-7,15-dioxo-8,14-dioxa-6,11,16-triazahenicosanedioate; 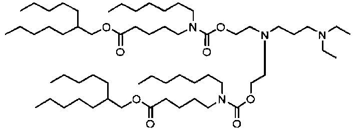
   didodecyl 8-(2-(diethylamino)ethyl)-3,13-bis(2-(dodecyloxy)-2-oxoethyl)-4,12-dioxo-5,11-dioxa-3,8,13-triazapentadecanedioate; 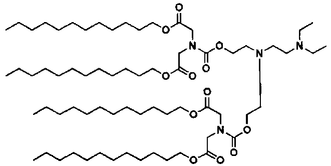
   diundecyl 8-(2-(diethylamino)ethyl)-4,12-dioxo-3,13-bis(2-oxo-2-(undecyloxy)ethyl)-5,11-dioxa-3,8,13-triazapentadecanedioate; 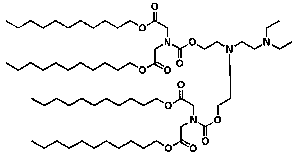
   ditridecyl 8-(2-(diethylamino)ethyl)-4,12-dioxo-3,13-bis(2-oxo-2-(tridecyloxy)ethyl)-5,11-dioxa-3,8,13-triazapentadecanedioate; 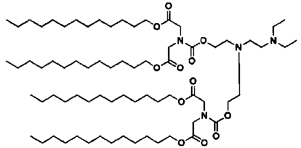
   bis(2-pentylheptyl) 11-(3-(diethylamino)propyl)-6,16-dioctyl-7,15-dioxo-8,14-dioxa-6,11,16-triazahenicosanedioate; 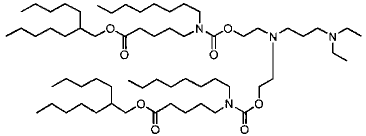
   bis(2-pentylheptyl) 11-(4-(diethylamino)butyl)-6,16-dioctyl-7,15-dioxo-8,14-dioxa-6,11,16-triazahenicosanedioate; 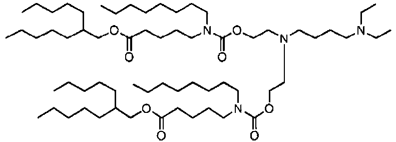
   bis(2-hexyloctyl) 11-(3-(diethylamino)propyl)-6,16-dioctyl-7,15-dioxo-8,14-dioxa-6,11,16-triazahenicosanedioate; 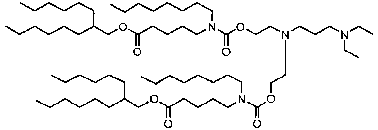
   bis(2-hexyloctyl) 11-(4-(diethylamino)butyl)-6,16-dioctyl-7,15-dioxo-8,14-dioxa-6,11,16-triazahenicosanedioate; 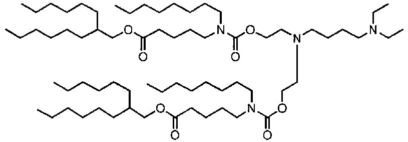
   1-heptyl 21-(2-pentylheptyl) 11-(2-(diethylamino)ethyl)-6,16-dioctyl-7,15-dioxo-8,14-dioxa-6,11,16-triazahenicosanedioate; 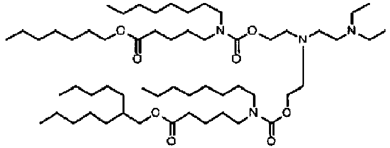
   1-heptyl 21-(2-hexyloctyl) 11-(2-(diethylamino)ethyl)-6,16-dioctyl-7,15-dioxo-8,14-dioxa-6,11,16-triazahenicosanedioate; 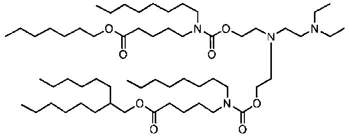
   1-octyl 21-(2-pentylheptyl) 11-(2-(diethylamino)ethyl)-6,16-dioctyl-7,15-dioxo-8,14-dioxa-6,11,16-triazahenicosanedioate; 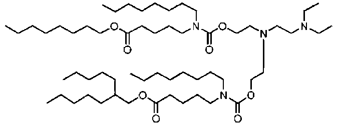
   1-(2-hexyloctyl) 21-octyl 11-(2-(diethylamino)ethyl)-6,16-dioctyl-7,15-dioxo-8,14-dioxa-6,11,16-triazahenicosanedioate; 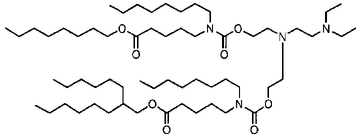
   21-heptyl 1-(2-hexyloctyl) 6-decyl-11-(2-(diethylamino)ethyl)-16-octyl-7,15-dioxo-8,14-dioxa-6,11,16-triazahenicosanedioate; 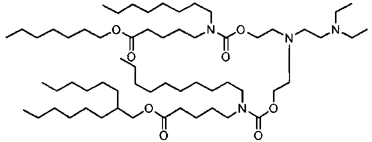
   1-(2-hexyloctyl) 21-octyl 6-decyl-11-(2-(diethylamino)ethyl)-16-octyl-7,15-dioxo-8,14-dioxa-6,11,16-triazahenicosanedioate; 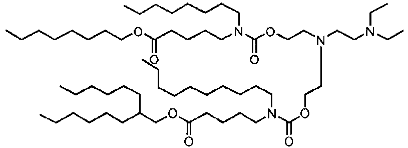
   bis(2-pentylheptyl) 12-(2-(diethylamino)ethyl)-7,17-diheptyl-8,16-dioxo-9,15-dioxa-7,12,17-triazatricosanedioate; 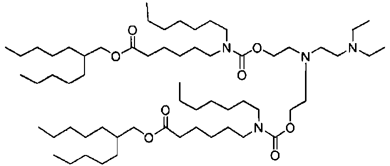
   bis(2-pentylheptyl) 12-(2-(diethylamino)ethyl)-8,16-dioxo-7,17-dipentyl-9,15-dioxa-7,12,17-triazatricosanedioate; 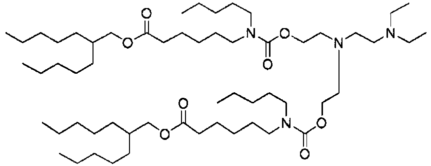
   bis(2-hexyloctyl) 7,17-dibutyl-12-(2-(diethylamino)ethyl)-8,16-dioxo-9,15-dioxa-7,12,17-triazatricosanedioate; 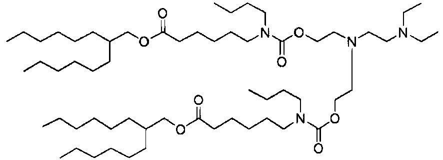
   bis(2-pentylheptyl) 12-(3-(diethylamino)propyl)-7,17-diheptyl-8,16-dioxo-9,15-dioxa-7,12,17-triazatricosanedioate; 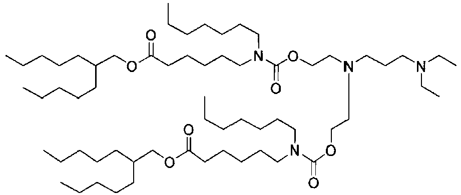
   bis(2-pentylheptyl) 12-(3-(diethylamino)propyl)-8,16-dioxo-7,17-dipentyl-9,15-dioxa-7,12,17-triazatricosanedioate; 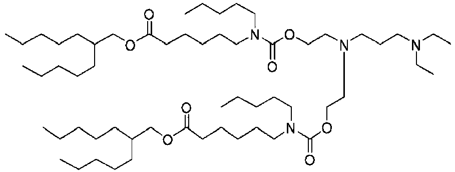
   bis(2-pentylheptyl) 12-(2-(diethylamino)ethyl)-7,17-dioctyl-8,16-dioxo-9,15-dioxa-7,12,17-triazatricosanedioate; 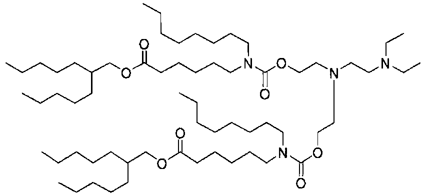
   bis(2-pentylheptyl) 12-(3-(diethylamino)propyl)-7,17-dioctyl-8,16-dioxo-9,15-dioxa-7,12,17-triazatricosanedioate. 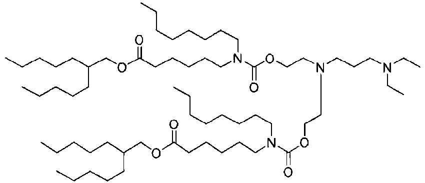
<5> A lipid composition comprising:
   the compound or a salt thereof according to any one of <1> to <4>; and
   a lipid.
<6> The lipid composition according to <5>, in which the lipid is at least one lipid selected from the group consisting of a neutral lipid and a lipid having a nonionic hydrophilic polymer chain.
<7> The lipid composition according to <5> or <6>, further comprising:
   a sterol.
<8> The lipid composition according to any one of <5> to <7>, further comprising:
   at least one selected from the group consisting of a nucleic acid, a protein, a peptide, and a low-molecular-weight compound.
<9> A pharmaceutical composition comprising:
   the lipid composition according to any one of <5> to <8>.
<10> A delivery carrier comprising:
   the lipid composition according to any one of <5> to <8>.

### Effect of the invention

By using the compound according to the embodiment of the present invention, it is possible to produce a lipid composition, a pharmaceutical composition, and a delivery carrier, which can achieve a high nucleic acid encapsulation rate and excellent delivery of nucleic acids. The lipid composition, the pharmaceutical composition, and the delivery carrier according to the embodiment of the present invention can achieve a high nucleic acid encapsulation rate and excellent delivery of nucleic acids.

### EMBODIMENTS FOR CARRING OUT THE INVENTION

Hereinafter, the present invention is specifically described.

In the present specification, "to" shows a range including numerical values described before and after "to" as a minimum value and a maximum value, respectively.

### <Compound according to embodiment of present invention>

The present invention relates to a compound represented by Formula (1) or a salt thereof, 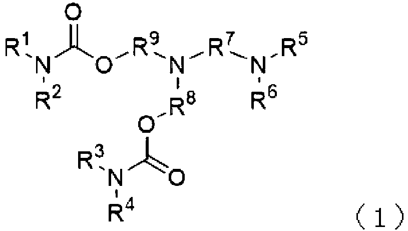
in the formula, R¹, R², R³, and R⁴ each independently represent a hydrogen atom or a hydrocarbon group having 1 to 24 carbon atoms, which may be substituted,
substituents on the hydrocarbon groups having 1 to 24 carbon atoms represented by R¹, R², R³, and R⁴, which may be substituted, each independently represent -C(O)O-R¹¹, - OC(O)-R¹², -O-R¹³, -CO-R¹⁴, -OC(O)O-R¹⁵, or -S-S-R¹⁶,
R¹¹, R¹², R¹³, R¹⁴, R¹⁵, and R¹⁶ each independently represent a hydrocarbon group having 1 to 24 carbon atoms which may be substituted with -S-R¹⁷, where R¹⁷ represents a hydrocarbon group having 1 to 12 carbon atoms,
R⁵ and R⁶ each independently represent a hydrocarbon group having 1 to 18 carbon atoms which may be substituted,
substituents on the hydrocarbon groups having 1 to 18 carbon atoms represented by R⁵ and R⁶, which may be substituted, each independently represent -OH, -COOH, -NR²¹R²², - OC(O)O-R²³, -C(O)O-R²⁴, -OC(O)-R²⁵, -O-R²⁶, -C(O)NR²⁷R²⁸, -NR²⁹C(O)R³⁰, - N(R³¹)S(O)₂R³², -N(R³³)C(O)N(R³⁴)R³⁵, -N(R³⁶)C(S)N(R³⁷)R³⁸, -OC(O)N(R³⁹)R⁴⁰, or - N(R⁴¹)C(O)OR⁴²,
R²¹ and R²² each independently represent a hydrogen atom or a hydrocarbon group having 1 to 8 carbon atoms,
R²³, R²⁴, R²⁵, R²⁶, R²⁷, R²⁸, R²⁹, R³⁰, R³¹, R³², R³³, R³⁴, R^{35,} R³⁶, R³⁷, R³⁸, R³⁹, R⁴⁰, R⁴¹, and R⁴² each independently represent a hydrogen atom or a hydrocarbon group having 1 to 24 carbon atoms, which may be substituted, and substituents on the hydrocarbon groups having 1 to 24 carbon atoms represented by R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴², which may be substituted, each independently represent an aryl group having 6 to 20 carbon atoms, a heterocyclic group, -OH, -COOH, or -NR⁵¹R⁵², where R⁵¹ and R⁵² each independently represent a hydrogen atom or a hydrocarbon group having 1 to 8 carbon atoms,
R⁷, R⁸, and R⁹ each independently represent a hydrocarbon group having 2 to 8 carbon atoms, and
R⁵ and R⁶, or R⁵ and R⁷, may be combined to form a 4- to 7-membered ring.

A hydrocarbon group having 1 to 24 carbon atoms, a hydrocarbon group having 1 to 18 carbon atoms, a hydrocarbon group having 1 to 12 carbon atoms, and a hydrocarbon group having 1 to 8 carbon atoms are each preferably an alkyl group, an alkenyl group, or an alkynyl group.

The alkyl group may be linear or branched, or may be chainlike or cyclic. Specifically, examples of the alkyl group include a methyl group, an ethyl group, a propyl group, an isopropyl group, a cyclopropyl group, a butyl group, an isobutyl group, a tert-butyl group, a cyclobutyl group, a pentyl group, a cyclopentyl group, a hexyl group, a cyclohexyl group, a heptyl group, an octyl group, a nonyl group, a decyl group, an undecyl group, a dodecyl group, a tridecyl group, a trimethyldodecyl group (preferably a 3,7,11-trimethyldodecyl group), a tetradecyl group, a pentadecyl group, a hexadecyl group, a tetramethylhexadecyl group (preferably a 3,7,11,15-tetramethylhexadecyl group), a heptadecyl group, an octadecyl group, a 2-butylhexyl group, a 2-butyloctyl group, a 1-pentylhexyl group, a 2-pentylheptyl group, a 3-pentyloctyl group, a 1-hexylheptyl group, a 1-hexylnonyl group, a 2-hexyloctyl group, a 2-hexyldecyl group, a 3-hexylnonyl group, a 1-heptyloctyl group, a 2-heptylnonyl group, a 2-heptylundecyl group, a 3-heptyldecyl group, a 1-octylnonyl group, a 2-octyldecyl group, a 2-octyldodecyl group, a 3-octylundecyl group, a 2-nonylundecyl group, a 3-nonyldodecyl group, a 2-decyldodecyl group, a 2-decyltetradecyl group, a 3-decyltridecyl group, a 2-(4,4-dimethylpentan-2-yl)-5,7,7-trimethyloctyl group, and the like.

The alkenyl group may be linear or branched, or may be chainlike or cyclic. Specifically, examples of the alkenyl group include an allyl group, a prenyl group, a pentenyl group, a hexenyl group, a heptenyl group, an octenyl group, a nonenyl group (preferably a (Z)-2-nonenyl group or an (E)-2-nonenyl group), a decenyl group, an undecenyl group, a dodecenyl group, a dodecadienyl group, a tridecenyl group (preferably a (Z)-tridec-8-enyl group), a tetradecenyl group (preferably a tetradec-9-enyl group), a pentadecenyl group (preferably a (Z)-pentadec-8-enyl group), a hexadecenyl group (preferably a (Z)-hexadec-9-enyl group), a hexadecadienyl group, a heptadecenyl group (preferably a (Z)-heptadec-8-enyl group), a heptadecadienyl group (preferably a (8Z,11Z)-heptadeca-8,11-dienyl group), an octadecenyl group (preferably a (Z)-octadec-9-enyl group), an octadecadienyl group (preferably a (9Z,12Z)-octadeca-9,12-dienyl group), and the like.

The alkynyl group may be linear or branched, or may be chainlike or cyclic. Specifically, examples of alkynyl group include a propargyl group, a butynyl group, a pentynyl group, a hexynyl group, a heptynyl group, an octynyl group, a nonynyl group, a decynyl group, an undecynyl group, a dodecynyl group, a tetradecynyl group, a pentadecynyl group, a hexadecynyl group, a heptadecynyl group, an octadecynyl group, and the like.

All of the above alkenyl groups preferably have one double bond or two double bonds. All of the above alkynyl groups preferably have one triple bond or two triple bonds.

The hydrocarbon groups having 2 to 8 carbon atoms represented by R⁷, R⁸, and R⁹ is each preferably an alkylene group, an alkenylene group, or an alkynylene group.

The alkylene group, the alkenylene group, or the alkynylene group having 2 to 8 carbon atoms may be linear or branched, or may be chainlike or cyclic.

Specific examples thereof include an ethylene group, a trimethylene group, a tetramethylene group, a pentamethylene group, a hexamethylene group, a heptamethylene group, and an octamethylene group.

The aryl group having 6 to 20 carbon atoms is preferably an aryl group having 6 to 18 carbon atoms, and more preferably an aryl group having 6 to 10 carbon atoms. Specifically, examples of the aryl group include a phenyl group, a naphthyl group, an anthracenyl group, a phenanthrenyl group, and the like.

The heterocyclic group means a heteroaryl group or a heteroaliphatic ring group.

The heteroaryl group means an aromatic heterocyclic group, may be an aromatic heterocyclic group in which an aromatic heterocyclic ring, an aromatic hydrocarbon ring, a heteroaliphatic ring, or an aliphatic hydrocarbon ring is fused, and is preferably a monocyclic nitrogen-containing heteroaryl group, a monocyclic oxygen-containing heteroaryl group, a monocyclic sulfur-containing heteroaryl group, a monocyclic nitrogen- and oxygen-containing heteroaryl group, a monocyclic nitrogen- and sulfur-containing heteroaryl group, a bicyclic nitrogen-containing heteroaryl group, a bicyclic oxygen-containing heteroaryl group, a bicyclic sulfur-containing heteroaryl group, a bicyclic nitrogen- and oxygen-containing heteroaryl group, or a bicyclic nitrogen- and sulfur-containing heteroaryl group. The 5-membered ring heteroaryl group is a monocyclic heteroaryl group, in which the number of atoms constituting the ring is five.

In addition, the aromatic heterocyclic ring means an aromatic ring having a heteroatom as a ring member, may be fused with an aromatic heterocyclic ring, an aromatic hydrocarbon ring, a heteroaliphatic ring, or an aliphatic hydrocarbon ring, and is preferably a monocyclic nitrogen-containing aromatic heterocyclic ring, a monocyclic oxygen-containing aromatic heterocyclic ring, a monocyclic sulfur-containing aromatic heterocyclic ring, a monocyclic nitrogen- and oxygen-containing aromatic heterocyclic ring, a monocyclic nitrogen- and sulfur-containing aromatic heterocyclic ring, a bicyclic nitrogen-containing aromatic heterocyclic ring, a bicyclic oxygen-containing aromatic heterocyclic ring, a bicyclic sulfur-containing aromatic heterocyclic ring, a bicyclic nitrogen- and oxygen-containing aromatic heterocyclic ring, or a bicyclic nitrogen- and sulfur-containing aromatic heterocyclic ring.

The monocyclic nitrogen-containing heteroaryl group means a heteroaryl group (this heteroaryl group may be partially saturated) in which a ring containing at least one nitrogen atom, such as a pyrrolinyl group, a pyrrolyl group, a tetrahydropyridyl group, a pyridyl group, an imidazolinyl group, an imidazolyl group, a pyrazolinyl group, a pyrazolyl group, a pyrazinyl group, a pyridazinyl group, a pyrimidinyl group, a triazolyl group, or a tetrazolyl group, has aromaticity. This heteroaryl group may be further fused with another aromatic ring or aliphatic ring.

The monocyclic oxygen-containing heteroaryl group means a heteroaryl group (this heteroaryl group may be partially saturated) in which a ring containing at least one oxygen atom, such as a furanyl group or a pyranyl group, has aromaticity. This heteroaryl group may be further fused with another aromatic ring or aliphatic ring.

The monocyclic nitrogen- and oxygen-containing heteroaryl group means an oxazolyl group, an isoxazolyl group, an oxadiazolyl group, or the like. This heteroaryl group may be further fused with another aromatic ring or aliphatic ring.

The monocyclic nitrogen- and sulfur-containing heteroaryl group means a thiazolyl group, an isothiazolyl group, a thiadiazolyl group, or the like. This heteroaryl group may be further fused with another aromatic ring or aliphatic ring.

The bicyclic nitrogen-containing heteroaryl group means a bicyclic heteroaryl group (this heteroaryl group may be partially saturated) in which a ring containing at least one nitrogen atom, such as an indolyl group, an isoindolyl group, a benzimidazolyl group, an indazolyl group, a benzotriazolyl group, a quinolyl group, an isoquinolyl group, a tetrahydroquinolyl group, a tetrahydroisoquinolyl group, a quinolizidyl group, a cinnolinyl group, a phthalazinyl group, a quinazolinyl group, a quinoxalinyl group, a naphthyridinyl group, a pyrrolopyridyl group, an imidazopyridyl group, a pyrazolopyridyl group, a pyridopyrazyl group, a purinyl group, a pteridinyl group, a 5,6,7,8-tetrahydrophthalazinyl group, a 5,6,7,8-tetrahydrocinnolinyl group, a 1,2,3,4-tetrahydropyrido[2,3-d]pyrazidinyl group, a 5,6,7,8-tetrahydro-[1,2,4]triazolo[4,3-a]pyrazidinyl group, a 5,6,7,8-tetrahydropyrido[3,4-d]pyrazidinyl group, a 5,6,7,8-tetrahydropyrido[3,2-c]pyrazidinyl group, a 5,6,7,8-tetrahydropyrido[4,3-c]pyrazidinyl group, a 6,7-dihydro-5H-cyclopenta[d]pyrazidinyl group, a 6,7-dihydro-5H-cyclopenta[c]pyrazidinyl group, a 2,3-dihydro-1H-pyrrolo[2,3-d]pyrazidinyl group, a 6,7-dihydro-5H-pyrrolo[3,4-d]pyrazidinyl group, a 6,7-dihydro-5H-pyrrolo[3,2-c]pyrazidinyl group, a 6,7-dihydro-5H-pyrrolo[3,4-c]pyrazidinyl group, or a 6,7-dihydro-5H-pyrrolo[2,3-c]pyrazidinyl group, has aromaticity.

The bicyclic oxygen-containing heteroaryl group means a bicyclic heteroaryl group (this heteroaryl group may be partially saturated) in which a ring containing at least one oxygen atom, such as a benzofuranyl group, an isobenzofuranyl group, and a chromenyl group, has aromaticity.

The bicyclic nitrogen- and oxygen-containing heteroaryl group means a bicyclic heteroaryl group (this heteroaryl group may be partially saturated) in which a ring containing at least one nitrogen atom and at least one oxygen atom, such as a benzoxazolyl group, a benzoisoxazolyl group, a benzoxadiazolyl group, a dihydropyranopyridyl group, a dihydrodioxinopyridyl group, a dihydropyridooxadienyl group, a 3,4-dihydro-2H-pyrano[2,3-d]pyridazinyl group, a 7,8-dihydro-5H-pyrano[3,4-d]pyridazinyl group, a 7,8-dihydro-6H-pyrano[3,2-c]pyridazinyl group, a 7,8-dihydro-5H-pyrano[4,3-c]pyridazinyl group, a 2,3-dihydrofuro[2,3-d]pyridazinyl group, a 5,7-dihydrofuro[3,4-d]pyridazinyl group, a 6,7-dihydrofuro[3,2-c]pyridazinyl group, a 5,7-dihydrofuro[3,4-c]pyridazinyl group, and a 5,6-dihydrofuro[2,3-c]pyridazinyl group, has aromaticity.

The heteroaliphatic ring group means a nitrogen-containing heteroaliphatic ring group, an oxygen-containing heteroaliphatic ring group, a sulfur-containing heteroaliphatic ring group, a nitrogen- and oxygen-containing heteroaliphatic ring group, a nitrogen- and sulfur-containing heteroaliphatic ring group, a heterobridged ring group, or a heterospiro ring group.

In addition, the heteroaliphatic ring means an aliphatic ring having a heteroatom as a ring member, and preferred examples thereof include a nitrogen-containing heteroaliphatic ring, an oxygen-containing heteroaliphatic ring, a sulfur-containing heteroaliphatic ring, a nitrogen- and oxygen-containing heteroaliphatic ring, a nitrogen- and sulfur-containing heteroaliphatic ring, a heterobridged ring, and a heterospiro ring.

The nitrogen-containing heteroaliphatic ring group means a heterocyclic aliphatic ring group in which a ring containing at least one nitrogen atom, such as an azetidinyl, a pyrrolidinyl, a piperidinyl, a homopiperidinyl, an octahydroazocinyl, an imidazolidinyl, a pyrazolidinyl, a piperazinyl, or a homopiperazinyl group, does not have aromaticity. This nitrogen-containing heteroaliphatic ring group may be further fused with another aromatic ring or aliphatic ring.

The oxygen-containing heteroaliphatic ring group means a tetrahydrofuranyl group, a tetrahydropyranyl group, an oxetanyl group, a 1,3-dioxanyl group, or the like. This oxygen-containing heteroaliphatic ring group may be further fused with another aromatic ring or aliphatic ring.

The nitrogen- and oxygen-containing heteroaliphatic ring group means a morpholinyl group, a 1,4-oxazepanyl group, or the like. This nitrogen- and oxygen-containing heteroaliphatic ring group may be further fused with another aromatic ring or aliphatic ring.

The heteroaliphatic ring C₁₋₈ alkyl group refers to a linear, branched, or cyclic C₁₋₈ alkyl group to which a heteroaliphatic ring group such as a pyrrolidinylmethyl group, a pyrrolidinylethyl group, a pyrrolidinylpropyl group, a pyrrolidinyloctyl group, a piperidinylmethyl group, or a tetrahydrofuranylmethyl group is bonded.

In Formula (1), preferably,
R¹ represents -R^{1a}-L¹-R^{1b}, R^{1a} represents a hydrocarbon group having 1 to 18 carbon atoms, L¹ represents -C(O)O-, -OC(O)-, -OC(O)O-, or -S-S-, and R^{1b} represents a hydrocarbon group having 1 to 18 carbon atoms,
R³ represents -R^{3a}-L³-R^{3b}, R^{3a} represents a hydrocarbon group having 1 to 18 carbon atoms, L³ represents -C(O)O-, -OC(O)-, -OC(O)O-, or -S-S-, and R^{3b} represents a hydrocarbon group having 1 to 18 carbon atoms,
R² and R⁴ each independently represent a hydrocarbon group having 1 to 18 carbon atoms, which may be substituted, and substituents on the hydrocarbon groups having 1 to 18 carbon atoms represented by R² and R⁴, which may be substituted, each independently represent -C(O)O-R¹¹, -OC(O)-R¹², -O-R¹³, -CO-R¹⁴, -OC(O)O-R¹⁵, or -S-S-R¹⁶,
R¹¹, R¹², R¹³, R¹⁴, R¹⁵, and R¹⁶ each independently represent a hydrocarbon group having 1 to 18 carbon atoms,
R⁵ and R⁶ each independently represent a hydrocarbon group having 1 to 12 carbon atoms, which may be substituted,
substituents on the hydrocarbon groups having 1 to 12 carbon atoms represented by R⁵ and R⁶, which may be substituted, each independently represent -OH, -O-R²⁶, -C(O)NR²⁷R²⁸, or -NR²⁹C(O)R³⁰,
R²⁶, R²⁷, R²⁸, R²⁹, and R³⁰ each independently represent a hydrogen atom or a hydrocarbon group having 1 to 12 carbon atoms, which may be substituted, and substituents on the hydrocarbon groups having 1 to 12 carbon atoms represented by R²⁶, R²⁷, R²⁸, R²⁹, and R³⁰, which may be substituted, each independently represent an aryl group having 6 to 10 carbon atoms or a heterocyclic group, and
R⁷, R⁸, and R⁹ each independently represent -(CH₂)ₙ-, and n represents an integer of 2 to 8.

In Formula (1), more preferably,
R¹ represents -R^{1a}-L¹-R^{1b}, R^{1a} represents a hydrocarbon group having 1 to 18 carbon atoms, L¹ represents -C(O)O- or -OC(O)-, and R^{1b} represents a hydrocarbon group having 1 to 18 carbon atoms,
R³ represents -R^{3a}-L³-R^{3b}, R^{3a} represents a hydrocarbon group having 1 to 18 carbon atoms, L³ represents -C(O)O- or -OC(O)-, and R^{3b} represents a hydrocarbon group having 1 to 18 carbon atoms,
R² and R⁴ each independently represent a hydrocarbon group having 1 to 10 carbon atoms,
R⁵ and R⁶ each independently represent a hydrocarbon group having 1 to 6 carbon atoms, which may be substituted,
substituents on the hydrocarbon groups having 1 to 6 carbon atoms represented by R⁵ and R⁶, which may be substituted, each independently represent -OH, -O-R²⁶, -C(O)NR²⁷R²⁸, or -NR²⁹C(O)R³⁰,
R²⁶, R²⁷, R²⁸, R²⁹, and R³⁰ each independently represent a hydrogen atom or a hydrocarbon group having 1 to 12 carbon atoms, which may be substituted, and substituents on the hydrocarbon groups having 1 to 12 carbon atoms represented by R²⁶, R²⁷, R²⁸, R²⁹, and R³⁰, which may be substituted, each independently represent an aryl group having 6 to 10 carbon atoms, and
R⁷, R⁸, and R⁹ each independently represent -(CH₂)ₙ-, and n represents an integer of 2 to 8.

In Formula (1), most preferably,
R¹ represents -R^{1a}-L¹-R^{1b}, R^{1a} represents a hydrocarbon group having 1 to 5 carbon atoms, L¹ represents -C(O)O-, R^{1b} represents a hydrocarbon group having 7 to 14 carbon atoms,
R³ represents -R^{3a}-L³-R^{3b}, R^{3a} represents a hydrocarbon group having 1 to 5 carbon atoms, L³ represents -C(O)O-, R^{3b} represents a hydrocarbon group having 7 to 14 carbon atoms,
R² and R⁴ each independently represent a hydrocarbon group having 3 to 8 carbon atoms,
R⁵ and R⁶ each independently represent a hydrocarbon group having 2 carbon atoms, and
R⁷, R⁸, and R⁹ each independently represent -(CH₂)ₙ-, and n represents an integer of 2 to 4.

The compound according to the embodiment of the present invention may form a salt.

Examples of the salt in a basic group include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salt in an acidic group include salts with alkali metals such as sodium and potassium; salts with alkaline earth metals such as calcium and magnesium; ammonium salts; salts with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine; and the like.

Among the above-described salts, for example, pharmacologically acceptable salts are preferable.

Specifically, as the compound according to the embodiment of the present invention, for example, the compounds described in Examples 1 to 51 which will be described later are preferable. However, the present invention is not limited thereto.

The compounds described in Examples 1 to 51 are called Compounds 1 to 51 respectively.

Among the above-described compounds, Compound 1, Compound 2, Compound 3, Compound 4, Compound 5, Compound 6, Compound 7, Compound 12, Compound 13, Compound 14, Compound 15, Compound 16, Compound 17, Compound 18, Compound 20, Compound 21, Compound 23, Compound 24, Compound 25, Compound 26, Compound 27, Compound 32, Compound 33, Compound 34, Compound 35, Compound 36, Compound 37, Compound 38, Compound 40, and Compound 42 are preferable.

Compound 1, Compound 2, Compound 3, Compound 4, Compound 5, Compound 17, Compound 20, Compound 23, Compound 24, Compound 25, Compound 26, Compound 27, Compound 33, Compound 35, Compound 36, Compound 37, Compound 38, Compound 40, and Compound 42 are more preferable.

Compound 1, Compound 2, Compound 3, Compound 4, Compound 5, Compound 17, Compound 25, Compound 26, Compound 27, Compound 33, Compound 35, Compound 36, Compound 37, Compound 38, Compound 40, and Compound 42 are still more preferable.

Compound 1, Compound 2, Compound 3, Compound 4, Compound 5, Compound 17, Compound 35, Compound 36, Compound 37, Compound 38, Compound 40, and Compound 42 are particularly preferable.

Compound 1, Compound 2, Compound 3, Compound 4, Compound 5, and Compound 37 are most preferable.

In addition, the compound according to the embodiment of the present invention also includes a compound having the following structure.

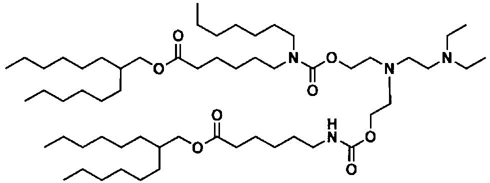

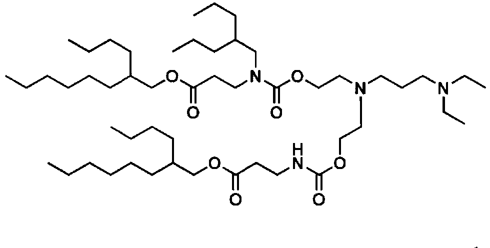

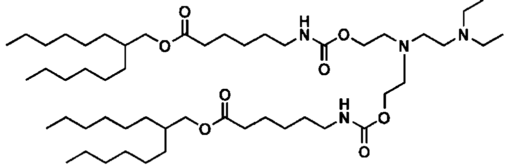

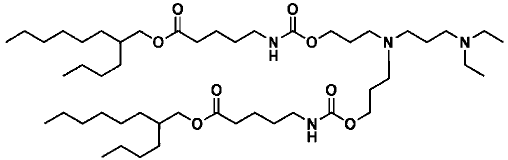

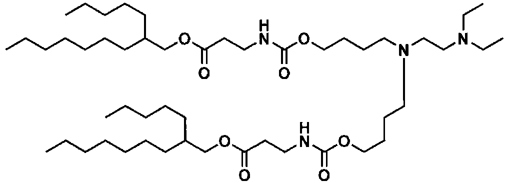

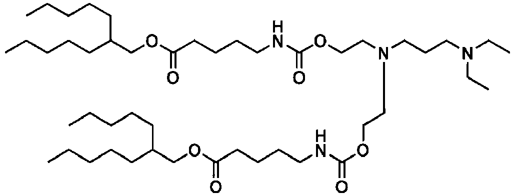

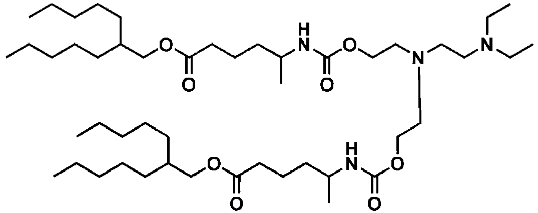

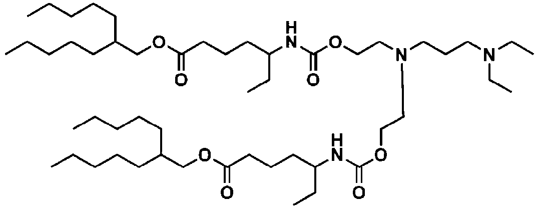

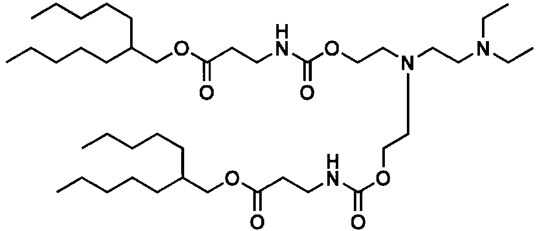

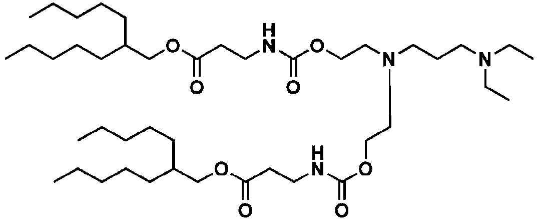

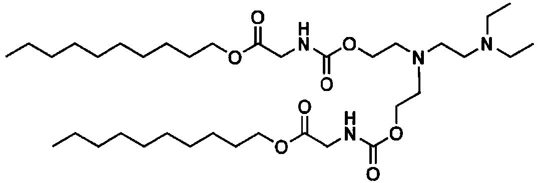

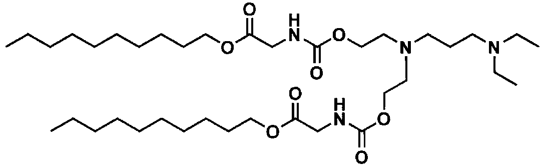

### <Manufacturing method>

The manufacturing method of the compound according to the embodiment of the present invention is described.

The compound according to the embodiment of the present invention can be manufactured using known methods in combination. For example, the compound can be manufactured by the following manufacturing method.

### [Manufacturing method 1]

A method for manufacturing the compound represented by Formula [1] from the compound represented by Formula [2].

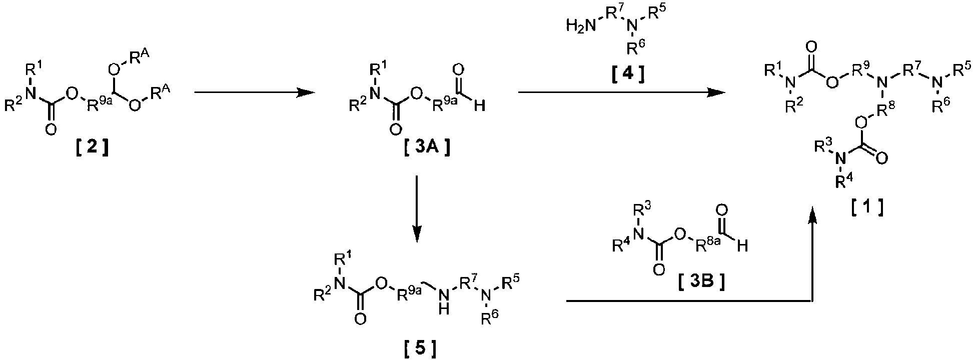

In the formulae, R¹, R², R³, R⁴, R⁵, R⁶, R⁷, R⁸, and R⁹ have the same meanings as described above, and R^{8a}, R^{9a}, and R^{A} each represent a hydrocarbon group having 1 to 7 carbon atoms.

(1-1)
The compound represented by Formula [3A] can be manufactured by reacting the compound represented by Formula [2] in the presence of water and acid, in the presence of a solvent, or in the absence of a solvent.

Examples of the acid used in this reaction include an inorganic acid and an organic acid. An organic acid is preferable, specific examples thereof include formic acid, acetic acid, trifluoroacetic acid, 4-toluenesulfonic acid, methanesulfonic acid, and the like, and formic acid is more preferable.

The used amount of the acid is only required to be 1 to 100 times (v/w) and preferably 1 to 10 times (v/w) the amount of the compound represented by Formula [2].

The used amount of the water is only required to be 0.1 to 100 times (v/w) and preferably 0.1 to 10 times (v/w) the amount of the compound represented by Formula [2].

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

The used amount of the solvent is not particularly limited, but is only required to be 0.1 to 50 times (v/w) the amount of the compound represented by Formula [2].

This reaction is required only to carry out at -30°C to 150°C, preferably at 0°C to 100°C for 5 minutes to 48 hours.

(1-2)
The compound represented by Formula [1] can be manufactured by reacting the compound represented by Formula [3A] with the compound represented by Formula [4] in the presence of a reducing agent.

As the compound represented by Formula [4], for example, N,N-diethylethylenediamine, N,N-diethyl-1,3-diaminopropane, and the like are known.

The solvent used in this reaction is not particularly limited as long as the solvent does not affect the reaction. Examples of the solvent include halogenated hydrocarbons, alcohols, ethers, esters, amides, nitriles, sulfoxides, and aromatic hydrocarbons. These solvents may be used by being mixed together.

Examples of the preferred solvent include esters, and ethyl acetate is more preferable.

The used amount of the solvent is not particularly limited, but is only required to be 1 to 500 times (v/w) the amount of the compound represented by Formula [3A].

Examples of the reducing agent used in this reaction include sodium borohydride, sodium cyanoborohydride, pyridine borane, 2-picoline borane, and sodium triacetoxyborohydride, and sodium triacetoxyborohydride is more preferable.

The used amount of the reducing agent is only required to be 1 to 100 times and preferably 1 to 10 times the molar amount of the compound represented by Formula [3A].

The used amount of the compound represented by Formula [4] is only required to be 0.1 to 1 times the molar amount of the compound represented by Formula [3A].

This reaction is required only to carry out at -30°C to 150°C, preferably at 0°C to 100°C for 5 minutes to 48 hours.

(1-3)
The compound represented by Formula [5] can be manufactured by reacting the compound represented by Formula [3A] with the compound represented by Formula [4] in the presence of a reducing agent.

This reaction may be performed according to the manufacturing method (1-2), and the compound represented by Formula [4] is only required to be used in an amount of 1 to 10 times the molar amount of the compound represented by Formula [3A].

(1-4)
The compound represented by Formula [1] can be manufactured by reacting the compound represented by Formula [3B] with the compound represented by Formula [5] in the presence of a reducing agent.

This reaction may be performed according to the manufacturing method (1-2), and the compound represented by Formula [3B] is only required to be used in an amount of 1 to 10 times the molar amount of the compound represented by Formula [5].

### [Manufacturing method 2]

A method for manufacturing the compound represented by Formula [2] from the compound represented by Formula [6] and the compound represented by Formula [7].

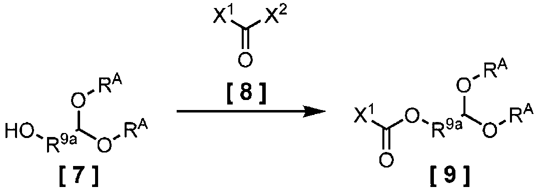

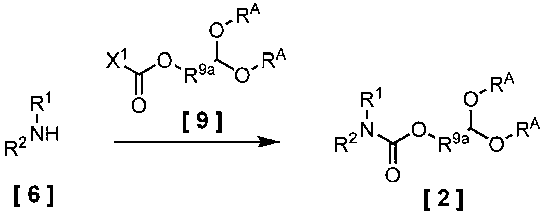

In the formulae, R¹, R², R^{9a}, and R^{A} have the same meanings as described above, and X¹ and X² each represent a leaving group.

Examples of the leaving group include a chloro group, a fluoro group, a bromo group, a trichloromethoxy group, a 4-nitro-phenoxy group, a 2,4-dinitrophenoxy group, a 2,4,6-trichlorophenoxy group, a pentafluorophenoxy group, a 2,3,5,6-tetrafluorophenoxy group, an imidazolyl group, a triazolyl group, a 3,5-dioxo-4-methyl-1,2,4-oxadiazolidinyl group, an N-hydroxysuccinimidyl group, and the like.

(2-1)
The compound represented by Formula [9] can be manufactured by reacting the compound represented by Formula [7] with the compound represented by Formula [8] in the presence or absence of a base.

As the compound represented by Formula [8], for example, 1,1'-carbonyldi(1,2,4-triazole), 1,1'-carbonyldiimidazole, 4-nitrophenyl chloroformate, triphosgene, phosgene, and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include ethers, and tetrahydrofuran is more preferable.

The used amount of the solvent is not particularly limited, but is only required to be 1 to 500 times (v/w) the amount of the compound represented by Formula [7].

Examples of the base used in this reaction include an inorganic base and an organic base. As the base, an organic base is preferable. Specifically, examples thereof include triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of the compound represented by Formula [7].

The used amount of the compound represented by Formula [3] is not particularly limited, but is only required to be 1 to 10 times the molar amount of the compound represented by Formula [2].

This reaction is required only to carry out at -30°C to 150°C, preferably at 0°C to 100°C for 5 minutes to 48 hours.

(2-2)
The compound represented by Formula [2] can be manufactured by reacting the compound represented by Formula [6] with the compound represented by Formula [9] in the presence of a base.

As the compound represented by Formula [6], for example, dioctylamine and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include nitriles, and acetonitrile is more preferable.

The used amount of the solvent is not particularly limited, but is only required to be 1 to 500 times (v/w) the amount of the compound represented by Formula [6].

Examples of the base used in this reaction include an inorganic base and an organic base. Specifically, examples thereof include potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of the compound represented by Formula [6].

The used amount of the compound represented by Formula [9] is not particularly limited, but is only required to be 0.1 to 10 times the molar amount of the compound represented by Formula [6].

This reaction is required only to carry out at -30°C to 150°C, preferably at 0°C to 100°C for 5 minutes to 48 hours.

### [Manufacturing method 3]

A method for manufacturing the compound represented by Formula [6A].

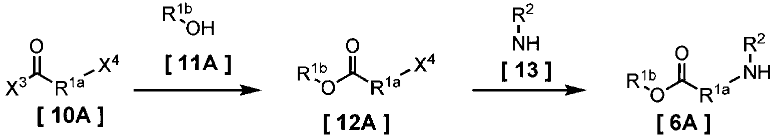

In the formulae, R², R^{1a}, and R^{1b} have the same meanings as described above, X³ represents a hydroxyl group and a leaving group, X⁴ represents a leaving group, and the leaving group has the same meaning as described above.

(3-1)
The compound represented by Formula [12A] can be manufactured by reacting the compound represented by Formula [10A] with the compound represented by Formula [11A] in the presence or absence of an acid, in the presence or absence of a condensing agent or an acid halide, and in the presence or absence of a base.

As the compound represented by Formula [10A], for example, 5-bromovaleric acid, chloroacetyl chloride, and the like are known.

As the compound represented by Formula [11A], for example, 2-butyl-1-octanol, 2-pentyl-1-heptanol, 1-decanol, and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include aromatic hydrocarbons and ethers, and toluene and tetrahydrofuran are more preferable.

The used amount of the solvent is not particularly limited, but is only required to be 1 to 500 times (v/w) the amount of the compound represented by Formula [10A].

Examples of the acid used in this reaction include an inorganic acid and an organic acid. As the acid, sulfonic acids are preferable. Specifically, examples thereof include sulfuric acid, 4-toluenesulfonic acid, methanesulfonic acid, and the like.

Examples of the condensing agent used in this reaction include carbodiimides such as N,N'-dicyclohexylcarbodiimide and 1-ethyl-3-(3-dimethylaminopropyl)carbodiimide; carbonyls such as carbonyldiimidazole; acid azides such as diphenylphosphoryl azide; acid cyanides such as diethylphosphoryl cyanide; uroniums such as 2-ethoxy-1-ethoxycarbonyl-1,2-dihydroquinoline; O-benzotriazol-1-yl-1,1,3,3-tetramethyluronium=hexafluorophosphate; O-(7-azabenzotriazol-1-yl)-1,1,3,3-tetramethyluronium=hexafluorophosphate; and the like.

Examples of the acid halide used in this reaction include carboxylic acid halides such as acetyl chloride and trifluoroacetyl chloride; sulfonic acid halides such as methanesulfonyl chloride and tosyl chloride; chloroformic acid esters such as ethyl chloroformate and isobutyl chloroformate; and the like.

Examples of the base used in this reaction include an inorganic base and an organic base. An organic base is preferable. Specifically, examples thereof include triethylamine, N,N-diisopropylethylamine, 4-methylmorpholine, pyridine, 1,8-diazabicyclo[5.4.0]-7-undecene, N,N-dimethylaminopyridine, and the like.

The used amount of the base is only required to be 1 to 50 times and preferably 1 to 10 times the molar amount of the compound represented by Formula [10A].

The used amount of the compound represented by Formula [11A] is not particularly limited, but is required to be 0.8 to 10 times (v/w) the amount of the compound represented by Formula [10A].

This reaction is required only to carry out at -30°C to 150°C, preferably at 0°C to 100°C for 5 minutes to 48 hours.

(3-2)
The compound represented by Formula [6A] can be manufactured by reacting the compound represented by Formula [12A] with the compound represented by Formula [13] in the presence or absence of a base and in the presence or absence of an additive.

As the compound represented by Formula [13], for example, 1-butylamine, 1-hexylamine, and the like are known.

The solvent used in this reaction is not particularly limited as long as the solvent does not affect the reaction. Examples of the solvent include halogenated hydrocarbons, ethers, esters, amides, nitriles, sulfoxides, and aromatic hydrocarbons. These solvents may be used by being mixed together.

Preferred examples of the solvent include nitriles and ethers, and acetonitrile and tetrahydrofuran are more preferable.

The used amount of the solvent is not particularly limited, but is only required to be 1 to 500 times (v/w) the amount of the compound represented by Formula [12A].

Examples of the base used in this reaction include an inorganic base and an organic base. Specifically, examples thereof include potassium hydroxide, sodium hydroxide, lithium hydroxide, potassium carbonate, sodium carbonate, lithium carbonate, potassium phosphate, sodium phosphate, lithium phosphate, triethylamine, N,N-diisopropylethylamine, 4-methylmorpholine, pyridine, 1,8-diazabicyclo[5.4.0]-7-undecene, N,N-dimethylaminopyridine, and the like, and the potassium carbonate is more preferable.

The used amount of the base is only required to be 1 to 50 times and preferably 1 to 10 times the molar amount of the compound represented by Formula [12A].

The used amount of the compound represented by Formula [13] is not particularly limited, but is only required to be 1 to 10 times the molar amount of the compound represented by Formula [12A].

Specific examples of the additive used in this reaction include lithium iodide, sodium iodide, potassium iodide, benzyltriethylammonium iodide, benzyltriethylammonium bromide, and the like.

The used amount of the additive used is only required to be 0.1 to 10 times the molar amount of the compound represented by Formula [12A].

This reaction is required only to carry out at -30°C to 150°C, preferably at 0°C to 100°C for 5 minutes to 48 hours.

### [Manufacturing method 4]

A method for manufacturing the compound represented by Formula [6B].

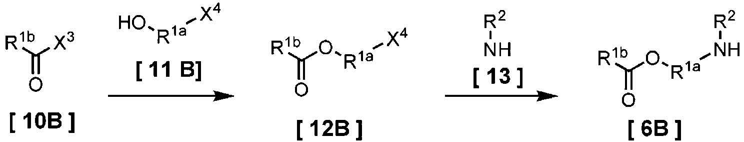

In the formulae, R², R^{1a}, R^{1b}, X³, and X⁴ have the same meanings as described above.

(4-1)
The compound represented by Formula [12B] can be manufactured by the same method as in the manufacturing method (3-1), except that the compound represented by Formula [10B] is used instead of the compound represented by Formula [10A] and the compound represented by Formula [11B] is used instead of the compound represented by Formula [11A].

(4-2)
The compound represented by Formula [6B] can be manufactured by the same method as in the manufacturing method (3-2) except that the compound represented by Formula [12B] is used instead of the compound represented by Formula [12A].

### [Manufacturing method 5]

A method for manufacturing the compound represented by Formula [6C].

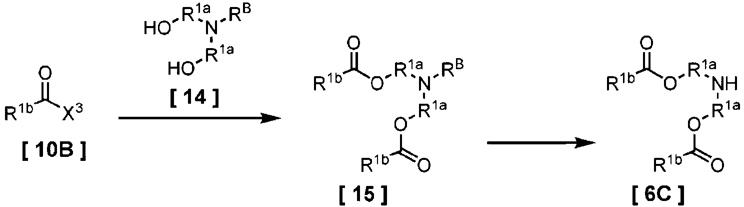

In the formulae, R^{1a}, R^{1b}, and X³ have the same meanings as described above, and R^{B} represents an amino protective group.

(5-1)
The compound represented by Formula [15] can be manufactured by reacting the compound represented by Formula [10B] with the compound represented by Formula [14] in the presence or absence of an acid, in the presence or absence of a condensing agent or an acid halide, and in the presence or absence of a base.

As the compound represented by Formula [10B], for example, decanoic acid, decanoyl chloride, and the like are known.

As the compound represented by Formula [15], for example, tert-butylbis(2-hydroxyethyl)carbamate is known.

This reaction may be performed according to the manufacturing method (3-1).

(5-2)
The compound represented by Formula [6C] can be manufactured by deprotecting the compound represented by Formula [15].

This reaction may be performed, for example, according to the method described in T. W. Greene et al., Protective Groups in Organic Synthesis, 4th Edition, pp. 696 to 926, 2007, John Wiley & Sons, INC.

### [Manufacturing method 6]

A method for manufacturing the compound represented by Formula [6D].

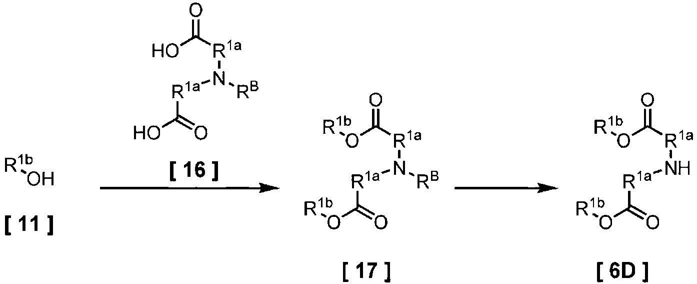

In the formulae, R^{1a}, R^{1b}, and R^{B} have the same meanings as described above.

(6-1)
The compound represented by Formula [6D] can be manufactured by reacting the compound represented by Formula [11] with the compound represented by Formula [16] in the presence or absence of an acid, in the presence or absence of a condensing agent or an acid halide, and in the presence or absence of a base.

As the compound represented by Formula [11], for example, 1-decanol and the like are known.

As the compound represented by Formula [16], for example, N-(tert-butoxycarbonyl)iminodiacetic acid and the like are known.

This reaction may be performed according to the manufacturing method (3-1).

(6-2)
The compound represented by Formula [6D] can be manufactured by deprotecting the compound represented by Formula [17].

This reaction may be performed according to the manufacturing method (3-2).

In a case where the compounds used in the above-described manufacturing methods have isomers (for example, an optical isomer, a geometric isomer, a tautomer, and the like), these isomers can also be used.

In addition, in a case where the compounds are in the form of solvates, hydrates, and crystals of various shapes, these solvates, hydrates, and crystals of various shapes can also be used.

In a case where the compounds used in the above-described manufacturing methods have, for example, an amino group, a hydroxyl group, and a carboxyl group, these groups can be protected with ordinary protecting groups in advance, and these protecting groups can be eliminated by known methods after the reaction.

The compounds obtained by the above-described manufacturing methods can be induced into other compounds by being subjected to known reaction such as condensation, addition, oxidation, reduction, transition, substitution, halogenation, dehydration, or hydrolysis, or by being subjected to these reactions that are appropriately combined.

### <Lipid composition>

In the present invention, a composition containing the compound according to an embodiment of the present invention or a salt thereof can be prepared. In preparing the lipid composition, in addition to the compound according to the embodiment of the present invention, it is possible to use at least one kind of lipid selected from the group consisting of a sterol and a lipid having a nonionic hydrophilic polymer chain. The lipid composition can further contain a neutral lipid. The lipid composition can further contain a nucleic acid.

In the lipid composition according to the embodiment of the present invention, the amount of the compound represented by Formula (1) according to the embodiment of the present invention or a salt thereof mixed in, with respect to the total mass of lipids, is preferably 20 mol% to 80 mol%, more preferably 30 mol% to 70 mol%, and even more preferably 35 mol% to 65 mol%.

### <Sterol>

The lipid composition according to the embodiment of the present invention preferably contains a sterol. In a case where the lipid composition according to the embodiment of the present invention contain a sterol, the fluidity of the membrane can be reduced, and hence the lipid composition can be effectively stabilized.

The sterol is not particularly limited, and examples thereof can include cholesterol, phytosterol (sitosterol, stigmasterol, fucosterol, spinasterol, brassicasterol, and the like), ergosterol, cholestanone, cholestenone, coprostanol, cholesteryl-2'-hydroxyethyl ether, and cholesteryl-4'-hydroxybutyl ether. Among these, cholesterol is preferable.

In the lipid composition according to the embodiment of the present invention, the amount of the sterol mixed in, with respect to the total mass of lipids, is preferably 10 mol% to 70 mol%, more preferably 20 mol% to 65 mol%, and even more preferably 25 mol% to 60 mol%.

### <Neutral lipid>

The lipid composition according to the embodiment of the present invention may contain a neutral lipid. The neutral lipid is not particularly limited, and examples thereof include phosphatidylcholine, phosphatidylethanolamine, sphingomyelin, ceramide, and the like. Among these, phosphatidylcholine is preferable. The neutral lipid may be a single neutral lipid or a combination of a plurality of different neutral lipids.

The phosphatidylcholine is not particularly limited, and examples thereof include soybean lecithin (SPC), hydrogenated soybean lecithin (HSPC), egg yolk lecithin (EPC), hydrogenated egg yolk lecithin (HEPC), dimyristoylphosphatidylcholine (DMPC), dipalmitoylphosphatidylcholine (DPPC), distearoylphosphatidylcholine (DSPC), 1-palmitoyl-2-oleoylphosphatidylcholine (POPC), dioleoylphosphatidylcholine (DOPC), and the like.

The phosphatidylethanolamine is not particularly limited, and examples thereof include dimyristoylphosphatidylethanolamine (DMPE), dipalmitoylphosphatidylethanolamine (DPPE), distearoylphosphatidylethanolamine (DSPE), dioleoylphosphatidylethanolamine (DOPE), dilinoleoylphosphatidylethanolamine (DLoPE), diphytanoylphosphatidylethanolamine (D(Phy)PE), 1-palmitoyl-2-oleoylphosphatidylethanolamine (POPE), ditetradecylphosphatidylethanolamine, dihexadecylphosphatidylethanolamine, dioctadecylphosphatidylethanolamine, diphytanylphosphatidylethanolamine, and the like.

The sphingomyelin is not particularly limited, and examples thereof include egg yolk-derived sphingomyelin, milk-derived sphingomyelin, and the like.

The ceramide is not particularly limited, and examples thereof include egg yolk-derived ceramide, milk-derived ceramide, and the like.

In the lipid composition according to the embodiment of the present invention, the amount of the neutral lipid mixed in, with respect to the total amount of the constituent lipid components, is preferably 0 mol% or more and 55 mol% or less and more preferably 0 mol% or more and 40 mol% or less.

### <Lipid having nonionic hydrophilic polymer chain>

The lipid composition according to the embodiment of the present invention may contain a lipid having a nonionic hydrophilic polymer chain in an oil phase. In the present invention, in a case where the lipid composition contains the lipid having a nonionic hydrophilic polymer chain in an oil phase, the dispersion of the lipid particles can be effectively stabilized.

The nonionic hydrophilic polymer is not particularly limited, and examples thereof include a nonionic vinyl-based polymer, a nonionic polyamino acid, a nonionic polyester, a nonionic polyether, a nonionic natural polymer, a nonionic modified natural polymer, and a block polymer or a graft copolymer having two or more kinds of these polymers as constitutional units.

Among these nonionic hydrophilic polymers, a nonionic polyether, a nonionic polyester, a nonionic polyamino acid, or a nonionic synthetic polypeptide is preferable, a nonionic polyether or a nonionic polyester is more preferable, a nonionic polyether or a nonionic monoalkoxy polyether is even more preferable, and polyethylene glycol (hereinafter, polyethylene glycol will be also called PEG) is particularly preferable.

The lipid having a nonionic hydrophilic polymer chain is not particularly limited, and examples thereof include PEG-modified phosphoethanolamine, a diacylglycerol PEG derivative, a monoacylglycerol PEG derivative, a dialkylglycerol PEG derivative, a cholesterol PEG derivative, a ceramide PEG derivative, and the like. Among these, monoacylglycerol PEG derivative or diacylglycerol PEG derivative is preferable.

The weight-average molecular weight of the PEG chain of the nonionic hydrophilic polymer derivative is preferably 500 to 5,000 and more preferably 750 to 3,000.

The nonionic hydrophilic polymer chain may be branched or may have a substituent such as a hydroxymethyl group.

In the lipid composition according to the embodiment of the present invention, the amount of the lipid having a nonionic hydrophilic polymer chain mixed in, with respect to the total amount of lipids, is preferably 0.1 mol% to 12 mol%, more preferably 0.3 mol% to 6 mol%, and still more preferably 0.5 mol% to 5 mol%.

### <Nucleic acid, protein, peptide, and low-molecular-weight compound>

The lipid composition according to the embodiment of the present invention may contain at least one selected from the group consisting of a nucleic acid, a protein, a peptide, and a low-molecular-weight compound.

Examples of the nucleic acid include plasmid DNA, nanoplasmid DNA, single-stranded DNA, double-stranded DNA, small interfering RNA (siRNA), microRNA (miRNA), mRNA, an antisense oligonucleotide (also referred to as ASO), ribozyme, an aptamer, dsRNA, saRNA, sgRNA, shRNA, tRNA, circular RNA and the like. The lipid composition may contain any of these. In addition, the lipid particles may contain a modified nucleic acid. As the nucleic acid, RNA is particularly preferable, and RNA having a number of bases of 5 to 20,000 is preferable.

In the lipid composition according to the embodiment of the present invention, the mass ratio of the lipids to the nucleic acid is preferably 2 to 1,000, more preferably 3 to 500, still more preferably 5 to 200, and particularly preferably 5 to 100.

Examples of the protein, peptide, and low-molecular-weight molecule include an intracellular protein, an intracellular peptide, a membrane protein, a membrane peptide, a secreted protein, a secreted peptide, a synthetic protein, a synthetic peptide, a natural low-molecular-weight compound, a synthetic low-molecular-weight compound, a compound having an antitumor activity, and the like.

More specifically, examples of the protein include a CRISPR-Cas protein, a gene editing-related protein such as a zinc-finger protein, and TALEN, a hormone such as erythropoietin, an endogenous factor such as VEGF, a cancer antigen protein, an antibody, and the like. Examples of the peptide include a functional domain or a recognition domain of the above-described protein. mRNA or DNA encoding the above-described protein may be contained.

As the peptide, a natural peptide is desirable. In addition, the peptide structure may be linear or may be cyclic. In a case where the peptide is cyclic, examples of a linking portion of the ring include an amide bond, a disulfide bond, and the like.

Examples of the low-molecular-weight compound include an anticancer agent, an antibacterial agent, an antifungal agent, and the like. These proteins, peptides, and low-molecular-weight compounds may have physiological activity in vivo or may not have physiological activity. Here, the low-molecular-weight compound refers to an organic compound having a molecular weight of about 1,000 or less.

### <Manufacturing method of lipid composition>

The manufacturing method of the lipid composition according to the embodiment of the present invention will be described.

The manufacturing method of the lipid composition is not limited, and for example, the lipid composition can be manufactured by a method in which all of the constituent components of the lipid composition or some of oil-soluble components of the lipid composition are dissolved in an organic solvent or the like to form an oil phase, water-soluble components of the lipid composition are dissolved in water to form a water phase, and then the oil phase and the water phase are mixed together. A micromixer may be used for mixing, or an emulsification using an emulsifying machine such as a homogenizer, an ultrasonic emulsifying machine, a high-pressure injection emulsifying machine, or the like may be performed.

Alternatively, the lipid composition can also be manufactured by a method in which a lipid-containing solution is subjected to evaporation to dryness using an evaporator under reduced pressure or subjected to spray drying using a spray drier, so that a dried mixture containing a lipid is prepared, and then the mixture is added to an aqueous solvent and further emulsified using the aforementioned emulsifying machine or the like.

Examples of a manufacturing method of a lipid composition containing a nucleic acid include a method including
a step (a) of dissolving constituent components of a lipid composition containing the compound according to the embodiment of the present invention in an organic solvent to obtain an oil phase, and dissolving a nucleic acid in an aqueous solvent to obtain a water phase;
a step (b) of mixing the oil phase and the water phase obtained in the step (a) to obtain a dispersion liquid of lipid particles;
a step (c) of diluting the dispersion liquid of the lipid particles obtained in the step (b);
a step (d) of removing the organic solvent from the dispersion liquid of the lipid particles; and
a step (e) of adjusting the concentration of the dispersion liquid of the lipid particles.

In the step (a), the constituent components of the lipid composition containing the compound according to the embodiment of the present invention are dissolved in an organic solvent (an alcohol such as ethanol, an ester, or the like). The total lipid concentration is not particularly limited, but is generally 1 mmol/L to 100 mmol/L, preferably 5 mmol/L to 50 mmol/L, and more preferably 10 mmol/L to 30 mmol/L.

The water phase can be obtained by dissolving a nucleic acid (for example, siRNA, mRNA, an antisense nucleic acid, or the like) in water or a buffer solution. The concentration of the nucleic acid is not particularly limited, but is preferably 1 to 1,000 µg/mL and more preferably 10 to 500 µg/mL. A component such as a buffer component or an antioxidant for pH adjustment can be added as necessary. The pH of the water phase is preferably 2.0 to 7.0 and more preferably 3.0 to 6.0. Acetic acid, citric acid, malic acid, phosphoric acid, MES, HEPES, or the like is preferably used as a buffer component for adjusting the pH, and as necessary, salts such as sodium chloride and potassium chloride may be added for the purpose of adjusting a ionic strength, or sugars or sugar alcohols, such as sucrose, trehalose, and mannitol may be added for the purpose of adjusting an osmotic pressure.

In the step (b), the oil phase and the water phase may be mixed by any method, and a batch type or an in-line method using a channel device may be used. As the in-line method, a microchannel device is preferably used, and as the microchannel device to be used, a Y-shaped mixer, a T-shaped mixer, a herringbone mixer, a ring micromixer, an impingement jet mixer, or the like can be used. The mixing ratio (volume ratio) of water phase:oil phase is preferably 5:1 to 1:1 and more preferably 4:1 to 2:1.

In the step (c), by mixing the dispersion liquid of lipid particles with the dilution solution, the content of the organic solvent can be reduced and the lipid particles can be stabilized. The dilution solution may be water, but the pH or ionic strength may be adjusted with the dilution solution. The component contained in the dilution solution can be optionally selected depending on the purpose. For example, for the purpose of adjusting the pH, a buffer solution (for example, a citrate buffer solution, a citrate buffered physiological saline, an acetate buffer solution, an acetate buffered physiological saline, a phosphate buffered physiological saline, a Tris buffer solution, an MES buffer solution, a HEPES buffer solution, or the like) may be used. In addition, sodium chloride, potassium chloride, sucrose, trehalose, fructose, mannitol, or the like may be contained for the purpose of adjusting the ionic strength or the osmotic pressure, and a buffer solution to which these additives are further added can also be used.

The dispersion liquid of lipid particles and the dilution solution may be mixed by any method, and a batch type or an in-line method using a channel device may be used. As the channel device used at the time of mixing, a Y-shaped mixer, a T-shaped mixer, or the like can be used. In addition, the time from mixing the oil phase with the water phase to mixing the dilution solution therewith is not particularly limited, but the dilution is preferably performed within 30 seconds and more preferably performed within 10 seconds after mixing the oil phase with the water phase.

The mixing ratio (liquid amount ratio) of the dispersion liquid of lipid particles to the dilution solution is preferably 1:0.5 to 1:10 and more preferably 1:1 to 1:5.

In some embodiments, in the step (c), the dispersion liquid of lipid particles may be mixed with the dilution solution a plurality of times depending on the purpose. In addition, the dilution solutions to be used may be the same as or different from each other. In the dispersion liquid of lipid particles, the particle diameter of the lipid particles may change depending on the pH, and thus the adjustment of the pH of the dispersion liquid is important. Therefore, for example, to adjust the pH of the dispersion liquid of lipid particles after mixing with the dilution solution, a buffer solution having a concentration and a pH suitable for the adjustment or a buffer solution further containing other components may be used.

Furthermore, the plurality of dilution steps may be continuously performed, and the interval between the dilution step and the next dilution step can be optionally set, and for example, the interval may be 10 seconds, 30 seconds, 1 minute, 5 minutes, 10 minutes, 30 minutes, 1 hour, 2 hours, 3 hours, 4 hours, 6 hours, 12 hours, or 24 hours.

In addition, the pH of the dispersion liquid of lipid particles after performing the step (c) is preferably 3.0 to 10.0, more preferably 3.5 to 9.0, and particularly preferably 4.0 to 8.5.

The lipid composition can be subjected to sizing if necessary. The method of sizing is not particularly limited, and the particle size can be reduced by using an extruder or the like.

In addition, the dispersion liquid containing the lipid composition according to the embodiment of the present invention can be frozen or freeze-dried by a general method.

In the step (d), a method of removing the organic solvent from the dispersion liquid of lipid particles is not particularly limited, and a general method can be used. For example, a pH buffer solution such as phosphate buffered physiological saline or a Tris buffer solution can be used as the dialysate, and additives such as any salt or sugar can be added as necessary for the purpose of adjusting the osmotic pressure or protecting the dialysate from freezing.

In the step (e), the concentration of the dispersion liquid of lipid particles obtained in the step (d) can be adjusted. In a case of diluting the dispersion liquid, the dispersion liquid can be diluted to an appropriate concentration by using a solution such as phosphate buffered physiological saline, physiological saline, a Tris buffer solution, or a sucrose-containing Tris buffer solution as a dilution solution. In a case of concentrating the dispersion liquid, the dispersion liquid obtained in the step (d) can be concentrated by ultrafiltration using an ultrafiltration membrane, or the like. It is preferable to use the concentrated dispersion as it is. Alternatively, it is preferable to adjust the concentrated dispersion liquid to a desired concentration by using the aforementioned diluent after the concentration.

In addition, in some embodiments, the organic solvent removal step (step (d)) and the concentration adjustment step (step (e)) can be continuously performed using tangential flow filtration (TFF). In the present step, the organic solvent removal step and the concentration adjustment step may be performed in any order. The organic solvent removal step and the concentration adjustment step may be each performed a plurality of times as necessary.

As the solution that can be used in the dialysis in the step (d) or the dilution in the step (e), an excipient, a freeze protective agent, a buffering agent, or an antioxidant may be added. The excipient or the freeze protective agent is not particularly limited, and examples thereof include sugars and sugar alcohols. Examples of the sugars include sucrose, trehalose, maltose, glucose, lactose, fructose, and the like, and examples of the sugar alcohols include mannitol, sorbitol, inositol, xylitol, and the like. The buffering agent is not particularly limited, and examples thereof include ACES, BES, bicine, CAPS, CHES, DIPSO, EPPS, HEPES, HEPPSO, MES, MOPS, MOPSO, TAPS, TAPSO, TES, tricine, tris, phosphoric acid, acetic acid, citric acid, and the like. Examples of the antioxidant include EDTA, ascorbic acid, tocopherol, and the like.

To make a pharmaceutical composition of the dispersion liquid of lipid particles of the present invention, it is preferable to perform sterile filtration. As a filtration method, a hollow fiber membrane, a reverse osmosis membrane, a membrane filter, or the like can be used to remove insoluble substances from the dispersion liquid of lipid particles. In the present invention, the filtration method is not particularly limited, but it is preferable to filter the dispersion liquid through a filter having a pore diameter capable of sterilization (preferably a filtration sterilization filter with a pore diameter of 0.2 µm). Furthermore, it is preferable that the sterile filtration be performed after Step (d) or Step (e).

In addition, if necessary, the dispersion liquid of lipid particles of the present invention can be frozen or freeze-dried. The dispersion liquid of the lipid particles of the present invention can be frozen or freeze-dried by a general method, and the method is not particularly limited.

### <Regarding lipid composition>

In the present invention, the lipid composition may be lipid particles. The lipid particle means a particle composed of a lipid and includes a composition having any of structures selected from a lipid aggregate in which lipids are aggregated, a micelle, a liposome, a lipid nanoparticle (LNP), or a lipoplex, but the structure of the lipid particle is not limited to these structures as long as the lipid particle is a composition containing a lipid. The liposome has a lipid bilayer structure and has a water phase in the inside, and includes a liposome which has a single bilayer membrane, and a multilayer liposome which has multiple layers stacked together. The present invention may include any of these liposomes.

The form of the lipid particles can be checked by electron microscopy, structural analysis using X-rays, or the like. For example, by a method using Cryo transmission electron microscopy (CryoTEM method), it is possible to check, for example, whether or not a lipid particle is, such as a liposome, a bimolecular lipid membrane structure (lamella structure) and a structure having an inner water layer, and whether or not a lipid particle has a structure having an inner core with a high electron density and packed with constituent components including a lipid. The X-ray small angle scattering (SAXS) analysis also makes it possible to check whether or not a lipid particle has a bimolecular lipid membrane structure (lamella structure).

The particle size of the lipid particles according to the embodiment of the present invention is not particularly limited, and is preferably 10 to 1,000 nm, more preferably 30 to 500 nm, and even more preferably 50 to 250 nm. The particle size of the lipid particles can be measured by a general method (for example, a dynamic light scattering method, a laser diffraction method, or the like).

### <Use of lipid composition>

As an example of the use of the lipid composition according to the embodiment of the present invention, a nucleic acid, a protein, a peptide, or a low-molecular-weight compound can be introduced into a cell by introducing a lipid composition containing a nucleic acid, a protein, a peptide, or a low-molecular-weight compound into the cell. In addition, in a case where the lipid composition according to the embodiment of the present invention contains a nucleic acid, a protein, a peptide, or a low-molecular-weight compound having a pharmaceutical use application, the lipid composition can be administered to a living body as a pharmaceutical composition.

In addition, the lipid composition may be a lipid composition containing only lipid components without nucleic acids, proteins, peptides, or low-molecular-weight compounds. A lipid composition containing a nucleic acid, a protein, a peptide, or a low-molecular-weight compound can also be obtained by preparing a lipid composition containing only a lipid component and then mixing the lipid composition with a nucleic acid, a protein, a peptide, or a low-molecular-weight compound.

In a case where the lipid composition in the present invention is used as a pharmaceutical composition, the lipid composition according to the embodiment of the present invention can be administered to a living body singly or by being mixed with a pharmaceutically acceptable dosing medium (for example, physiological saline or a phosphate buffer solution).

The concentration of the lipid composition in the mixture with a pharmaceutically acceptable carrier is not particularly limited, and can be set to 0.05% by mass to 90% by mass in general. In addition, other pharmaceutically acceptable additives, for example, a pH adjusting buffer and an osmotic pressure adjusting agent, may be added to the pharmaceutical composition containing the lipid composition according to the embodiment of the present invention.

The route of administration when the pharmaceutical composition containing the lipid composition according to the embodiment of the present invention is administered is not particularly limited. The nucleic acid drug can be administered by any method. Examples of the administration method include oral administration and parenteral administration (intraarticular administration, intravenous administration, intraarterial administration, subcutaneous administration, intracutaneous administration, intravitreal administration, intraperitoneal administration, intramuscular administration, intravaginal administration, intravesical administration, intrathecal administration, pulmonary administration, rectal administration, colonic administration, buccal administration, nasal administration, intracisternal administration, inhalation, and the like). Parenteral administration is preferable. As the method of administration, intravenous injection, subcutaneous injection, intracutaneous injection, or intramuscular injection is preferable. The pharmaceutical composition containing the lipid composition according to the embodiment of the present invention can also be administered by being directly injected into the affected area.

The dosage form of the lipid composition according to the embodiment of the present invention is not particularly limited. In a case of performing oral administration, the lipid composition according to the embodiment of the present invention can be used in the form of tablets, troches, capsules, pills, suspension, syrup, or the like by being combined with an appropriate excipient. In addition, pharmaceutical preparation suitable for parenteral administration can appropriately contain an antioxidant, a buffering agent, a bacteriostat, and additives such as an isotonic sterile injection, a suspending agent, a solubilizer, a thickener, a stabilizer, or a preservative.

### <Delivery carrier>

The lipid composition according to the embodiment of the present invention can retain a nucleic acid at a high encapsulation rate and thereby is extremely useful as a delivery carrier for a nucleic acid. According to the delivery carrier using the present invention, the nucleic acid and the like can be introduced into the cells, for example, by mixing the obtained lipid composition with the nucleic acid and the like and transfecting the mixture in vitro or in vivo. In addition, the delivery carrier using the present invention is also useful as a nucleic acid delivery carrier in nucleic acid drugs. That is, the lipid composition according to the embodiment of the present invention is useful as a composition for nucleic acid delivery in vitro, ex vivo, or in vivo (preferably in vivo).

Next, the present invention is described based on examples, but the present invention is not limited thereto.

### Examples

Unless otherwise specified, in purification by column chromatography, the automatic purification device ISOLERA (Biotage AB, Inc.), the medium pressure preparative purification device Purif-espoir-2 (Shoko Science Co., Ltd.), or the medium pressure liquid chromatograph YFLC W-prep 2XY (Yamazen Corporation) was used.

Unless otherwise specified, as a carrier in silica gel column chromatography, Chromatorex Q-Pack SI 50 (FUJI SILYSIA CHEMICAL LTD.), or high flash column WO01, WO02, WO03, WO04, or WO05 (Yamazen Corporation) was used.

For NH silica gel, Chromatorex Q-Pack NH 60 (FUJI SILYSIA CHEMICAL LTD.) was used.

NMR spectra were measured using a Bruker AVNEO400 (manufactured by Bruker Corporation) and using tetramethylsilane as an internal standard, and all δ values were shown in ppm.

MS spectra were measured using an ACQUITY SQD LC/MS System (manufactured by Waters Corporation).

### [Comparative Example 1]

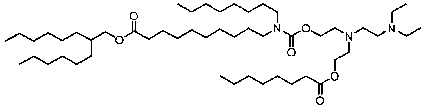

The synthesis was performed according to Example (2-hexyloctyl 3-ethyl-6-(2-(octanoyloxy)ethyl)-11-octyl-10-oxo-9-oxa-3,6,11-triazahenicosan-21-oate) described in WO2020/246581A.

### [Example 1]

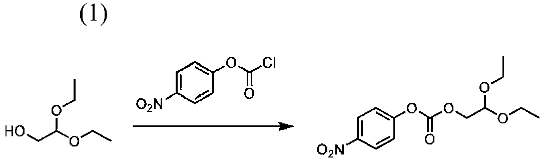

4-Nitrophenyl chloroformate (11.3 g) was added in two divided portions to a mixture of 2,2-diethoxyethanol (5.0 g), tetrahydrofuran (25 mL), and triethylamine (15.6 mL) under ice cooling, and the mixture was stirred under ice cooling for 1 hour. Water (25 mL) and hexane (25 mL) were added to the reaction mixture under ice cooling, and the organic layer was separated. The obtained organic layer was washed with water (25 mL) and saturated saline, and anhydrous sodium sulfate was then added thereto to dry the organic layer, and the solvent was distilled off under reduced pressure. The obtained residue was purified by silica gel column chromatography (ethyl acetate-hexane) to obtain 2,2-diethoxyethyl(4-nitrophenyl) carbonate (12.2 g) as a light yellow oily substance.

¹H-NMR (CDCl₃) δ: 8.30-8.26 (2H, m), 7.41-7.37 (2H, m), 4.79 (1H, t, J = 5.3 Hz), 4.28 (2H, d, J = 5.3 Hz), 3.80-3.72 (2H, m), 3.66-3.58 (2H, m), 1.26 (6H, t, J = 7.0 Hz).

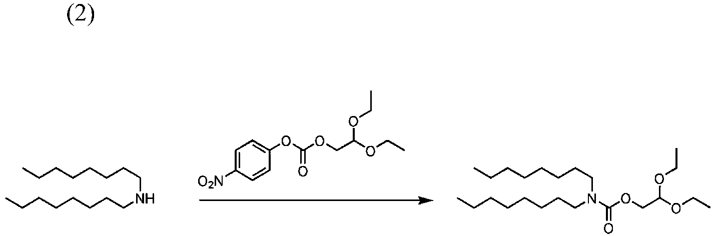

A mixture of 2,2-diethoxyethyl(4-nitrophenyl) carbonate (1.0 g), acetonitrile (5 mL), dioctylamine (0.84 g), and triethylamine (0.93 mL) was stirred at 60°C for 5 hours. Ethyl acetate (5 mL), hexane (5 mL), and water were added to the reaction mixture cooled to room temperature, and the organic layer was separated. The obtained organic layer was washed with water (25 mL) and saturated saline, and anhydrous sodium sulfate was then added thereto to dry the organic layer, and the solvent was distilled off under reduced pressure. The obtained residue was purified by silica gel column chromatography (ethyl acetate-hexane) to obtain 2,2-diethoxyethyl N,N-dioctylcarbamate (1.1 g) as a light yellow oily substance.

¹H-NMR (CDCl₃) δ: 24.69 (1H, t, J = 5.5 Hz), 4.08 (2H, d, J = 5.5 Hz), 3.75-3.67 (2H, m), 3.61-3.53 (2H, m), 3.22-3.15 (4H, m), 1.55-1.47 (4H, m), 1.33-1.20 (26H, m), 0.90-0.85 (6H, m).

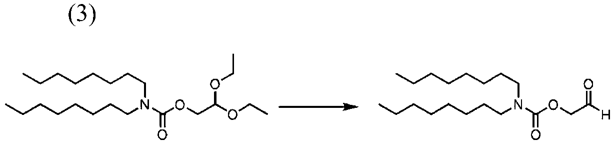

A mixture of 2,2-diethoxyethyl N,N-dioctylcarbamate (1.0 g), formic acid (10 mL), and water (2.5 mL) was stirred at 50°C for 4 hours, toluene was then added thereto, and the solvent was distilled off under reduced pressure. The operation of adding toluene again and distilling off the solvent under reduced pressure was repeated twice to obtain 2-oxoethyl N,N-dioctylcarbamate (0.98 g) as a crude product of a light yellow oily substance.

¹H-NMR (CDCl₃) δ: 9.63 (1H, s), 4.63 (2H, s), 3.27-3.21 (4H, m), 1.61-1.48 (4H, m), 1.32-1.22 (20H, m), 0.90-0.86 (6H, m).

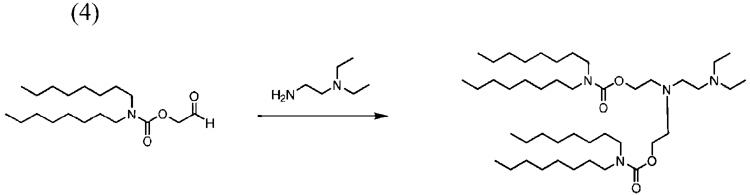

N,N-Diethylethylenediamine (0.145 g), acetic acid (74 mg), and sodium triacetoxyborohydride (1.58 g) were added to an ethyl acetate (8 mL) solution of 2-oxoethyl N,N-dioctylcarbamate (0.815 g) at room temperature, and the mixture was stirred at room temperature for 5 hours. A 20% aqueous potassium carbonate solution (27 mL) was added to the reaction mixture, the organic layer was then separated, washed with water and saturated saline, anhydrous sodium sulfate was then added thereto to dry the organic layer, and the solvent was distilled off under reduced pressure. The obtained residue was purified by silica gel column chromatography (methanol-ethyl acetate-hexane) to obtain ((2-(diethylamino)ethyl)azanediyl)bis(ethan-2,1-diyl)bis(dioctylcarbamate) (0.574 g) as a colorless oily substance.

¹H-NMR (CDCl₃) δ: 4.11 (4H, t, J = 6.3 Hz), 3.23-3.11 (8H, m), 2.80 (4H, t, J = 6.3 Hz), 2.68-2.64 (2H, m), 2.55-2.49 (6H, m), 1.65-1.43 (8H, m), 1.34-1.18 (40H, m), 1.01 (6H, t, J = 7.1 Hz), 0.90-0.85 (12H, m).

MS m/z (M + H): 740.

### [Example 2]

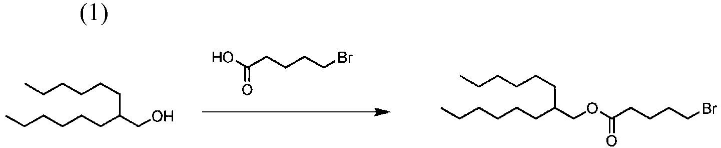

Sulfuric acid (0.5 mL) was added to a toluene (25 mL) mixture of 2-hexyl-1-octanol (5.0 g) and 5-bromovaleric acid (4.6 g), and the mixture was stirred at 110°C for 5 hours. The reaction mixture was cooled to room temperature and then purified by silica gel column chromatography (ethyl acetate-hexane) to obtain 2-hexyloctyl 5-bromopentanoate (8.2 g) as a colorless oily substance.

¹H-NMR (CDCl₃) δ: 3.98 (2H, d, J = 5.7 Hz), 3.42 (2H, t, J = 6.5 Hz), 2.34 (2H, t, J = 7.2 Hz), 1.94-1.87 (2H, m), 1.82-1.74 (2H, m), 1.65-1.57 (1H, m), 1.32-1.23 (20H, m), 0.90-0.86 (6H, m).

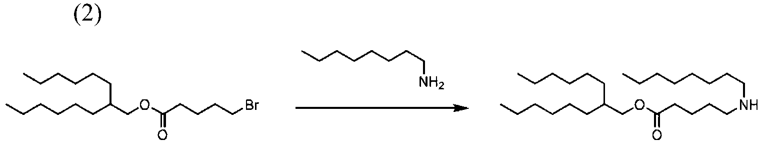

Potassium carbonate (1.3 g) was added to a mixture of 2-hexyloctyl 5-bromopentanoate (1.2 g), n-octylamine (1.2 g), and 1-methyl-2-pyrrolidone (6 mL), and the mixture was stirred at 60°C for 5 hours. The reaction mixture was cooled to room temperature, then ethyl acetate (12 mL) and water (6 mL) were added thereto, and the organic layer was separated. The organic layer was washed with saturated saline, and anhydrous sodium sulfate was then added thereto to dry the organic layer, and the solvent was distilled off under reduced pressure. The obtained residue was purified by silica gel column chromatography (methanol-ethyl acetate-hexane) to obtain 2-hexyloctyl 5-(octylamino)pentanoate (1.25 g) as a light yellow oily substance.

¹H-NMR (CDCl₃) δ: 3.96 (2H, d, J = 5.8 Hz), 2.63-2.52 (4H, m), 2.32 (2H, t, J = 7.4 Hz), 2.06-1.98 (1H, m), 1.70-1.40 (7H, m), 1.34-1.20 (30H, m), 0.90-0.87 (9H, m).

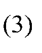

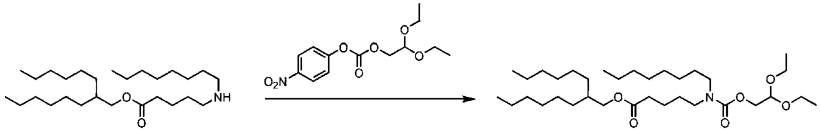

A mixture of 2-hexyloctyl 5-(octylamino)pentanoate (1.25 g), acetonitrile (4 mL), 2,2-diethoxyethyl(4-nitrophenyl) carbonate (0.49 g), and triethylamine (0.46 mL) was stirred at 60°C for 4 hours. Ethyl acetate (4 mL) and water (4 mL) were added to the reaction mixture cooled to room temperature, and the organic layer was separated. The obtained organic layer was washed with water and saturated saline, anhydrous sodium sulfate was then added thereto to dry the organic layer, and the solvent was distilled off under reduced pressure. The obtained residue was purified by silica gel column chromatography (ethyl acetate-hexane) to obtain 2-hexyloctyl 5-(((2,2-diethoxyethoxy)carbonyl)(octyl)amino)pentanoate (0.83 g) as a light yellow oily substance.

¹H-NMR (CDCl₃) δ: 4.69 (1H, t, J = 5.4 Hz), 4.08 (2H, d, J = 5.5 Hz), 3.97 (2H, d, J = 5.7 Hz), 3.74-3.62 (2H, m), 3.60-3.52 (2H, m), 3.26-3.14 (4H, m), 2.36-2.30 (2H, m), 1.65-1.46 (7H, m), 1.33-1.19 (36H, m), 0.90-0.85 (9H, m).

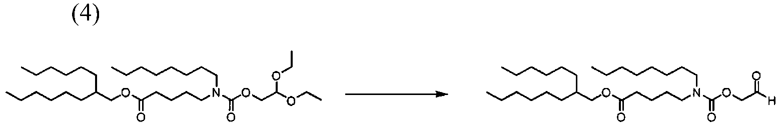

A mixture of 2-hexyloctyl 5-(((2,2-diethoxyethoxy)carbonyl)(octyl)amino)pentanoate (0.83 g), formic acid (6 mL), and water (1.5 mL) was stirred at 50°C for 3 hours, toluene was then added thereto, and the solvent was distilled off under reduced pressure. The operation of adding toluene again and distilling off the solvent under reduced pressure was repeated twice to obtain 2-hexyloctyl 5-(octyl((2-oxoethoxy)carbonyl)amino)pentanoate (0.94 g) as a crude product of a light yellow oily substance.

¹H-NMR (CDCl₃) δ: 4.10 (4H, t, J = 6.3 Hz), 3.97 (4H, d, J = 5.7 Hz), 3.25-3.11 (8H, m), 2.79 (4H, t, J = 6.4 Hz), 2.69-2.63 (2H, m), 2.56-2.47 (6H, m), 2.36-2.29 (4H, m), 1.64-1.49 (14H, m), 1.32-1.21 (60H, m), 1.01 (6H, t, J = 7.0 Hz), 0.90-0.85 (18H, m).

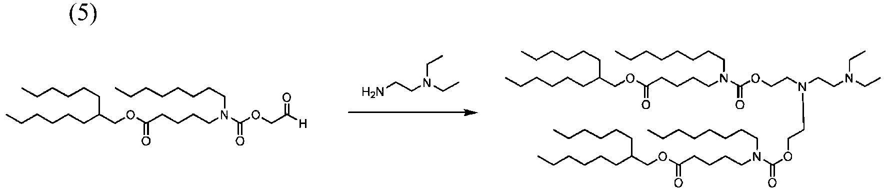

N,N-diethylethylenediamine (0.083 g), acetic acid (43 mg), and sodium triacetoxyborohydride (0.91 g) were added to an ethyl acetate (8 mL) solution of 2-hexyloctyl 5-(octyl((2-oxoethoxy)carbonyl)amino)pentanoate (0.73 g) at room temperature, and the mixture was stirred at room temperature for 5 hours. A 20% aqueous potassium carbonate solution (10 mL) was added to the reaction mixture, the organic layer was then separated and washed with water and saturated saline, anhydrous sodium sulfate was then added thereto to dry the organic layer, and the solvent was distilled off under reduced pressure. The obtained residue was purified by silica gel column chromatography (methanol-ethyl acetate-hexane) to obtain bis(2-hexyloctyl) 11-(2-(diethylamino)ethyl)-6,16-dioctyl-7,15-dioxo-8,14-dioxa-6,11,16-triazahenicosanedicate (0.39 g) as a light yellow oily substance.

¹H-NMR (CDCl₃) δ: 4.10 (4H, t, J = 6.3 Hz), 3.97 (4H, d, J = 5.7 Hz), 3.25-3.11 (8H, m), 2.79 (4H, t, J = 6.4 Hz), 2.69-2.63 (2H, m), 2.56-2.47 (6H, m), 2.36-2.29 (4H, m), 1.64-1.49 (14H, m), 1.32-1.21 (60H, m), 1.01 (6H, t, J = 7.0 Hz), 0.90-0.85 (18H, m).

MS m/z (M + H): 1108.

### [Example 3]

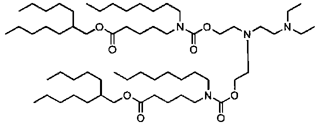

Bis(2-pentylheptyl) 11-(2-(diethylamino)ethyl)-6,16-dioctyl-7,15-dioxo-8,14-dioxa-6,11,16-triazahenicosanedioate as a colorless oily substance was obtained by the same method as that in Example 2, except that 2-pentyl-1-heptanol was used instead of 2-hexyl-1-octanol in (1) of Example 2.

¹H-NMR (CDCl₃) δ: 4.10 (4H, t, J = 6.3 Hz), 3.97 (4H, d, J = 5.8 Hz), 3.26-3.11 (8H, m), 2.79 (4H, t, J = 6.3 Hz), 2.69-2.63 (2H, m), 2.54-2.48 (6H, m), 2.36-2.29 (4H, m), 1.65-1.45 (14H, m), 1.35-1.91 (52H, m), 1.00 (6H, t, J = 7.1 Hz), 0.90-0.86 (18H, m).

MS m/z (M + H): 1052.

### [Example 4]

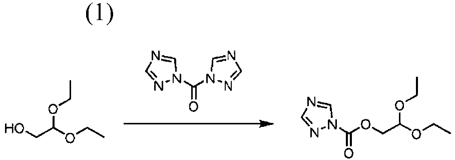

1,1'-Carbonyldi(1,2,4-triazole) (18.3 g) was added to a tetrahydrofuran (100 mL) solution of 2,2-diethoxyethanol (10.0 g), and the mixture was heated at 30°C and stirred for 1 hour. The reaction mixture was cooled to room temperature, then hexane (100 mL) and saturated aqueous sodium bicarbonate (100 mL) were added thereto, and the organic layer was separated. The obtained organic layer was washed with water (50 mL) and saturated saline, anhydrous sodium sulfate was then added thereto to dry the organic layer, and the solvent was distilled off under reduced pressure. The obtained residue was purified by silica gel column chromatography (ethyl acetate-hexane) to obtain 2,2-diethoxyethyl 1H-1,2,4-triazole-1-carboxylate (10.4 g) as a colorless oily substance.

¹H-NMR (CDCl₃) δ: 8.83 (1H, s), 8.09 (1H, s), 4.87 (1H, t, J = 5.3 Hz), 4.49 (2H, d, J = 5.3 Hz), 3.80-3.73 (2H, m), 3.66-3.56 (2H, m), 1.23 (6H, t, J = 7.0 Hz).

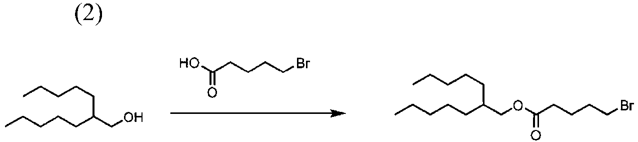

4-Toluenesulfonic acid monohydrate (168 mg) was added to a toluene (30 mL) mixture of 2-pentyl-1-heptanol (6.0 g) and 5-bromovaleric acid (6.4 g) at room temperature, and then the mixture was stirred for 2 hours with heating under reflux while removing water with a Dean-Stark apparatus. The reaction mixture was cooled to room temperature and then purified by silica gel column chromatography (ethyl acetate-hexane) to obtain 2-pentylheptyl 5-bromopentanoate (10.8 g) as a colorless oily substance.

¹H-NMR (CDCl₃) δ: 3.98 (2H, d, J = 5.8 Hz), 3.42 (2H, t, J = 6.6 Hz), 2.35 (2H, t, J = 7.2 Hz), 1.94-1.87 (2H, m), 1.82-1.74 (2H, m), 1.65-1.59 (1H, m), 1.34-1.23 (16H, m), 0.89 (6H, t, J = 6.9 Hz).

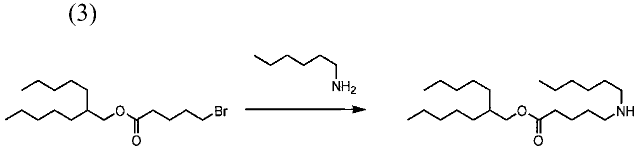

Potassium carbonate (1.45 g) was added to a mixture of 2-pentylheptyl 5-bromopentanoate (1.2 g), n-hexylamine (1.05 g), and acetonitrile (6 mL), and the mixture was stirred at 60°C for 1 hour. The reaction mixture was cooled to room temperature, then ethyl acetate (24 mL) and water (12 mL) were added thereto, and the organic layer was separated. The organic layer was washed with water and saturated saline, anhydrous sodium sulfate was then added thereto to dry the organic layer, and the solvent was distilled off under reduced pressure. The obtained residue was purified by NH silica gel column chromatography (ethyl acetate-hexane) to obtain 2-pentylheptyl 5-(hexylamino)pentanoate (0.864 g) as a light yellow oily substance.

¹H-NMR (CDCl₃) δ: 3.96 (2H, d, J = 5.8 Hz), 2.62-2.55 (4H, m), 2.32 (2H, t, J = 7.4 Hz), 1.70-1.43 (8H, m), 1.34-1.20 (22H, m), 0.90-0.85 (9H, m).

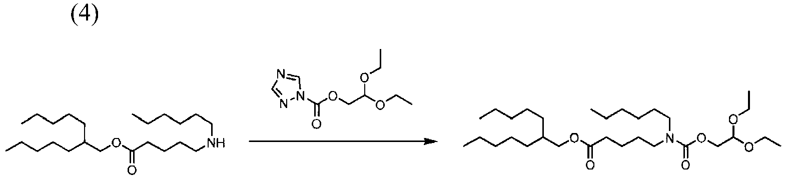

A mixture of 2-pentylheptyl 5-(hexylamino)pentanoate (0.86 g), 2,2-diethoxyethyl 1H-1,2,4-triazole-1-carboxylate (0.53 g), acetonitrile (4.3 mL), triethylamine (0.65 mL), and N,N-dimethylaminopyridine (10 mg) was stirred at 60°C for 2 hours. Ethyl acetate (20 mL) and water (20 mL) were added to the reaction mixture cooled to room temperature, and the organic layer was separated. The obtained organic layer was washed with saturated aqueous ammonium chloride solution and saturated saline, anhydrous sodium sulfate was then added thereto to dry the organic layer, and the solvent was distilled off under reduced pressure. The obtained residue was purified by NH silica gel column chromatography (ethyl acetate-hexane) to obtain 2-pentylheptyl 5-(((2,2-diethoxyethoxy)carbonyl)(hexyl)amino)pentanoate (0.95 g) as a colorless oily substance.

¹H-NMR (CDCl₃) δ: 4.69 (1H, t, J = 5.5 Hz), 4.08 (2H, d, J = 5.5 Hz), 3.97 (2H, d, J = 5.7 Hz), 3.74-3.67 (2H, m), 3.60-3.52 (2H, m), 3.26-3.14 (4H, m), 2.35-2.30 (2H, m), 1.65-1.48 (7H, m), 1.33-1.19 (28H, m), 0.90-0.86 (9H, m).

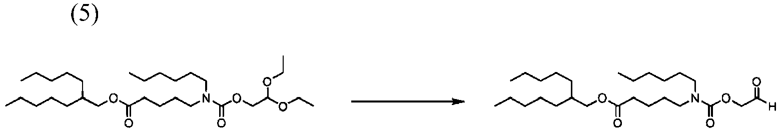

A mixture of 2-pentylheptyl 5-(((2,2-diethoxyethoxy)carbonyl)(hexyl)amino)pentanoate (0.95 g), formic acid (4 mL), and water (1 mL) was stirred at 40°C for 2 hours, toluene was then added thereto, and the mixture was distilled off under reduced pressure. The operation of adding toluene again and distilling off the solvent under reduced pressure was repeated twice to obtain 2-pentylheptyl 5-(hexyl((2-oxoethoxy)carbonyl)amino)pentanoate as a crude product of a light yellow oily substance.

¹H-NMR (CDCl₃) δ: 9.62 (1H, s), 4.63-4.60 (2H, m), 3.97 (2H, d, J = 5.7 Hz), 3.30-3.17 (4H, m), 2.36-2.31 (2H, m), 1.66-1.50 (7H, m), 1.34-1.21 (22H, m), 0.90-0.86 (9H, m).

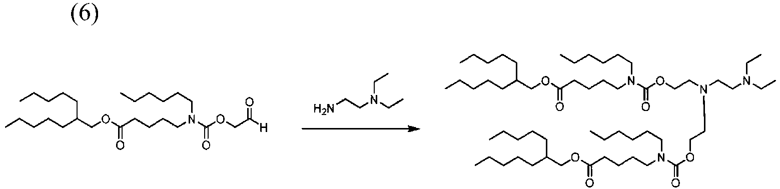

N,N-Diethylethylenediamine (0.127 g) and sodium triacetoxyborohydride (1.14 g) were added to an ethyl acetate (10 mL) solution of the crude product of 2-pentylheptyl 5-(hexyl((2-oxoethoxy)carbonyl)amino)pentanoate of (5) of Example 4 at room temperature, and the mixture was stirred at room temperature for 1 hour. A 10% aqueous potassium carbonate solution (10 mL) was added to the reaction mixture, the organic layer was then separated, and washed with water and saturated saline, anhydrous sodium sulfate was then added thereto to dry the organic layer, and the solvent was distilled off under reduced pressure. The obtained residue was purified by NH silica gel column chromatography (ethyl acetate-hexane) to obtain bis(2-pentylheptyl) 11-(2-(diethylamino)ethyl)-6,16-dihexyl-7,15-dioxo-8,14-dioxa-6,11,16-triazahenicosanedioate (0.67 g) as a light yellow oily substance.

¹H-NMR (CDCl₃) δ: 4.10 (4H, t, J = 6.4 Hz), 3.97 (4H, d, J = 5.8 Hz), 3.25-3.10 (8H, m), 2.79 (4H, t, J = 6.4 Hz), 2.68-2.64 (2H, m), 2.54-2.49 (6H, m), 2.35-2.29 (4H, m), 1.64-1.45 (14H, m), 1.33-1.21 (44H, m), 1.01 (6H, t, J = 7.1 Hz), 0.90-0.86 (18H, m).

MS m/z (M + H): 996.

### [Example 5]

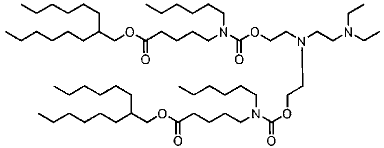

Bis(2-hexyloctyl) 11-(2-(diethylamino)ethyl)-6,16-dihexyl-7,15-dioxo-8,14-dioxa-6,11,16-triazahenicosanedioate as a colorless oily substance was obtained by the same method as that in Example 2, except that n-hexylamine was used instead of n-octylamine in (1) of Example 2.

¹H-NMR (CDCl₃) δ: 4.10 (4H, t, J = 6.3 Hz), 3.97 (4H, d, J = 5.7 Hz), 3.25-3.12 (8H, m), 2.79 (4H, t, J = 6.3 Hz), 2.69-2.64 (2H, m), 2.54-2.49 (6H, m), 2.35-2.29 (4H, m), 1.64-1.45 (14H, m), 1.34-1.20 (52H, m), 1.01 (6H, t, J = 7.1 Hz), 0.90-0.86 (18H, m).

MS m/z (M + H): 1052.

### [Example 6]

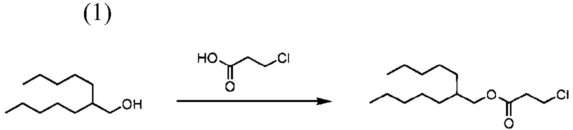

2-Pentylheptyl 3-chloropropanoate as a light yellow oily substance was obtained by the same method as that in (2) of Example 4, except that 3-chloropropionic acid was used instead of 5-bromovaleric acid in (2) of Example 4.

¹H-NMR (CDCl₃) δ: 4.04 (2H, d, J = 5.7 Hz), 3.76 (2H, t, J = 6.7 Hz), 2.80 (2H, t, J = 6.7 Hz), 1.67-1.60 (1H, m), 1.35-1.21 (16H, m), 0.89 (6H, t, J = 6.9 Hz).

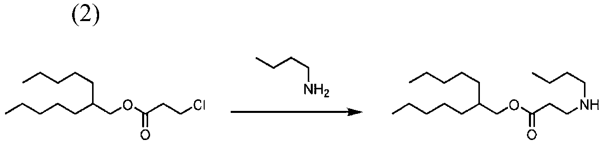

2-Pentylheptyl 3-(butylamino)propanoate as a light yellow oily substance was obtained by the same method as that in (3) of Example 4, except that 2-pentylheptyl 3-chloropropanoate was used instead of 2-pentylheptyl 5-bromopentanoate, n-butylamine was used instead of n-hexylamine, and benzyltriethylammonium bromide was used as an additive in (3) of Example 4.

¹H-NMR (CDCl₃) δ: 3.99 (2H, d, J = 5.7 Hz), 2.88 (2H, t, J = 6.5 Hz), 2.61 (2H, t, J = 7.2 Hz), 2.52 (2H, t, J = 6.5 Hz), 1.65-1.21 (22H, m), 0.93-0.86 (9H, m).

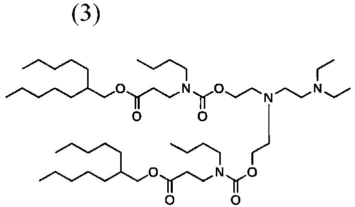

Bis(2-pentylheptyl) 4,14-dibutyl-9-(2-(diethylamino)ethyl)-5,13-dioxo-6,12-dioxa-4,9,14-triazaheptadecanedioate as a colorless oily substance was obtained by the same method as that in Example 2, except that 2-pentylheptyl 3-(butylamino)propanoate was used instead of 2-hexyloctyl 5-(octylamino)pentanoate in (3) of Example 2.

¹H-NMR (CDCl₃) δ: 4.14-4.08 (4H, m), 3.98 (4H, d, J = 5.8 Hz), 3.54-3.45 (4H, m), 3.27-3.16 (4H, m), 2.80 (4H, t, J = 6.4 Hz), 2.67-2.48 (12H, m), 1.66-1.45 (6H, m), 1.36-1.21 (36H, m), 1.01 (6H, t, J = 7.1 Hz), 0.94-0.86 (18H, m).

MS m/z (M + H): 884.

### [Example 7]

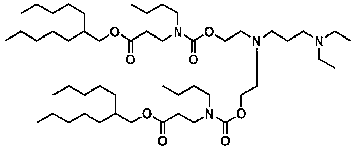

Bis(2-pentylheptyl) 4,14-dibutyl-9-(3-(diethylamino)propyl)-5,13-dioxo-6,12-dioxa-4,9,14-triazaheptadecanedioate as a colorless oily substance was obtained by the same method as that in Example 6, except that N,N-diethyl-1,3-diaminopropane was used instead of N,N-diethylethylenediamine in (3) of Example 6.

¹H-NMR (CDCl₃) δ: 4.14-4.08 (4H, m), 3.98 (4H, d, J = 5.8 Hz), 3.53-3.45 (4H, m), 3.27-3.17 (4H, m), 2.76 (4H, t, J = 6.6 Hz), 2.63-2.48 (10H, m), 2.43-2.38 (2H, m), 1.66-1.45 (8H, m), 1.35-1.21 (36H, m), 1.01 (6H, t, J = 7.1 Hz), 0.94-0.86 (18H, m).

MS m/z (M + H): 898.

### [Example 8]

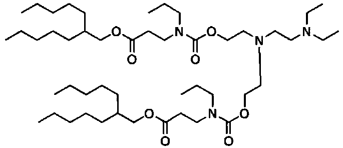

Bis(2-pentylheptyl) 9-(2-(diethylamino)ethyl)-5,13-dioxo-4,14-dipropyl-6,12-dioxa-4,9,14-triazaheptadecanedioate as a colorless oily substance was obtained by the same method as that in Example 6, except that n-propylamine was used instead of n-butylamine in (2) of Example 6.

¹H-NMR (CDCl₃) δ: 4.13-4.09 (4H, m), 3.98 (4H, d, J = 5.8 Hz), 3.54-3.45 (4H, m), 3.24-3.14 (4H, m), 2.80 (4H, t, J = 6.4 Hz), 2.67-2.48 (12H, m), 1.65-1.50 (6H, m), 1.34-1.21 (32H, m), 1.01 (6H, t, J = 7.1 Hz), 0.94-0.85 (18H, m).

MS m/z (M + H): 856.

### [Example 9]

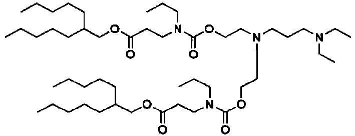

Bis(2-pentylheptyl) 9-(3-(diethylamino)propyl)-5,13-dioxo-4,14-dipropyl-6,12-dioxa-4,9,14-triazaheptadecanedioate as a colorless oily substance was obtained by the same method as that in Example 6, except that n-propylamine was used instead of n-butylamine in (2) of Example 6, and N,N-diethyl-1,3-diaminopropane was used instead of N,N-diethylethylenediamine in (3) of Example 6.

¹H-NMR (CDCl₃) δ: 4.13-4.09 (4H, m), 3.98 (4H, d, J = 5.8 Hz), 3.54-3.44 (4H, m), 3.24-3.14 (4H, m), 2.77 (4H, t, J = 6.4 Hz), 2.63-2.47 (10H, m), 2.44-2.38 (2H, m), 1.65-1.50 (8H, m), 1.35-1.21 (32H, m), 1.00 (6H, t, J = 7.1 Hz), 0.90-0.86 (18H, m).

MS m/z (M + H): 870.

### [Example 10]

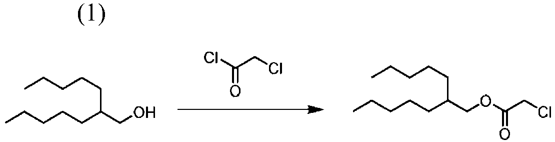

Chloroacetyl chloride (1.82 g) was added to a tetrahydrofuran (20 mL) solution of 2-pentyl-1-heptanol (2.00 g), and pyridine (2.6 mL) was added thereto under ice cooling. The mixture was stirred at room temperature for 20 minutes, water (20 mL) and hexane (10 mL) were added to the reaction mixture, and then the organic layer was separated. The organic layer was washed with water and saturated saline, anhydrous sodium sulfate was then added thereto to dry the organic layer, and the solvent was distilled off under reduced pressure to obtain a crude product of 2-pentylheptyl 2-chloroacetate (3.85 g).

¹H-NMR (CDCl₃) δ: 4.10 (2H, d, J = 5.8 Hz), 4.06 (2H, s), 1.79-1.61 (1H, m), 1.35-1.22 (16H, m), 0.89 (6H, t, J = 7.0 Hz).

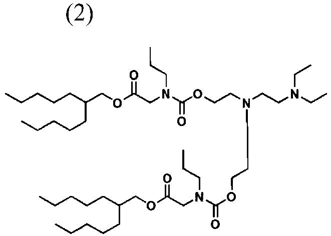

Bis(2-pentylheptyl) 8-(2-(diethylamino)ethyl)-4,12-dioxo-3,13-dipropyl-5,11-dioxa-3,8,13-triazapentadecanedioate as a colorless oily substance was obtained by the same method as that in Example 2, except that 2-pentylheptyl 2-chloroacetate was used instead of 2-hexyloctyl 5-bromopentanoate and n-propylamine was used instead of n-octylamine in (2) of Example 2.

¹H-NMR (CDCl₃) δ: 4.13-4.06 (4H, m), 4.04-3.92 (8H, m), 3.29-3.21 (4H, m), 2.84-2.71 (4H, m), 2.69-2.60 (2H, m), 2.56-2.45 (6H, m), 1.66-1.58 (2H, m), 1.58-1.50 (4H, m), 1.35-1.21 (32H, m), 1.01 (6H, t, J = 7.0 Hz), 0.91-0.86 (18H, m).

MS m/z (M + H): 828.

### [Example 11]

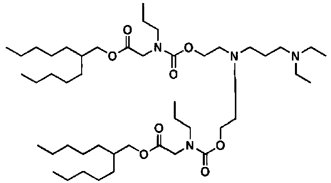

Bis(2-pentylheptyl) 8-(3-(diethylamino)propyl)-4,12-dioxo-3,13-dipropyl-5,11-dioxa-3,8,13-triazapentadecanedioate as a colorless oily substance was obtained by the same method as that in Example 10, except that N,N-diethyl-1,3-diaminopropane was used instead of N,N-diethylethylenediamine in (2) of Example 10.

¹H-NMR (CDCl₃) δ: 4.14-4.06 (4H, m), 4.04-3.92 (8H, m), 3.29-3.21 (4H, m), 2.81-2.68 (4H, m), 2.58-2.47 (6H, m), 2.44-2.37 (2H, m), 1.64-1.49 (8H, m), 1.34-1.21 (32H, m), 1.01 (6H, t, J = 7.1 Hz), 0.91-0.86 (18H, m).

MS m/z (M + H): 842.

### [Example 12]

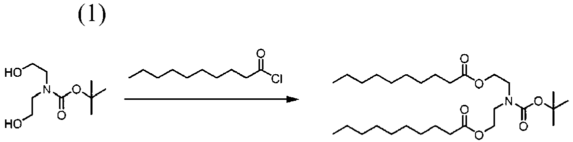

Decanoyl chloride (11 mL) was added dropwise to a tetrahydrofuran (50 mL) solution of tert-butyl N,N-bis(2-hydroxyethyl)carbamate (5.00 g) and triethylamine (8.15 mL) under ice cooling, and the mixture was stirred at room temperature for 4 hours. Hexane (50 mL) and water (50 mL) were added to the reaction mixture, and the organic layer was separated. The obtained organic layer was washed with water (50 mL) and saturated saline, anhydrous sodium sulfate was then added thereto to dry the organic layer, and the solvent was distilled off under reduced pressure to obtain yellow oily ((tert-butoxycarbonyl)azanediyl)bis(ethan-2,1-diyl)bis(decanoate) (13.1 g) as a crude product.

¹H-NMR (CDCl₃) δ: 4.21-4.13 (4H, m), 3.52-3.44 (4H, m), 2.30 (4H, t, J = 7.5 Hz), 1.67-1.55 (4H, m), 1.46 (9H, s), 1.33-1.23 (24H, m), 0.88 (6H, t, J = 6.8 Hz).

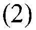

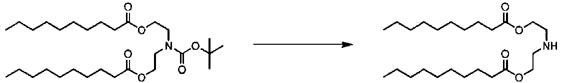

Trifluoroacetic acid (20 mL) was added to a mixture of ((tert-butoxycarbonyl)azanediyl)bis(ethan-2,1-diyl)bis(decanoate) (13.0 g) and water (1 mL) at room temperature, and the mixture was stirred overnight. The mixture was distilled off under reduced pressure, hexane (60 mL), ethyl acetate (30 mL), and a 20% aqueous potassium carbonate solution (40 mL) were added to the residue, and the organic layer was separated. Anhydrous sodium sulfate was added to the obtained organic layer, the organic layer was dried, the solvent was distilled off under reduced pressure, and the residue was purified by silica gel column chromatography (ethyl acetate-hexane) to obtain azanediylbis(ethane-2,1-diyl)bis(decanoate) (7.39 g) as a light yellow oily substance.

MS m/z (M + H): 415.

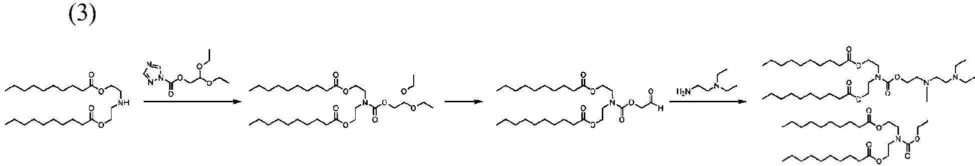

2-(2-(2-(bis(2-decanoyloxyethyl)carbamoyloxy)ethyl-(2-(diethylamino)ethyl)amino)ethoxycarbonyl-(2-decanooyloxyethyl)amino)ethyl decanoate as a colorless oily substance was obtained by the same method as that in (4) of Example 4, (5) of Example 4, and (6) of Example 4, except that azanediylbis(ethan-2,1-diyl)bis(decanate) was used instead of 2-pentylheptyl 5-(hexylamino)pentanoate in (4) of Example 4.

¹H-NMR (CDCl₃) δ: 4.22-4.11 (12H, m), 3.56-3.49 (8H, m), 2.79 (4H, t, J = 6.4 Hz), 2.68-2.63 (2H, m), 2.54-2.47 (6H, m), 2.30 (8H, t, J = 7.5 Hz), 1.64-1.54 (8H, m), 1.33-1.22 (48H, m), 1.01 (6H, t, J = 7.1 Hz), 0.88 (12H, t, J = 6.8 Hz).

MS m/z (M + H): 1084.

### [Example 13]

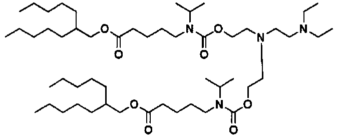

Bis(2-pentylheptyl) 11-(2-(diethylamino)ethyl)-6,16-diisopropyl-7,15-dioxo-8,14-dioxa-6,11,16-triazahenicosanedioate as a colorless oily substance was obtained by the same method as that in Example 4, except that isopropylamine was used instead of n-hexylamine in (3) of Example 4.

¹H-NMR (CDCl₃) δ: 4.32-4.05 (2H, m), 4.11 (4H, t, J = 6.3 Hz), 4.00 (4H, d, J = 5.8 Hz), 3.15-3.02 (4H, m), 2.80 (4H, t, J = 6.4 Hz), 2.68-2.64 (2H, m), 2.54-2.49 (6H, m), 2.32 (4H, t, J = 7.0 Hz), 1.64-1.53 (10H, m), 1.34-1.21 (32H, m), 1.13 (12H, d, J = 6.8 Hz), 1.01 (6H, t, J = 7.1 Hz), 0.90-0.86 (12H, m).

MS m/z (M + H): 912.

### [Example 14]

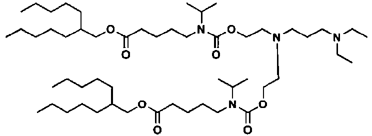

Bis(2-pentylheptyl) 11-(3-(diethylamino)propyl)-6,16-diisopropyl-7,15-dioxo-8,14-dioxa-6,11,16-triazahenicosanedioate as a colorless oily substance was obtained by the same method as that in Example 4, except that isopropylamine was used instead of n-hexylamine in (3) of Example 4 and N,N-diethyl-1,3-diaminopropane was used instead of N,N-diethylethylenediamine in (6) of Example 4.

¹H-NMR (CDCl₃) δ: 4.30-4.05 (2H, m), 4.11 (4H, t, J = 6.4 Hz), 3.97 (4H, d, J = 5.8 Hz), 3.16-3.00 (4H, m), 2.77 (4H, t, J = 6.4 Hz), 2.57-2.47 (6H, m), 2.43-2.39 (2H, m), 2.32 (4H, t, J = 7.0 Hz), 1.66-1.53 (12H, m), 1.35-1.21 (32H, m), 1.13 (12H, d, J = 6.8 Hz), 1.00 (6H, t, J = 7.1 Hz), 0.88 (12H, t, J = 6.9 Hz).

MS m/z (M + H): 926.

### [Example 15]

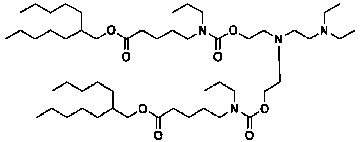

Bis(2-pentylheptyl) 11-(2-(diethylamino)ethyl)-7,15-dioxo-6,16-dipropyl-8,14-dioxa-6,11,16-triazahenicosanedioate as a colorless oily substance was obtained by the same method as that in Example 4, except that n-propylamine was used instead of n-hexylamine in (3) of Example 4.

¹H-NMR (CDCl₃) δ: 4.10 (4H, t, J = 6.3 Hz), 3.97 (4H, d, J = 5.8 Hz), 3.26-3.09 (8H, m), 2.79 (4H, t, J = 6.3 Hz), 2.68-2.64 (2H, m), 2.54-2.49 (6H, m), 2.34-2.31 (4H, m), 1.66-1.49 (14H, m), 1.35-1.21 (32H, m), 1.01 (6H, t, J = 7.1 Hz), 0.90-0.85 (18H, m).

MS m/z (M + H): 912.

### [Example 16]

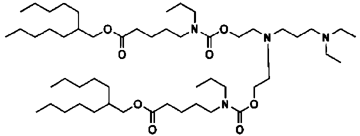

Bis(2-pentylheptyl) 11-(3-(diethylamino)propyl)-7,15-dioxo-6,16-dipropyl-8,14-dioxa-6,11,16-triazahenicosanedioate as a colorless oily substance was obtained by the same method as that in Example 4, except that n-propylamine was used instead of n-hexylamine in (3) of Example 4 and N,N-diethyl-1,3-diaminopropane was used instead of N,N-diethylethylenediamine in (6) of Example 4.

¹H-NMR (CDCl₃) δ: 4.10 (4H, t, J = 6.4 Hz), 3.97 (4H, d, J = 5.8 Hz), 3.26-3.09 (8H, m), 2.76 (4H, t, J = 6.4 Hz), 2.57-2.48 (6H, m), 2.43-2.39 (2H, m), 2.34-2.31 (4H, m), 1.66-1.49 (16H, m), 1.36-1.21 (32H, m), 1.01 (6H, t, J = 7.1 Hz), 0.90-0.84 (18H, m).

MS m/z (M + H): 926.

### [Example 17]

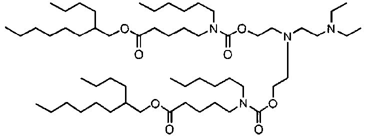

Bis(2-butyloctyl) 11-(2-(diethylamino)ethyl)-6,16-dihexyl-7,15-dioxo-8,14-dioxa-6,11,16-triazahenicosanedioate as a colorless oily substance was obtained by the same method as that in Example 4, except that 2-butyl-1-octanol was used instead of 2-pentyl-1-heptanol in (2) of Example 4.

¹H-NMR (CDCl₃) δ: 4.10 (4H, t, J = 6.4Hz), 3.97 (4H, d, J = 5.8 Hz), 3.25-3.12 (8H, m), 2.80 (4H, t, J = 6.4 Hz), 2.70-2.62 (2H, m), 2.58-2.46 (6H, m), 2.34-2.31 (4H, m), 1.65-1.45 (14H, m), 1.35-1.21 (44H, m), 1.07-0.97 (6H, m), 0.90-0.86 (18H, m).

MS m/z (M + H): 997.

### [Example 18]

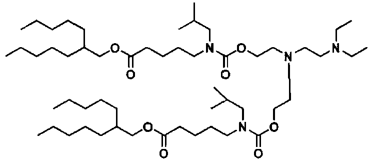

Bis(2-pentylheptyl) 11-(2-(diethylamino)ethyl)-6,16-diisobutyl-7,15-dioxo-8,14-dioxa-6,11,16-triazahenicosanedioate as a colorless oily substance was obtained by the same method as that in Example 4, except that isobutylamine was used instead of n-hexylamine in (3) of Example 4.

¹H-NMR (CDCl₃) δ: 4.13-4.07 (4H, m), 3.97 (4H, d, J = 5.8 Hz), 3.26-3.09 (4H, m), 3.06-2.99 (4H, m), 2.79 (4H, t, J = 6.3 Hz), 2.68-2.64 (2H, m), 2.54-2.49 (6H, m), 2.35-2.30 (4H, m), 1.95-1.85 (2H, m), 1.65-1.51 (10H, m), 1.35-1.21 (32H, m), 1.01 (6H, t, J = 7.1 Hz), 0.90-0.85 (24H, m).

MS m/z (M + H): 940.

### [Example 19]

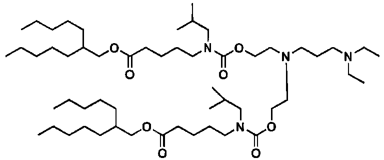

Bis(2-pentylheptyl) 11-(3-(diethylamino)propyl)-6,16-diisobutyl-7,15-dioxo-8,14-dioxa-6,11,16-triazahenicosanedioate as a colorless oily substance was obtained by the same method as that in Example 4, except that isobutylamine was used instead of n-hexylamine in (3) of Example 4 and N,N-diethyl-1,3-diaminopropane was used instead of N,N-diethylethylenediamine in (6) of Example 4.

¹H-NMR (CDCl₃) δ: 4.13-4.07 (4H, m), 3.97 (4H, d, J = 5.8 Hz), 3.26-3.12 (4H, m), 3.05-2.99 (4H, m), 2.76 (4H, t, J = 6.4 Hz), 2.57-2.48 (6H, m), 2.43-2.39 (2H, m), 2.35-2.30 (4H, m), 1.95-1.84 (2H, m), 1.65-1.51 (12H, m), 1.35-1.21 (32H, m), 1.01 (6H, t, J = 7.1 Hz), 0.90-0.85 (24H, m).

MS m/z (M + H): 954.

### [Example 20]

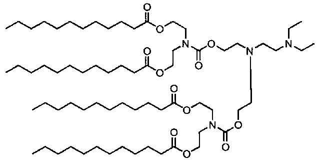

2-(2-(2-(bis(2-dodecanoyloxyethyl)carbamoyloxy)ethyl-(2-(diethylamino)ethyl)amino)ethoxycarbonyl-(2-dodecanoyloxyethyl)amino)ethyl dodecanoate as a colorless oily substance was obtained by the same method as that in Example 12, except that dodecanoyl chloride was used instead of decanoyl chloride in (1) of Example 12.
¹H-NMR (CDCl₃) δ: 4.22-4.11 (12H, m), 3.56-3.49 (8H, m), 2.79 (4H, t, J = 6.4 Hz), 2.68-2.63 (2H, m), 2.55-2.48 (6H, m), 2.30 (8H, t, J = 7.5 Hz), 1.64-1.54 (8H, m), 1.33-1.22 (64H, m), 1.01 (6H, t, J = 7.1 Hz), 0.88 (12H, t, J = 6.8 Hz).

### [Example 21]

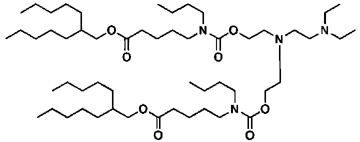

Bis(2-pentylheptyl) 6,16-dibutyl-11-(2-(diethylamino)ethyl)-7,15-dioxo-8,14-dioxa-6,11,16-triazahenicosanedioate as a colorless oily substance was obtained by the same method as that in Example 4, except that n-butylamine was used instead of n-hexylamine in (3) of Example 4.

¹H-NMR (CDCl₃) δ: 4.10 (4H, t, J = 6.3 Hz), 3.97 (4H, d, J = 5.8 Hz), 3.25-3.13 (8H, m), 2.79 (4H, t, J = 6.4 Hz), 2.68-2.64 (2H, m), 2.55-2.49 (6H, m), 2.34-2.31 (4H, m), 1.66-1.44 (14H, m), 1.35-1.20 (36H, m), 1.01 (6H, t, J = 7.1 Hz), 0.92-0.87 (18H, m).

MS m/z (M + H): 940.

### [Example 22]

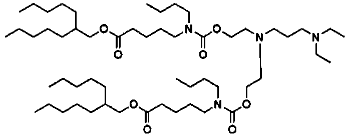

Bis(2-pentylheptyl) 6,16-dibutyl-11-(3-(diethylamino)propyl)-7,15-dioxo-8,14-dioxa-6,11,16-triazahenicosanedioate as a colorless oily substance was obtained by the same method as that in Example 4, except that n-butylamine was used instead of n-hexylamine in (3) of Example 4 and N,N-diethyl-1,3-diaminopropane was used instead of N,N-diethylethylenediamine in (6) of Example 4.

¹H-NMR (CDCl₃) δ: 4.10 (4H, t, J = 6.4 Hz), 3.97 (4H, d, J = 5.8 Hz), 3.24-3.13 (8H, m), 2.76 (4H, t, J = 6.4 Hz), 2.55-2.48 (6H, m), 2.43-2.40 (2H, m), 2.34-2.31 (4H, m), 1.66-1.44 (16H, m), 1.35-1.20 (36H, m), 1.00 (6H, t, J = 7.1 Hz), 0.93-0.87 (18H, m).

MS m/z (M + H): 954.

### [Example 23]

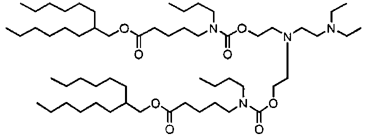

Bis(2-hexyloctyl) 6,16-dibutyl-11-(2-(diethylamino)ethyl)-7,15-dioxo-8,14-dioxa-6,11,16-triazahenicosanedioate as a colorless oily substance was obtained by the same method as that in Example 4, except that 2-hexyl-1-octanol was used instead of 2-pentyl-1-heptanol in (2) of Example 4 and n-butylamine was used instead of n-hexylamine in (3) of Example 4.

¹H-NMR (CDCl₃) δ: 4.10 (4H, t, J = 6.3 Hz), 3.96 (4H, d, J = 5.7 Hz), 3.27-3.12 (8H, m), 2.79 (4H, t, J = 6.3 Hz), 2.72-2.61 (2H, m), 2.60-2.44 (6H, m), 2.32 (4H, t, J = 6.2 Hz), 1.66-1.44 (14H, m), 1.36-1.20 (44H, m), 1.08-0.96 (6H, m), 0.95-0.83 (18H, m).

MS m/z (M + H): 996.

### [Example 24]

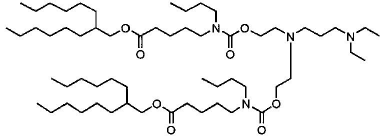

Bis(2-hexyloctyl) 6,16-dibutyl-11-(3-(diethylamino)propyl)-7,15-dioxo-8,14-dioxa-6,11,16-triazahenicosanedioate as a colorless oily substance was obtained by the same method as that in Example 4, except that 2-hexylo-1-octanol was used instead of 2-pentyl-1-heptanol in (2) of Example 4, n-butylamine was used instead of n-hexylamine in (3) of Example 4, and N,N-diethyl-1,3-diaminopropane was used instead of N,N-diethylethylenediamine in (6) of Example 4.

¹H-NMR (CDCl₃) δ: 4.09 (4H, t, J = 6.4 Hz), 3.96 (4H, d, J = 5.8 Hz), 3.26-3.12 (8H, m), 2.76 (4H, t, J = 6.4 Hz), 2.66-2.36 (8H, m), 2.32 (4H, t, J = 6.2 Hz), 1.66-1.43 (16H, m), 1.36-1.18 (44H, m), 1.08-0.95 (6H, m), 0.95-0.83 (18H, m).

MS m/z (M + H): 1010.

### [Example 25]

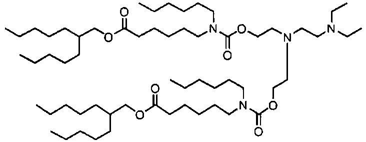

Bis(2-pentylheptyl) 12-(2-(diethylamino)ethyl)-7,17-dihexyl-8,16-dioxo-9,15-dioxa-7,12,17-triazatricosanedioate as a colorless oily substance was obtained by the same method as that in Example 4, except that 6-bromohexanoic acid was used instead of 5-bromovaleric acid in (2) of Example 4.

¹H-NMR (CDCl₃) δ: 4.10 (4H, t, J = 6.4 Hz), 3.96 (4H, d, J = 5.7 Hz), 3.25-3.10 (8H, m), 2.79 (4H, t, J = 6.2 Hz), 2.72-2.61 (2H, m), 2.58-2.43 (6H, m), 2.30 (4H, t, J = 7.4 Hz), 1.69-1.44 (14H, m), 1.36-1.19 (48H, m), 1.07-0.96 (6H, m), 0.93-0.82 (18H, m).

MS m/z (M + H): 1024.

### [Example 26]

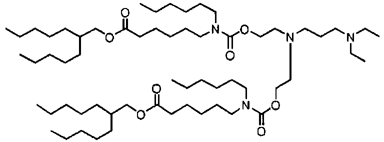

Bis(2-pentylheptyl) 12-(3-(diethylamino)propyl)-7,17-dihexyl-8,16-dioxo-9,15-dioxa-7,12,17-triazatricosanedioate as a colorless oily substance was obtained by the same method as that in Example 4, except that 6-bromohexanoic acid was used instead of 5-bromovaleric acid in (2) of Example 4 and N,N-diethyl-1,3-diaminopropane was used instead of N,N-diethylethylenediamine in (6) of Example 4.

¹H-NMR (CDCl₃) δ: 4.09 (4H, t, J = 6.4 Hz), 3.96 (4H, d, J = 5.8 Hz), 3.24-3.08 (8H, m), 2.76 (4H, t, J = 6.4 Hz), 2.64-2.35 (8H, m), 2.30 (4H, t, J = 7.4 Hz), 1.68-1.44 (16H, m), 1.35-1.18 (48H, m), 1.09-0.95 (6H, m), 0.93-0.84 (18H, m).

MS m/z (M + H): 1038.

### [Example 27]

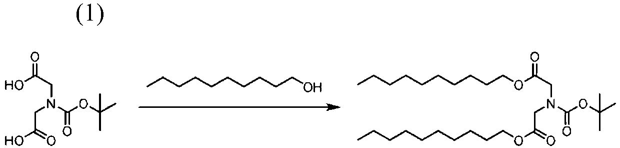

1-Decanol (2.71 g), triethylamine (5.98 mL), and N,N-dimethylaminopyridine (1.05 g) were added to a dichloromethane (20 mL) mixture of N-(tert-butoxycarbonyl)iminodiacetic acid (2.00 g) and 1-ethyl-3-(3-dimethylaminopropyl)carbodiimide hydrochloride (1.97 g) at room temperature, and the mixture was stirred for 10 minutes. 1-Ethyl-3-(3-dimethylaminopropyl)carbodiimide hydrochloride (1.97 g) was added to the reaction mixture, and the mixture was stirred at 40°C for 6 hours. Water (20 mL) was added to the reaction mixture, and the organic layer was separated. Ethyl acetate (20 mL) was added to the aqueous layer, the organic layer was washed with saturated saline, combined with the organic layer obtained above, anhydrous sodium sulfate was added thereto to dry the organic layer, and the solvent was distilled off under reduced pressure. The residue was purified by silica gel column chromatography (ethyl acetate-hexane) to obtain didecyl 2,2'-((tert-butoxycarbonyl)azanediyl)diacetate (3.70 g) as a light yellow oily substance.

¹H-NMR (CDCl₃) δ: 4.16-3.98 (8H, m), 1.68-1.54 (8H, m), 1.44 (9H, s), 1.37-1.23 (24H, m), 0.88 (6H, t, J = 6.8 Hz).

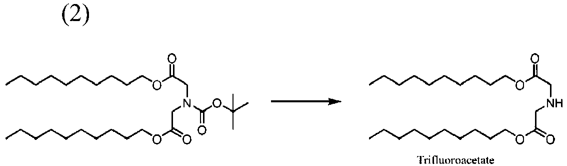

Trifluoroacetic acid (3.0 mL) was added to a mixture of didecyl 2,2'-((tert-butoxycarbonyl)azanediyl)diacetate (2.00 g), toluene (1.0 mL), and water (0.15 mL) under ice cooling, the mixture was stirred at 30°C for 4 hours, and the solvent was distilled off under reduced pressure. Toluene (20 mL) was added to the residue, the operation of distilling off the residue under reduced pressure was repeated three times, hexane (30 mL) was added to the obtained residue, and the mixture was stirred. The precipitated solid was collected by filtration to obtain a trifluoroacetate of didodecyl 2,2'-azanediyldiacetate (2.15 g) as a white solid.

¹H-NMR (CDCl₃) δ: 5.24 (2H, brs), 4.21 (4H, t, J = 6.8 Hz), 4.01 (4H, s), 1.70-1.58 (4H, m), 1.40-1.19 (28H, m), 0.88 (6H, t, J = 6.8 Hz).

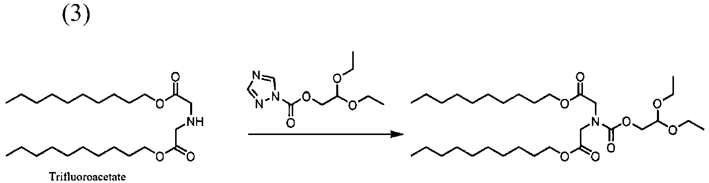

A mixture of a trifluoroacetate of didecyl 2,2'-azanediyldiacetate (1.50 g), 2,2-diethoxyethyl 1H-1,2,4-triazole-1-carboxylate (0.65 g), acetonitrile (6 mL), triethylamine (1.19 mL), and N,N-dimethylaminopyridine (0.35 g) was stirred at 50°C for 4 hours. Ethyl acetate (10 mL) and water (5 mL) were added to the reaction mixture cooled to room temperature, and the organic layer was separated. The obtained organic layer was washed with saturated saline, anhydrous sodium sulfate was added thereto to dry the organic layer, and the solvent was distilled off under reduced pressure. The obtained residue was purified by silica gel column chromatography (ethyl acetate-hexane) to obtain didecyl 2,2-(((2,2-diethoxyethoxy)carbonyl)azanediyl)diacetate (1.13 g) as a colorless oily substance.

¹H-NMR (CDCl₃) δ: 4.65 (1H, t, J = 5.5 Hz), 4.17-4.07 (10H, m), 3.75-3.64 (2H, m), 3.60-3.50 (2H, m), 1.69-1.50 (8H, m), 1.39-1.17 (30H, m), 0.88 (6H, t, J = 6.8 Hz).

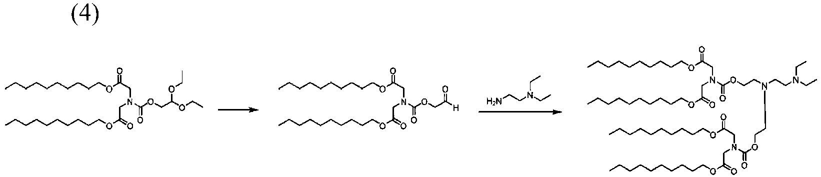

Decyl 2-(2-(2-(bis(2-decoxy-2-oxo-ethyl)carbamoyloxy)ethyl-(2-(diethylamino)ethyl)amino)ethoxycarbonyl-(2-decoxy-2-oxo-ethyl)amino)acetate as a colorless oily substance was obtained by the same method as that in (5) of Example 4 and (6) of Example 4, except that didecyl 2,2'-(((2,2-diethoxyethoxy)carbonyl)azanediyl)diacetate was used instead of (((2,2-diethoxyethoxy)carbonyl)(hexyl)amino)pentanoic acid in (5) of Example 4.

¹H-NMR (CDCl₃) δ: 4.19-4.04 (20H, m), 2.83-2.72 (4H, m), 2.66-2.58 (2H, m), 2.56-2.43 (6H, m), 1.67-1.49 (16H, m), 1.37-1.19 (48H, m), 1.06-0.95 (6H, m), 0.88 (12H, t, J = 6.8 Hz).

MS m/z (M + H): 1084.

### [Example 28]

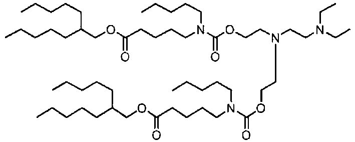

Bis(2-pentylheptyl) 11-(2-(diethylamino)ethyl)-7,15-dioxo-6,16-dipentyl-8,14-dioxa-6,11,16-triazahenicosanedioate as a colorless oily substance was obtained by the same method as that in Example 4, except that n-pentylamine was used instead of n-hexylamine in (3) of Example 4.

¹H-NMR (CDCl₃) δ: 4.10, (4H, t, J = 6.4 Hz), 3.96 (4H, d, J = 5.8 Hz), 3.27-3.09 (8H, m), 2.79 (4H, t, J = 6.2 Hz), 2.73-2.41 (8H, m), 2.32 (4H, t, J = 6.4 Hz), 1.67-1.44 (14H, m), 1.38-1.16 (40H, m), 1.10-0.96 (6H, m), 0.94-0.82 (18H, m).

MS m/z (M + H): 968.

### [Example 29]

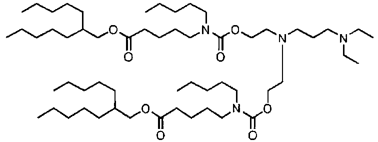

Bis(2-pentylheptyl) 11-(3-(diethylamino)propyl)-7,15-dioxo-6,16-dipentylo-8,14-dioxa-6,11,16-triazahenicosanedioate as a colorless oily substance was obtained by the same method as that in Example 4, except that n-pentylamine was used instead of n-hexylamine in (3) of Example 4 and N,N-diethyl-1,3-diaminopropane was used instead of N,N-diethylethylenediamine in (6) of Example 4.

¹H-NMR (CDCl₃) δ: 4.09 (4H, t, J = 6.4 Hz), 3.96 (4H, d, J = 5.7 Hz), 3.27-3.10 (8H, m), 2.76 (4H, t, J = 6.4 Hz), 2.63-2.37 (8H, m), 2.32 (4H, t, J = 6.6 Hz), 1.70-1.44 (16H, m), 1.38-1.17 (40H, m), 1.10-0.95 (6H, m), 0.92-0.84 (18H, m).

MS m/z (M + H): 982.

### [Example 30]

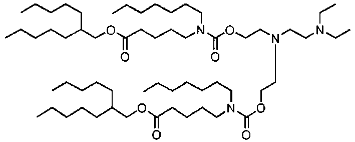

Bis(2-pentylheptyl) 11-(2-(diethylamino)ethyl)-6,16-diheptyl-7,15-dioxo-8,14-dioxa-6,11,16-triazahenicosanedioate as a colorless oily substance was obtained by the same method as that in Example 4, except that n-heptylamine was used instead of n-hexylamine in (3) of Example 4.

¹H-NMR (CDCl₃) δ: 4.10 (4H, t, J = 6.4 Hz), 3.96 (4H, d, J = 5.8 Hz), 3.28-3.09 (8H, m), 2.79 (4H, t, J = 6.4 Hz), 2.73-2.60 (2H, m), 2.59-2.41 (6H, m), 2.32 (4H, t, J = 6.3 Hz), 1.68-1.44 (14H, m), 1.36-1.18 (48H, m), 1.07-0.95 (6H, m), 0.92-0.81 (18H, m).

MS m/z (M + H): 1025.

### [Example 31]

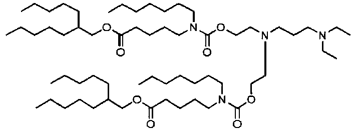

Bis(2-pentylheptyl) 11-(3-(diethylamino)propyl)-6,16-diheptyl-7,15-dioxo-8,14-dioxa-6,11,16-triazahenicosanedioate as a colorless oily substance was obtained by the same method as that in Example 4, except that n-heptylamine was used instead of n-hexylamine in (3) of Example 4 and N,N-diethyl-1,3-diaminopropane was used instead of N,N-diethylethylenediamine in (6) of Example 4.

¹H-NMR (CDCl₃) δ: 4.09 (4H, t, J = 6.6 Hz), 3.96 (4H, d, J = 5.8 Hz), 3.26-3.09 (8H, m), 2.76 (4H, t, J = 6.4 Hz), 2.65-2.36 (8H, m), 2.32 (4H, t, J = 6.3 Hz), 1.67-1.43 (16H, m), 1.37-1.17 (48H, m), 1.07-0.95 (6H, m), 0.93-0.81 (18H, m).

MS m/z (M + H): 1039.

### [Example 32]

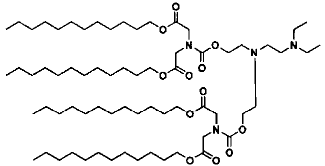

Didecyl 8-(2-(diethylamino)ethyl)-3,13-bis(2-(dodecyloxy)-2-oxoethyl)-4,12-dioxo-5,11-dioxa-3,8,13-triazapentadecanedioate as a colorless oily substance was obtained by the same method as that in Example 27, except that 1-dodecanol was used instead of 1-decanol in (1) of Example 27.

¹H-NMR (CDCl₃) δ: 4.20-4.03 (20H, m), 2.82-2.73 (4H, m), 2.67-2.58 (2H, m), 2.57-2.43 (6H, m), 1.68-1.51 (16H, m), 1.37-1.19 (64H, m), 1.07-0.95 (6H, m), 0.88 (12H, t, J = 6.8 Hz).

MS m/z (M + H): 1196.

### [Example 33]

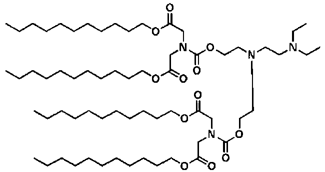

Diundecyl 8-(2-(diethylamino)ethyl)-4,12-dioxo-3,13-bis(2-oxo-2-(undecyloxy)ethyl)-5,11-dioxa-3,8,13-triazapentadecanedioate as a colorless oily substance was obtained by the same method as that in Example 27, except that 1-undecanol was used instead of 1-decanol in (1) of Example 27.

¹H-NMR (CDCl₃) δ: 4.21-4.06 (20H, m), 2.85-2.72 (4H, m), 2.66-2.57 (2H, m), 2.56-2.41 (6H, m), 1.69-1.48 (16H, m), 1.37-1.19 (56H, m), 1.06-0.94 (6H, m), 0.88 (12H, t, J = 6.8 Hz).

MS m/z (M + H): 1140.

### [Example 34]

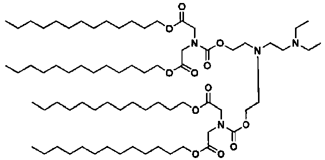

Ditridecyl 8-(2-(diethylamino)ethyl)-4,12-dioxo-3,13-bis(2-oxo-2-(tridecyloxy)ethyl)-5,11-dioxa-3,8,13-triazapentadecanedioate as a colorless oily substance was obtained by the same method as that in Example 27, except that 1-tridecanol was used instead of 1-decanol in (1) of Example 27.

¹H-NMR (CDCl₃) δ: 4.19-4.06 (20H, m), 2.84-2.71 (4H, m), 2.68-2.56 (2H, m), 2.55-2.41 (6H, m), 1.69-1.49 (16H, m), 1.38-1.18 (72H, m), 1.07-0.95 (6H, m), 0.88 (12H, t, J = 6.8 Hz).

### [Example 35]

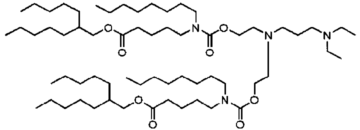

Bis(2-pentylheptyl) 11-(3-(diethylamino)propyl)-6,16-dioctyl-7,15-dioxo-8,14-dioxa-6,11,16-triazahenicosanedioate as a colorless oily substance was obtained by the same method as that in Example 4, except that n-octylamine was used instead of n-hexylamine in (3) of Example 4 and N,N-diethyl-1,3-diaminopropane was used instead of N,N-diethylethylenediamine in (6) of Example 4.

¹H-NMR (CDCl₃) δ: 4.09 (4H, t, J = 6.5 Hz), 3.96 (4H, d, J = 5.8 Hz), 3.27-3.09 (8H, m), 2.76 (4H, t, J = 6.4 Hz), 2.66-2.36 (8H, m), 2.32 (4H, t, J = 6.2 Hz), 1.68-1.43 (16H, m), 1.36-1.18 (52H, m), 1.08-0.95 (6H, m), 0.93-0.83 (18H, m).

MS m/z (M + H): 1067.

### [Example 36]

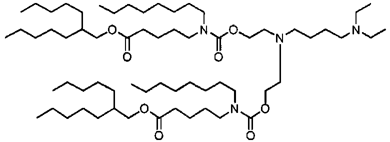

Bis(2-pentylheptyl) 11-(4-(diethylamino)butyl)-6,16-dioctyl-7,15-dioxo-8,14-dioxa-6,11,16-triazahenicosanedioate as a colorless oily substance was obtained by the same method as that in Example 4, except that n-octylamine was used instead of n-hexylamine in (3) of Example 4 and 4-(diethylamino)butylamine was used instead of N,N-diethylethylenediamine in (6) of Example 4.

¹H-NMR (CDCl₃) δ: 4.08 (4H, t, J = 6.5 Hz), 3.96 (4H, d, J = 5.8 Hz), 3.26-3.10 (8H, m), 2.75 (4H, t, J = 6.4 Hz), 2.62-2.36 (8H, m), 2.35-2.27 (4H, m), 1.66-1.37 (18H, m), 1.36-1.18 (52H, m), 1.09-0.95 (6H, m), 0.92-0.82 (18H, m).

MS m/z (M + H): 1081.

### [Example 37]

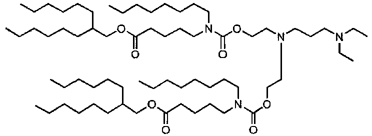

Bis(2-hexyloctyl) 11-(3-(diethylamino)propyl)-6,16-dioctyl-7,15-dioxo-8,14-dioxa-6,11,16-triazahenicosanedioate was obtained by the same method as that in (6) of Example 4, except that 2-hexyloctyl 5-(octyl((2-oxoethoxy)carbonyl)amino)pentanoate was used instead of 2-pentylheptyl 5-(hexyl((2-oxoethoxy)carbonyl)amino)pentanoate and N,N-diethyl-1,3-diaminopropane was used instead of N,N-diethylethylenediamine in (6) of Example 4.

¹H-NMR (CDCl₃) δ: 4.09 (4H, t, J = 6.4 Hz), 3.96 (4H, d, J = 5.8 Hz), 3.27-3.07 (8H, m), 2.76 (4H, t, J = 6.4 Hz), 2.67-2.36 (8H, m), 2.32 (4H, t, J = 6.4 Hz), 1.67-1.44 (16H, m), 1.36-1.16 (60H, m), 1.06-0.96 (6H, m), 0.92-0.82 (18H, m).

MS m/z (M + H): 1123.

### [Example 38]

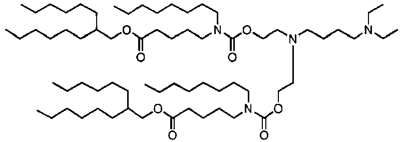

Bis(2-hexyloctyl) 11-(4-(diethylamino)butyl)-6,16-dioctyl-7,15-dioxo-8,14-dioxa-6,11,16-triazahenicosanedioate was obtained by the same method as that in (6) of Example 4, except that 2-hexyloctyl 5-(octyl((2-oxoethoxy)carbonyl)amino)pentanoate was used instead of 2-pentylheptyl 5-(hexyl((2-oxoethoxy)carbonyl)amino)pentanoate and 4-(diethylamino)butylamine was used instead of N,N-diethylethylenediamine in (6) of Example 4.

¹H-NMR (CDCl₃) δ: 4.08 (4H, t, J = 6.4 Hz), 3.96 (4H, d, J = 5.7 Hz), 3.27-3.08 (8H, m), 2.75 (4H, t, J = 6.5 Hz), 2.64-2.37 (8H, m), 2.36-2.27 (4H, m), 1.68-1.36 (18H, m), 1.35-1.17 (60H, m), 1.06-0.95 (6H, m), 0.93-0.80 (18H, m).

MS m/z (M + H): 1137.

### [Example 39]

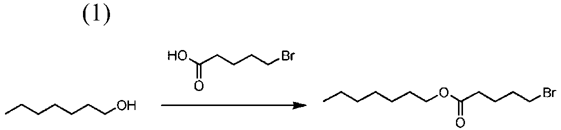

Heptyl 5-bromopentanoate as a colorless oily substance was obtained by the same method as that in (2) of Example 4, except that 1-heptanol was used instead of 2-pentyl-1-heptanol in (2) of Example 4.

¹H-NMR (CDCl₃) δ: 4.06 (2H, t, J = 6.8 Hz), 3.42 (2H, t, J = 6.6 Hz), 2.34 (2H, t, J = 7.3 Hz), 1.93-1.87 (2H, m), 1.82-1.74 (2H, m), 1.67-1.58 (2H, m), 1.37-1.24 (8H, m), 0.89 (3H, t, J = 6.9 Hz).

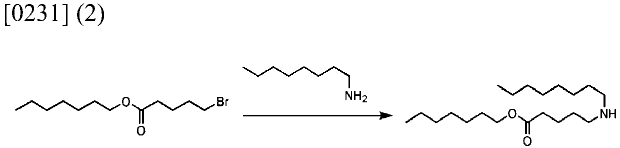

5-(Octylamino)heptylpentanoate as a colorless oily substance was obtained by the same method as that in (3) of Example 4, except that heptyl 5-bromopentanoate was used instead of 2-pentylheptyl 5-bromopentanoate and n-octylamine was used instead of n-hexylamine in (3) of Example 4.

¹H-NMR (CDCl₃) δ: 4.06 (2H, t, J = 6.8 Hz), 2.66-2.59 (4H, m), 2.33 (2H, t, J = 7.3 Hz), 1.71-1.48 (9H, m), 1.36-1.22 (18H, m), 0.90-0.86 (6H, m).

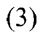

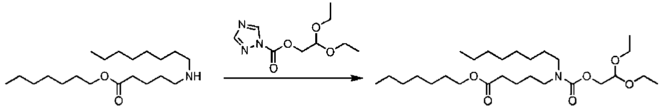

Heptyl 5-(((2,2-diethoxyethoxy)carbonyl)(octyl)amino)pentanoate as a colorless oily substance was obtained by the same method as that in (4) of Example 4, except that heptyl 5-(octylamino)pentanoate was used instead of 2-pentylheptyl 5-(hexylamino)pentanoate in (4) of Example 4.

¹H-NMR (CDCl₃) δ: 4.71-4.67 (1H, m), 4.09-4.03 (4H, m), 3.75-3.67 (2H, m), 3.61-3.53 (2H, m), 3.26-3.14 (4H, m), 2.35-2.29 (2H, m), 1.64-1.46 (8H, m), 1.33-1.19 (24H, m), 0.90-0.85 (6H, m).

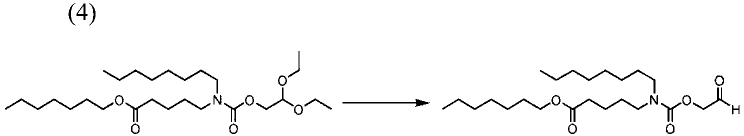

Heptyl 5-(octyl((2-oxoethoxy)carbonyl)amino)pentanoate as a colorless oily substance was obtained by the same method as that in (5) of Example 4, except that heptyl 5-(((2,2-diethoxyethoxy)carbonyl)(octyl)amino)pentanoate was used instead of 2-pentylheptyl 5-(((2,2-diethoxyethoxy)carbonyl)(hexyl)amino)pentanoate in (5) of Example 4.

¹H-NMR (CDCl₃) δ: 9.63 (1H, s), 4.61 (2H, d, J = 3.6 Hz), 4.06 (2H, t, J = 6.7 Hz), 3.32-3.22 (4H, m), 2.36-2.32 (2H, m), 1.66-1.50 (8H, m), 1.36-1.22 (18H, m), 0.90-0.86 (6H, m).

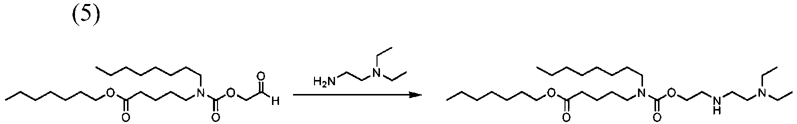

N,N-diethylethylenediamine (0.33 g), acetic acid (0.10 mL), and sodium triacetoxyborohydride (0.92 g) were added to an ethyl acetate (6 mL) solution of heptyl 5-(octyl((2-oxoethoxy)carbonyl)amino)pentanoate (0.60 g) at room temperature, and the mixture was stirred at the same temperature for 4 hours. A saturated aqueous sodium hydrogen carbonate solution was added to the reaction mixture, and the organic layer thus separated was washed with saturated saline. Anhydrous sodium sulfate was added thereto to dry the organic layer, the solvent was distilled off under reduced pressure, and the obtained residue was purified by NH silica gel column chromatography (ethyl acetate-hexane) to obtain heptyl 3-ethyl-11-octyl-10-oxo-9-oxa-3,6,11-triazahexadecane-16-oate (0.57 g) as a colorless oily substance.

¹H-NMR (CDCl₃) δ: 4.19 (2H, t, J = 5.5 Hz), 4.05 (2H, t, J = 6.7 Hz), 3.29-3.10 (4H, m), 2.89 (2H, t, J = 5.4 Hz), 2.83-2.50 (8H, m), 2.32 (2H, t, J = 6.8 Hz), 1.88-1.46 (10H, m), 1.37-1.19 (16H, m), 1.14-0.96 (6H, m), 0.91-0.82 (6H, m).

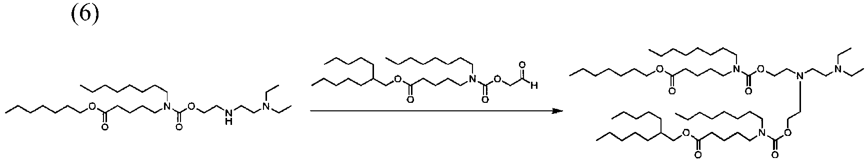

2-Pentylheptyl 5-(octyl((2-oxoethoxy)carbonyl)amino)pentanoate (0.21 g) as the synthetic intermediate in Example 3 and sodium triacetoxyborohydride (0.12 g) were added to an ethyl acetate (2 mL) solution of heptyl 3-ethyl-11-octyl-10-oxo-9-oxa-3,6,11-triazahexadecane-16-oate (0.15 g) at room temperature, and the mixture was stirred at the same temperature for 4 hours. A saturated aqueous sodium hydrogen carbonate solution was added to the reaction mixture, and the organic layer thus separated was washed with saturated saline. Anhydrous sodium sulfate was added thereto to dry the organic layer, the solvent was distilled off under reduced pressure, and the obtained residue was purified by NH silica gel column chromatography (ethyl acetate-hexane) to obtain 1-heptyl 21-(2-pentylheptyl) 11-(2-(diethylamino)ethyl)-6,16-dioctyl-7,15-dioxo-8,14-dioxa-6,11,16-triazahenicosanedioate (0.21 g) as a colorless oily substance.

¹H-NMR (CDCl₃) δ: 4.10 (4H, t, J = 6.3 Hz), 4.05 (2H, t, J = 6.8 Hz), 3.96 (2H, d, J = 5.8 Hz), 3.27-3.09 (8H, m), 2.79 (4H, t, J = 6.2 Hz), 2.73-2.61 (2H, m), 2.60-2.42 (6H, m), 2.38-2.26 (4H, m), 1.68-1.44 (17H, m), 1.38-1.19 (42H, m), 1.09-0.95 (6H, m), 0.93-0.82 (15H, m).

MS m/z (M + H): 982.

### [Example 40]

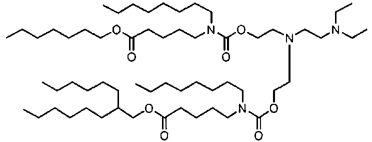

1-Heptyl 21-(2-hexyloctyl) 11-(2-(diethylamino)ethyl)-6,16-dioctyl-7,15-dioxo-8,14-dioxa-6,11,16-triazahenicosanedioate as a colorless oily substance was obtained by the same method as that in Example 39, except that 2-hexyloctyl 5-(octyl((2-oxoethoxy)carbonyl)amino)pentanoate obtained in (4) of Example 2 was used instead of 2-pentylheptyl 5-(octyl((2-oxoethoxy)carbonyl)amino)pentanoate in (6) of Example 39.

¹H-NMR (CDCl₃) δ: 4.10 (4H, t, J = 6.3 Hz), 4.05 (2H, t, J = 6.8 Hz), 3.96 (2H, d, J = 5.8 Hz), 3.27-3.08 (8H, m), 2.79 (4H, t, J = 6.2 Hz), 2.72-2.61 (2H, m), 2.60-2.42 (6H, m), 2.36-2.27 (4H, m), 1.68-1.44 (17H, m), 1.37-1.17 (46H, m), 1.10-0.96 (6H, m), 0.94-0.82 (15H, m).

MS m/z (M + H): 1010.

### [Example 41]

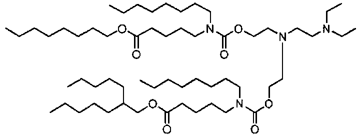

1-ocryl 21-(2-pentylheptyl) 11-(2-(diethylamino)ethyl)-6,16-dioctyl-7,15-dioxo-8,14-dioxa-6,11,16-triazahenicosanedioate as a colorless oily substance was obtained by the same method as that in Example 39, except that 1-octanol was used instead of 1-heptanol in (1) of Example 39.

¹H-NMR (CDCl₃) δ: 4.10 (4H, t, J = 6.3 Hz), 4.05 (2H, t, J = 6.8 Hz), 3.96 (2H, d, J = 5.7 Hz), 3.27-3.06 (8H, m), 2.79 (4H, t, J = 6.3 Hz), 2.72-2.61 (2H, m), 2.60-2.44 (6H, m), 2.38-2.27 (4H, m), 1.70-1.43 (17H, m), 1.36-1.15 (44H, m), 1.09-0.95 (6H, m), 0.92-0.81 (15H, m).

MS m/z (M + H): 996.

### [Example 42]

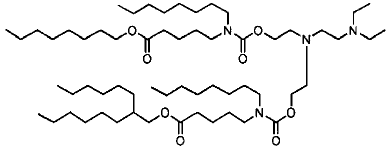

1-(2-Hexyloctyl) 21-octyl 11-(2-(diethylamino)ethyl)-6,16-dioctyl-7,15-dioxo-8,14-dioxa-6,11,16-triazahenicosanedioate was obtained by the same method as that in Example 39, except that 1-octanol was used instead of 1-heptanol in (1) of Example 39, and 2-hexyloctyl 5-(octyl((2-oxoethoxy)carbonyl)amino)pentanoate obtained in (4) of Example 2 was used instead of 2-pentylheptyl 5-(octyl((2-oxoethoxy)carbonyl)amino)pentanoate in (6) of Example 39.

¹H-NMR (CDCl₃) δ: 4.10 (4H, t, J = 6.4 Hz), 4.05 (2H, t, J = 6.8 Hz), 3.96 (2H, d, J = 5.7 Hz), 3.27-3.09 (8H, m), 2.79 (4H, t, J = 6.0 Hz), 2.72-2.61 (2H, m), 2.60-2.42 (6H, m), 2.37-2.28 (4H, m), 1.68-1.43 (17H, m), 1.38-1.17 (48H, m), 1.06-0.96 (6H, m), 0.93-0.81 (15H, m).

MS m/z (M + H): 1024.

### [Example 43]

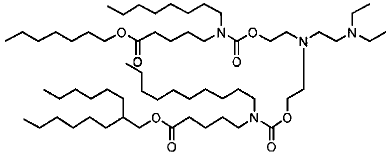

21-Heptyl 1-(2-hexyloctyl) 6-decyl-11-(2-(diethylamino)ethyl)-16-octyl-7,15-dioxo-8,14-dioxa-6,11,16-triazahenicosanedioate as a colorless oily substance was obtained by the same method as that in (6) of Example 39, except that 2-hexyloctyl 5-(decyl((2-oxoethoxy)carbonyl)amino)pentanoate obtained by the same method as that in Example 4 was used instead of 2-pentylheptyl 5-(octyl((2-oxoethoxy)carbonyl)amino)pentanoate in (6) of Example 39.

¹H-NMR (CDCl₃) δ: 4.10 (4H, t, J = 6.3 Hz), 4.05 (2H, t, J = 6.8 Hz), 3.96 (2H, d, J = 5.7 Hz), 3.26-3.08 (8H, m), 2.79 (4H, t, J = 6.3 Hz), 2.72-2.60 (2H, m), 2.59-2.44 (6H, m), 2.36-2.27 (4H, m), 1.71-1.43 (17H, m), 1.36-1.17 (50H, m), 1.07-0.95 (6H, m), 0.94-0.82 (15H, m).

MS m/z (M + H): 1038.

### [Example 44]

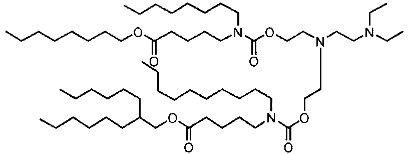

1-(2-Hexyloctyl) 21-octyl 6-decyl-11-(2-(diethylamino)ethyl)-16-octyl-7,15-dioxo-8,14-dioxa-6,11,16-triazahenicosanedioate was obtained by the same method as that in Example 39, except that 1-octanol was used instead of 1-heptanol in (1) of Example 39, and 2-hexyloctyl 5-(decyl((2-oxoethoxy)carbonyl)amino)pentanoate obtained by the same method as that in Example 4 was used instead of 2-pentylheptyl 5-(octyl((2-oxoethoxy)carbonyl)amino)pentanoate in (6) of Example 39.

¹H-NMR (CDCl₃) δ: 4.10 (4H, t, J = 6.3 Hz), 4.05 (2H, t, J = 6.8 Hz), 3.96 (2H, d, J = 5.7 Hz), 3.27-3.09 (8H, m), 2.79 (4H, t, J = 6.3 Hz), 2.73-2.60 (2H, m), 2.59-2.43 (6H, m), 2.37-2.27 (4H, m), 1.69-1.41 (17H, m), 1.38-1.17 (52H, m), 1.07-0.96 (6H, m), 0.93-0.81 (15H, m).

MS m/z (M + H): 1052.

### [Example 45]

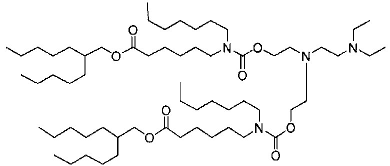

Bis(2-pentylheptyl) 12-(2-(diethylamino)ethyl)-7,17-diheptyl-8,16-dioxo-9,15-dioxa-7,12,17-triazatricosanedioate as a colorless oily substance was obtained by the same method as that in Example 4, except that 6-bromohexanoic acid was used instead of 5-bromovaleric acid in (2) of Example 4 and n-heptylamine was used instead of n-hexylamine in (3) of Example 4.

¹H-NMR (CDCl₃) δ: 4.10 (4H, t, J = 6.3 Hz), 3.97 (4H, d, J = 5.8 Hz), 3.28-3.08 (8H, m), 2.79 (4H, t, J = 6.4 Hz), 2.72-2.60 (2H, m), 2.59-2.42 (6H, m), 2.30 (4H, t, J = 7.5 Hz), 1.70-1.41 (14H, m), 1.38-1.16 (52H, m), 1.08-0.95 (6H, m), 0.95-0.81 (18H, m).

MS m/z (M + H): 1053.

### [Example 46]

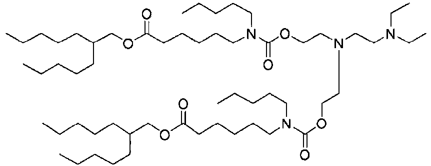

Bis(2-pentylheptyl) 12-(2-(diethylamino)ethyl)-8,16-dioxo-7,17-dipentyl-9,15-dioxa-7,12,17-triazatricosanedioate as a colorless oily substance was obtained by the same method as that in Example 4, except that 6-bromohexanoic acid was used instead of 5-bromovaleric acid in (2) of Example 4 and n-pentylamine was used instead of n-hexylamine in (3) of Example 4.

¹H-NMR (CDCl₃) δ: 4.10 (4H, t, J = 6.4 Hz), 3.97 (4H, d, J = 5.8 Hz), 3.28-3.08 (8H, m), 2.79 (4H, t, J = 6.4 Hz), 2.72-2.60 (2H, m), 2.59-2.42 (6H, m), 2.30 (4H, t, J = 6.2 Hz), 1.70-1.41 (14H, m), 1.38-1.16 (44H, m), 1.01 (6H, t, J = 7.1 Hz), 0.95-0.81 (18H, m).

MS m/z (M + H): 996.

### [Example 47]

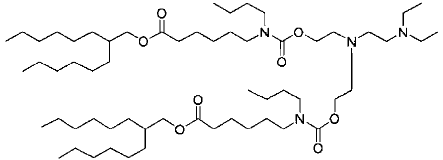

Bis(2-hexyloctyl) 7,17-dibutyl-12-(2-(diethylamino)ethyl)-8,16-dioxo-9,15-dioxa-7,12,17-triazatricosanedioate as a colorless oily substance was obtained by the same method as that in Example 4, except that 2-hexyl-1-octanol was used instead of 2-pentyl-1-heptanol and 6-bromohexanoic acid was used instead of 5-bromovaleric acid in (2) of Example 4, and n-butylamine was used instead of n-hexylamine in (3) of Example 4.

¹H-NMR (CDCl₃) δ: 4.10 (4H, t, J = 6.3 Hz), 3.97 (4H, d, J = 5.7 Hz), 3.26-3.09 (8H, m), 2.79 (4H, t, J = 6.3 Hz), 2.72-2.61 (2H, m), 2.57-2.45 (6H, m), 2.30 (4H, t, J = 7.5 Hz), 1.66-1.44 (14H, m), 1.38-1.17 (48H, m), 1.01 (6H, t, J = 7.1 Hz), 0.95-0.83 (18H, m).

MS m/z (M + H): 1024.

### [Example 48]

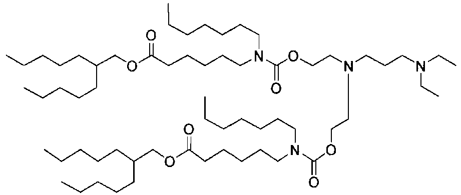

Bis(2-pentylheptyl) 12-(3-(diethylamino)propyl)-7,17-diheptyl-8,16-dioxo-9,15-dioxa-7,12,17-triazatricosanedioate as a colorless oily substance was obtained by the same method as that in Example 4, except that 6-bromohexanoic acid was used instead of 5-bromovaleric acid in (2) of Example 4, n-heptylamine was used instead of n-hexylamine in (3) of Example 4, and N,N-diethyl-1,3-diaminopropane was used instead of N,N-diethylethylenediamine in (6) of Example 4.

¹H-NMR (CDCl₃) δ: 4.10 (4H, t, J = 6.4 Hz), 3.97 (4H, d, J = 5.8 Hz), 3.28-3.08 (8H, m), 2.76 (4H, t, J = 6.4 Hz), 2.61-2.45 (6H, m), 2.45-2.36 (2H, m), 2.30 (4H, t, J = 7.5 Hz), 1.70-1.41 (16H, m), 1.38-1.16 (52H, m), 1.00 (6H, t, J = 7.1 Hz), 0.95-0.81 (18H, m).

MS m/z (M + H): 1066.

### [Example 49]

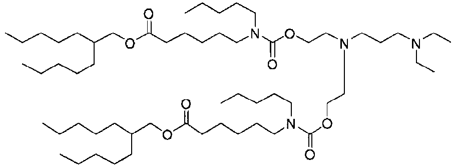

Bis(2-pentylheptyl) 12-(3-(diethylamino)propyl)-8,16-dioxo-7,17-dipentyl-9,15-dioxa-7,12,17-triazatricosanedioate as a colorless oily substance was obtained by the same method as that in Example 4, except that 6-bromohexanoic acid was used instead of 5-bromovaleric acid in (2) of Example 4, n-pentylamine was used instead of n-hexylamine in (3) of Example 4, and N,N-diethyl-1,3-diaminopropane was used instead of N,N-diethylethylenediamine in (6) of Example 4.

¹H-NMR (CDCl₃) δ: 4.10 (4H, t, J = 6.4 Hz), 3.97 (4H, d, J = 5.8 Hz), 3.28-3.08 (8H, m), 2.76 (4H, t, J = 6.4 Hz), 2.61-2.45 (6H, m), 2.45-2.36 (2H, m), 2.30 (4H, t, J = 6.6 Hz), 1.70-1.41 (16H, m), 1.38-1.16 (44H, m), 1.01 (6H, t, J = 7.1 Hz), 0.95-0.81 (18H, m).

MS m/z (M + H): 1010.

### [Example 50]

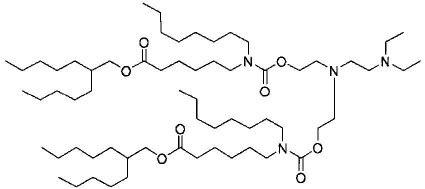

Bis(2-pentylheptyl) 12-(2-(diethylamino)ethyl)-7,17-dioctyl-8,16-dioxo-9,15-dioxa-7,12,17-triazatricosanedioate as a colorless oily substance was obtained by the same method as that in Example 4, except that 6-bromohexanoic acid was used instead of 5-bromovaleric acid in (2) of Example 4 and n-octylamine was used instead of n-hexylamine in (3) of Example 4.

¹H-NMR (CDCl₃) δ: 4.10 (4H, t, J = 5.9 Hz), 3.97 (4H, d, J = 5.8 Hz), 3.26-3.07 (8H, m), 2.79 (4H, t, J = 6.4 Hz), 2.72-2.60 (2H, m), 2.56-2.45 (6H, m), 2.30 (4H, t, J = 7.5 Hz), 1.70-1.40 (14H, m), 1.38-1.16 (56H, m), 1.01 (6H, t, J = 7.1 Hz), 0.93-0.83 (18H, m).

MS m/z (M + H): 1080.

### [Example 51]

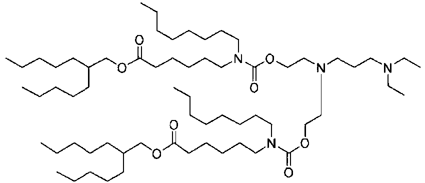

Bis(2-pentylheptyl) 12-(3-(diethylamino)propyl)-7,17-dioctyl-8,16-dioxo-9,15-dioxa-7,12,17-triazatricosanedioate as a colorless oily substance was obtained by the same method as that in Example 4, except that 6-bromohexanoic acid was used instead of 5-bromovaleric acid in (2) of Example 4, n-octylamine was used instead of n-hexylamine in (3) of Example 4, and N,N-diethyl-1,3-diaminopropane was used instead of N,N-diethylethylenediamine in (6) of Example 4.

¹H-NMR (CDCl₃) δ: 4.10 (4H, t, J = 6.4 Hz), 3.97 (4H, d, J = 5.8 Hz), 3.26-3.07 (8H, m), 2.79 (4H, t, J = 6.4 Hz), 2.76 (4H, t, J = 6.4 Hz), 2.60-2.45 (6H, m), 2.30 (4H, t, J = 7.5 Hz), 1.70-1.40 (16H, m), 1.38-1.16 (56H, m), 1.00 (6H, t, J = 7.1 Hz), 0.93-0.83 (18H, m).

MS m/z (M + H): 1094.

### Test Example 1: Preparation of mRNA-encapsulating lipid nanoparticles and measurement of reporter protein expression rate in mice

### <Preparation of EPO mRNA-encapsulating lipid nanoparticles>

The compounds described in Table 1, the neutral lipid, cholesterol (product name: Cholesterol HP; NIPPON FINE CHEMICAL CO., LTD.), and 1,2-dimyristoyl-rac-glycero-3-(methylpolyoxyethylene 2000) (hereinafter, called DMG-PEG2000) (product name: SUNBRIGHT(R) GM-020; NOF corporation) were dissolved in ethanol at a molar ratio of ionized lipid:neutral lipid:cholesterol:DMG-PEG2000 = 50:10:38.5:1.5 mol% such that the total lipid concentration was 12.5 mmol/L, to obtain an oil phase.

As the neutral lipid, 1,2-distearoyl-sn-glycero-3-phosphocholine (product name: COATSOME (R) MC-8080; NOF corporation) (hereinafter, DSPC), or L-α-dioleoylphosphatidylethanolamine (hereinafter, DOPE) (product name: COATSOME (R) MC-8181; NOF corporation) was used.

EPO mRNA (product name: CleanCap EPO mRNA (5moU); TriLink) was diluted with 50 mmol/L citrate buffer having a pH 4.0 such that the weight ratio of total lipid concentration to mRNA concentration is about 20:1 to 64:1, thereby obtaining a water phase. Then, the water phase and the oil phase were mixed together using NanoAssemblr (Precision NanoSystems) such that the volume ratio of water phase:oil phase was 3:1. The mixed solution was diluted 2-fold with any of phosphate buffered physiological saline (PBS), water, or a 20 mmol/L Tris buffer solution (pH 7.4) to obtain a dispersion liquid of mRNA lipid nanoparticles. The dispersion liquid was dialyzed with 20 mmol/L Tris buffer solution (pH 7.4) containing 8% sucrose using a dialysis cassette (Slide-A-Lyzer G2, MWCO: 10 kD, Thermo Fisher Scientific) to remove ethanol, thereby obtaining EPO mRNA-encapsulating lipid nanoparticles.

### <Measurement of particle size>

The particle size of the mRNA-encapsulating lipid nanoparticles was measured by 5-fold diluting the dispersion liquid of lipid nanoparticle with phosphate buffered physiological saline (PBS) using a particle size measurement device NanoSaqla (Otsuka Electronics Co., Ltd.). The results are shown in Table 1.

### <Evaluation of encapsulation rate of mRNA>

### (Quantification of total mRNA concentration)

EPO mRNA was diluted with MilliQ water to prepare a diluted sample in 2-fold dilution series from 100 µg/mL to 3.1 µg/mL, and a calibration curve solution was prepared. 50 µL of the calibration curve solution or the mRNA lipid nanoparticles was mixed with 450 µL of methanol to prepare a measurement solution.

The absorbance of each measurement solution at 260 nm and 330 nm was measured using a UV plate reader (Multiskan Go, Thermo Fisher Scientific), the absorbance at 330 nm was subtracted from the absorbance at 260 nm, and the result was defined as the absorbance of each measurement solution. The total water phase nucleic acid concentration was calculated from the calibration curve using the absorbance of each sample measurement solution.

### (Quantification of mRNA concentration in outer water phase)

The concentration of the outer water phase nucleic acid was quantified by a standard addition method using a QuanT-iT RiboGreen RNA Assay Kit (Thermo Fisher Scientific). First, a 20× TE buffer included in the above kit was diluted with water, thereby obtaining a 1× TE buffer. TE represents Tris/EDTA (ethylenediaminetetraacetic acid). EPO mRNA was diluted with a TE buffer such that the final concentration was 0 to 400 ng/mL to prepare a nucleic acid diluted series. 10 µL of mRNA lipid nanoparticles diluted 5-fold with TE buffer and 90 µL of a nucleic acid dilution series were mixed in a 96-well plate, 100 µL of a Ribogreen reagent diluted 200-fold with TE buffer was added to each well, and then fluorescence (excitation wavelength: 485 nm, fluorescence wavelength: 535 nm) was measured using a fluorescence plate reader (Infinite 200 Pro M nano +, TECAN). From the obtained results, the outer water phase nucleic acid concentration of each measurement solution was calculated according to the standard addition method.

### (Calculation of encapsulation rate)

By using the quantification results of the total mRNA concentration and the mRNA concentration in the outer water phase that were obtained through the above steps, the mRNA encapsulation rate of the mRNA lipid nanoparticles was calculated according to the following Equation. The results are shown in Table 1. mRNA encapsulation rate (%) = (total mRNA concentration-mRNA concentration in outer water phase)/total mRNA concentration × 100

**[Table 1]**

| Compound used | Neutral lipid | Mixed solution | Lipid/Nucleic acid (wt/wt) | Particle size (nm) | Nucleic acid encapsulation rate (%) |
|---|---|---|---|---|---|
| Comparative Example 1 | DSPC | PBS | 20 | 206 | 62% |
| Example 2 | DSPC | PBS | 20 | 82 | 94% |
| Example 3 | DSPC | PBS | 20 | 78 | 94% |
| Example 4 | DSPC | PBS | 20 | 94 | 92% |
| Example 5 | DSPC | PBS | 20 | 88 | 93% |
| Example 6 | DSPC | PBS | 20 | 183 | 58% |
| Example 7 | DSPC | PBS | 20 | 215 | 73% |
| Example 12 | DSPC | PBS | 20 | 169 | 62% |
| Example 13 | DSPC | PBS | 20 | 188 | 88% |
| Example 14 | DSPC | PBS | 20 | 185 | 97% |
| Example 15 | DSPC | PBS | 20 | 238 | 79% |
| Example 16 | DSPC | PBS | 20 | 187 | 93% |
| Example 17 | DSPC | PBS | 20 | 165 | 80% |
| Example 18 | DSPC | PBS | 20 | 224 | 43% |
| Example 20 | DSPC | PBS | 20 | 151 | 82% |
| Example 21 | DSPC | PBS | 20 | 230 | 64% |
| Example 23 | DSPC | PBS | 20 | 180 | 79% |
| Example 24 | DSPC | PBS | 20 | 197 | 92% |
| Example 25 | DSPC | PBS | 20 | 167 | 78% |
| Example 26 | DSPC | PBS | 20 | 178 | 91% |
| Example 27 | DSPC | PBS | 20 | 149 | 82% |
| Example 32 | DSPC | PBS | 20 | 203 | 60% |
| Example 33 | DSPC | PBS | 20 | 155 | 78% |
| Example 34 | DSPC | PBS | 20 | 332 | 33% |
| Example 35 | DSPC | PBS | 20 | 165 | 93% |
| Example 36 | DSPC | PBS | 20 | 145 | 93% |
| Example 37 | DSPC | PBS | 20 | 136 | 93% |
| Example 38 | DSPC | PBS | 20 | 137 | 93% |
| Example 40 | DSPC | PBS | 20 | 162 | 74% |
| Example 42 | DSPC | PBS | 20 | 154 | 82% |
| Example 1 | DOPE | PBS | 64 | 88.6 | >99% |
| Example 2 | DOPE | PBS | 64 | 95.6 | 99% |
| Example 3 | DOPE | PBS | 64 | 94.5 | 99% |
| Example 4 | DOPE | PBS | 64 | 109.5 | >99% |
| Example 5 | DOPE | PBS | 64 | 106.8 | 99% |
| Example 4 | DSPC | Water | 20 | 93 | 91% |
| Example 4 | DSPC | 20mM Tris | 20 | 96 | 91% |
| Example 45 | DSPC | PBS | 20 | 84 | 95% |
| Example 46 | DSPC | PBS | 20 | 104 | 93% |
| Example 47 | DSPC | PBS | 20 | 114 | 91% |
| Example 48 | DSPC | PBS | 20 | 90 | 96% |
| Example 49 | DSPC | PBS | 20 | 126 | 94% |
| Example 50 | DSPC | PBS | 20 | 82 | 96% |
| Example 51 | DSPC | PBS | 20 | 93 | 94% |

### <Measurement of EPO enzyme activity>

The dispersion liquid of mRNA lipid nanoparticles prepared in above <Preparation of EPO mRNA-encapsulating lipid nanoparticles> was intravenously administered to ICR mice at a mRNA dosage of 0.1 mg/kg. 6 hours after the administration, blood was collected from the caudal vena cava, thereby obtaining plasma. The human EPO enzyme activity was quantified by using the obtained blood plasma and Erythropoietin (EPO) Human Elisa Kit (Abcam.plc). The quantitative value is described as a relative amount of EPO protein in a case where Comparative Example 1 is 1.

The results are shown in Table 2.

**[Table 2]**

| Compound used | Neutral lipid | Lipid/Nucleic acid (wt/wt) | Relative amount of EPO protein (to Comparative Example 1) |
|---|---|---|---|
| Comparative Example 1 | DSPC | 20 | 1.00 |
| Example 2 | DSPC | 20 | 6.70 |
| Example 3 | DSPC | 20 | 6.15 |
| Example 4 | DSPC | 20 | 12.27 |
| Example 5 | DSPC | 20 | 9.14 |
| Example 17 | DSPC | 20 | 5.76 |
| Example 20 | DSPC | 20 | 1.63 |
| Example 23 | DSPC | 20 | 1.61 |
| Example 24 | DSPC | 20 | 1.48 |
| Example 25 | DSPC | 20 | 3.56 |
| Example 26 | DSPC | 20 | 4.10 |
| Example 27 | DSPC | 20 | 3.85 |
| Example 33 | DSPC | 20 | 2.90 |
| Example 35 | DSPC | 20 | 6.49 |
| Example 36 | DSPC | 20 | 6.40 |
| Example 37 | DSPC | 20 | 11.39 |
| Example 38 | DSPC | 20 | 9.99 |
| Example 40 | DSPC | 20 | 8.10 |
| Example 42 | DSPC | 20 | 6.39 |
| Example 1 | DOPE | 64 | 21.60 |
| Example 2 | DOPE | 64 | 24.57 |
| Example 3 | DOPE | 64 | 13.55 |
| Example 4 | DOPE | 64 | 13.62 |
| Example 5 | DOPE | 64 | 11.74 |
| Example 45 | DSPC | 20 | 2.19 |
| Example 46 | DSPC | 20 | 1.54 |
| Example 47 | DSPC | 20 | 1.46 |
| Example 48 | DSPC | 20 | 2.26 |
| Example 49 | DSPC | 20 | 1.12 |
| Example 50 | DSPC | 20 | 1.55 |
| Example 51 | DSPC | 20 | 2.88 |

In comparison with the nucleic acid lipid composition in Comparative Examples, the nucleic acid lipid composition according to the embodiment of the present invention showed a higher EPO protein expression rate.

## Claims

1. A compound represented by Formula (1) or a salt thereof, 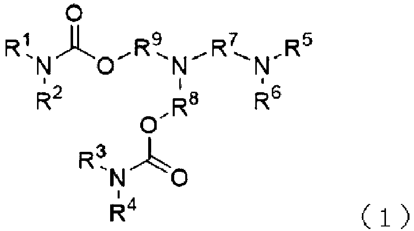
in the formula, R¹, R², R³, and R⁴ each independently represent a hydrogen atom or a hydrocarbon group having 1 to 24 carbon atoms, which may be substituted,
substituents on the hydrocarbon groups having 1 to 24 carbon atoms represented by R¹, R², R³, and R⁴, which may be substituted, each independently represent -C(O)O-R¹¹, - OC(O)-R¹², -O-R¹³, -CO-R¹⁴, -OC(O)O-R¹⁵, or -S-S-R¹⁶,
R¹¹, R¹², R¹³, R¹⁴, R¹⁵, and R¹⁶ each independently represent a hydrocarbon group having 1 to 24 carbon atoms which may be substituted with -S-R¹⁷, where R¹⁷ represents a hydrocarbon group having 1 to 12 carbon atoms,
R⁵ and R⁶ each independently represent a hydrocarbon group having 1 to 18 carbon atoms which may be substituted,
substituents on the hydrocarbon groups having 1 to 18 carbon atoms represented by R⁵ and R⁶, which may be substituted, each independently represent -OH, -COOH, -NR²¹R²², - OC(O)O-R²³, -C(O)O-R²⁴, -OC(O)-R²⁵, -O-R²⁶, -C(O)NR²⁷R²⁸, -NR²⁹C(O)R³⁰, - N(R³¹)S(O)₂R³², -N(R³³)C(O)N(R³⁴)R³⁵, -N(R³⁶)C(S)N(R³⁷)R³⁸, -OC(O)N(R³⁹)R⁴⁰, or - N(R⁴¹)C(O)OR⁴²,
R²¹ and R²² each independently represent a hydrogen atom or a hydrocarbon group having 1 to 8 carbon atoms,
R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴² each independently represent a hydrogen atom or a hydrocarbon group having 1 to 24 carbon atoms, which may be substituted, and substituents on the hydrocarbon groups having 1 to 24 carbon atoms represented by R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, and R⁴², which may be substituted, each independently represent an aryl group having 6 to 20 carbon atoms, a heterocyclic group, -OH, -COOH, or -NR⁵¹R⁵², where R⁵¹ and R⁵² each independently represent a hydrogen atom or a hydrocarbon group having 1 to 8 carbon atoms,
R⁷, R⁸, and R⁹ each independently represent a hydrocarbon group having 2 to 8 carbon atoms, and
R⁵ and R⁶, or R⁵ and R⁷, may be combined to form a 4- to 7-membered ring.

2. The compound or a salt thereof according to claim 1,
wherein R¹ represents -R^{1a}-L¹-R^{1b}, R^{1a} represents a hydrocarbon group having 1 to 18 carbon atoms, L¹ represents -C(O)O-, -OC(O)-, -OC(O)O-, or -S-S-, and R^{1b} represents a hydrocarbon group having 1 to 18 carbon atoms,
R³ represents -R^{3a}-L³-R^{3b}, R^{3a} represents a hydrocarbon group having 1 to 18 carbon atoms, L³ represents -C(O)O-, -OC(O)-, -OC(O)O-, or -S-S-, and R^{3b} represents a hydrocarbon group having 1 to 18 carbon atoms,
R² and R⁴ each independently represent a hydrocarbon group having 1 to 18 carbon atoms, which may be substituted, and substituents on the hydrocarbon groups having 1 to 18 carbon atoms represented by R² and R⁴, which may be substituted, each independently represent -C(O)O-R¹¹, -OC(O)-R¹², -O-R¹³, -CO-R ¹⁴, -OC(O)O-R¹⁵, or -S-S-R¹⁶,
R¹¹, R¹², R¹³, R¹⁴, R¹⁵, and R¹⁶ each independently represent a hydrocarbon group having 1 to 18 carbon atoms,
R⁵ and R⁶ each independently represent a hydrocarbon group having 1 to 12 carbon atoms, which may be substituted,
substituents on the hydrocarbon groups having 1 to 12 carbon atoms represented by R⁵ and R⁶, which may be substituted, each independently represent -OH, -O-R²⁶, -C(O)NR²⁷R²⁸, or -NR²⁹C(O)R³⁰,
R²⁶, R²⁷, R²⁸, R²⁹, and R³⁰ each independently represent a hydrogen atom or a hydrocarbon group having 1 to 12 carbon atoms, which may be substituted, and substituents on the hydrocarbon groups having 1 to 12 carbon atoms represented by R²⁶, R²⁷, R²⁸, R²⁹, and R³⁰, which may be substituted, each independently represent an aryl group having 6 to 10 carbon atoms or a heterocyclic group, and
R⁷, R⁸, and R⁹ each independently represent -(CH₂)ₙ-, and n represents an integer of 2 to 8.

3. The compound or a salt thereof according to claim 1,
wherein R¹ represents -R^{1a}-L¹-R^{1b}, R^{1a} represents a hydrocarbon group having 1 to 18 carbon atoms, L¹ represents -C(O)O- or -OC(O)-, and R^{1b} represents a hydrocarbon group having 1 to 18 carbon atoms,
R³ represents -R^{3a}-L³-R^{3b}, R^{3a} represents a hydrocarbon group having 1 to 18 carbon atoms, L³ represents -C(O)O- or -OC(O)-, and R^{3b} represents a hydrocarbon group having 1 to 18 carbon atoms,
R² and R⁴ each independently represent a hydrocarbon group having 1 to 10 carbon atoms,
R⁵ and R⁶ each independently represent a hydrocarbon group having 1 to 6 carbon atoms, which may be substituted,
substituents on the hydrocarbon groups having 1 to 6 carbon atoms represented by R⁵ and R⁶, which may be substituted, each independently represent -OH, -O-R²⁶, -C(O)NR²⁷R²⁸, or -NR²⁹C(O)R³⁰,
R²⁶, R²⁷, R²⁸, R²⁹, and R³⁰ each independently represent a hydrogen atom or a hydrocarbon group having 1 to 12 carbon atoms, which may be substituted, and substituents on the hydrocarbon groups having 1 to 12 carbon atoms represented by R²⁶, R²⁷, R²⁸, R²⁹, and R³⁰, which may be substituted, each independently represent an aryl group having 6 to 10 carbon atoms, and
R⁷, R⁸, and R⁹ each independently represent -(CH₂)ₙ-, and n represents an integer of 2 to 8.

4. A compound or a salt thereof selected from the following compounds:
((2-(diethylamino)ethyl)azanediyl)bis(ethan-2,1-diyl)bis(dioctylcarbamate) 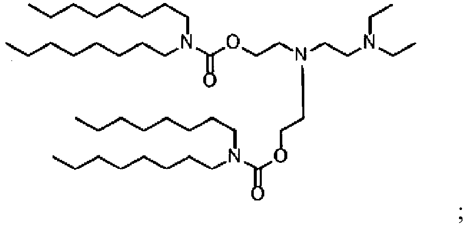
bis(2-hexyloctyl) 11-(2-(diethylamino)ethyl)-6,16-dioctyl-7,15-dioxo-8,14-dioxa-6,11,16-triazahenicosanedioate 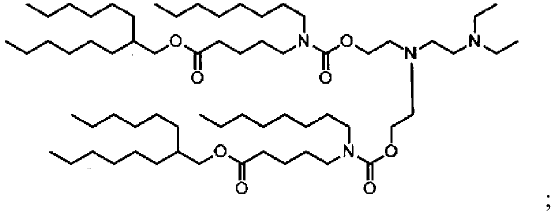
bis(2-pentylheptyl) 11-(2-(diethylamino)ethyl)-6,16-dioctyl-7,15-dioxo-8,14-dioxa-6,11,16-triazahenicosanedioate 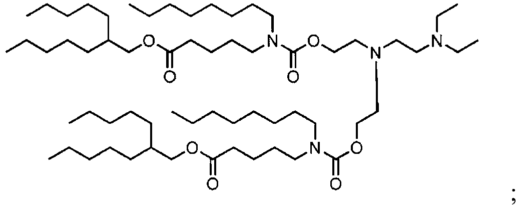
bis(2-pentylheptyl) 11-(2-(diethylamino)ethyl)-6,16-dihexyl-7,15-dioxo-8,14-dioxa-6,11,16-triazahenicosanedioate 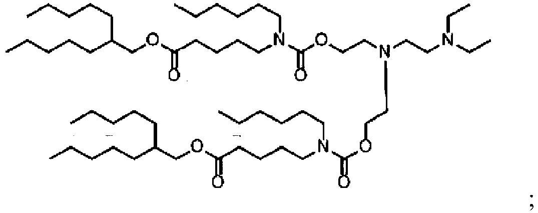
bis(2-hexyloctyl) 11-(2-(diethylamino)ethyl)-6,16-dihexyl-7,15-dioxo-8,14-dioxa-6,11,16-triazahenicosanedioate 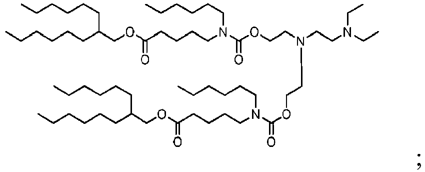
bis(2-pentylheptyl) 4,14-dibutyl-9-(2-(diethylamino)ethyl)-5,13-dioxo-6,12-dioxa-4,9,14-triazaheptadecanedioate 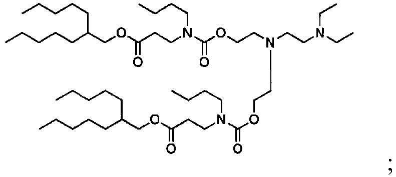
bis(2-pentylheptyl) 4,14-dibutyl-9-(3-(diethylamino)propyl)-5,13-dioxo-6,12-dioxa-4,9,14-triazaheptadecanedioate 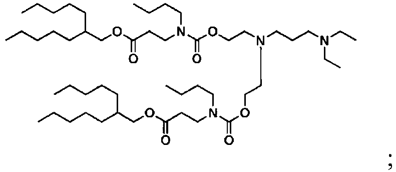
bis(2-pentylheptyl) 9-(2-(diethylamino)ethyl)-5,13-dioxo-4,14-dipropyl-6,12-dioxa-4,9,14-triazaheptadecanedioate 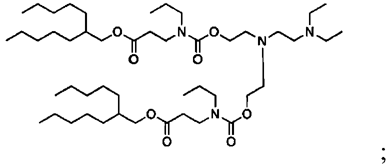
bis(2-pentylheptyl) 9-(3-(diethylamino)propyl)-5,13-dioxo-4,14-dipropyl-6,12-dioxa-4,9,14-triazaheptadecanedioate 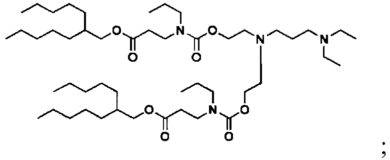
bis(2-pentylheptyl) 8-(2-(diethylamino)ethyl)-4,12-dioxo-3,13-dipropyl-5,11-dioxa-3,8,13-triazapentadecanedioate 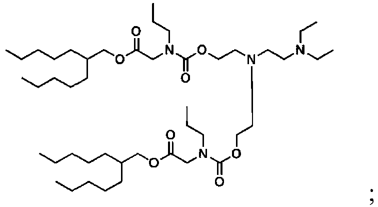
bis(2-pentylheptyl) 8-(3-(diethylamino)propyl)-4,12-dioxo-3,13-dipropyl-5,11-dioxa-3,8,13-triazapentadecanedioate 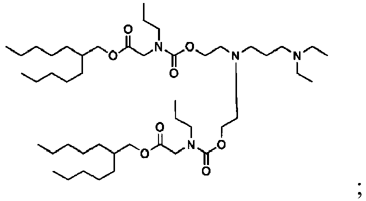
2-(2-(2-(bis(2-decanoyloxyethyl)carbamoyloxy)ethyl-(2-(diethylamino)ethyl)amino)ethoxycarbonyl-(2-decanoyloxyethyl)amino)ethyl decanoate 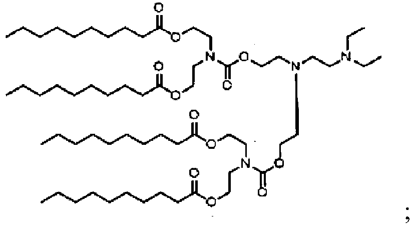
bis(2-pentylheptyl) 11-(2-(diethylamino)ethyl)-6,16-diisopropyl-7,15-dioxo-8,14-dioxa-6,11,16-triazahenicosanedioate 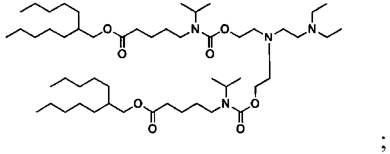
bis(2-pentylheptyl) 11-(3-(diethylamino)propyl)-6,16-diisopropyl-7,15-dioxo-8,14-dioxa-6,11,16-triazahenicosanedioate 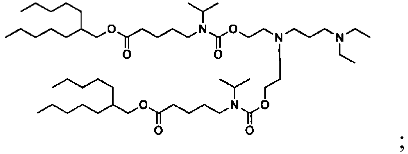
bis(2-pentylheptyl) 11-(2-(diethylamino)ethyl)-7,15-dioxo-6,16-dipropyl-8,14-dioxa-6,11,16-triazahenicosanedioate 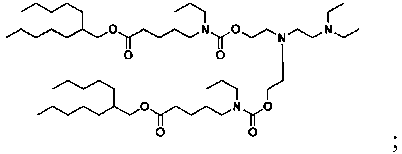
bis(2-pentylheptyl) 11-(3-(diethylamino)propyl)-7,15-dioxo-6,16-dipropyl-8,14-dioxa-6,11,16-triazahenicosanedioate 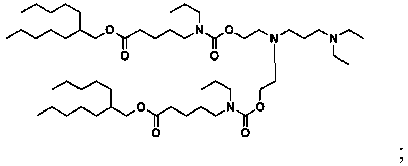
bis(2-butyloctyl) 11-(2-(diethylamino)ethyl)-6,16-dihexyl-7,15-dioxo-8,14-dioxa-6,11,16-triazahenicosanedioate 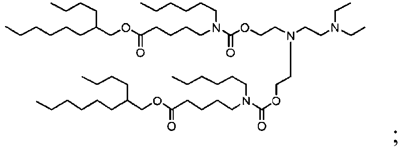
bis(2-pentylheptyl) 11-(2-(diethylamino)ethyl)-6,16-diisobutyl-7,15-dioxo-8,14-dioxa-6,11,16-triazahenicosanedioate 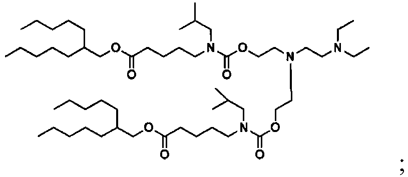
bis(2-pentylheptyl) 11-(3-(diethylamino)propyl)-6,16-diisobutyl-7,15-dioxo-8,14-dioxa-6,11,16-triazahenicosanedioate 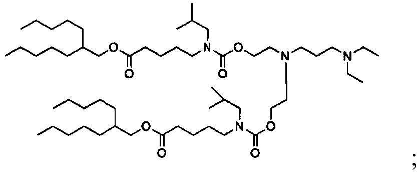
2-(2-(2-(bis(2-dodecanoyloxyethyl)carbamoyloxy)ethyl-(2-(diethylamino)ethyl)amino)ethoxycarbonyl-(2-dodecanoyloxyethyl)amino)ethyl dodecanoate 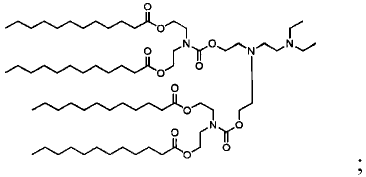
bis(2-pentylheptyl) 6,16-dibutyl-11-(2-(diethylamino)ethyl)-7,15-dioxo-8,14-dioxa-6,11,16-triazahenicosanedioate 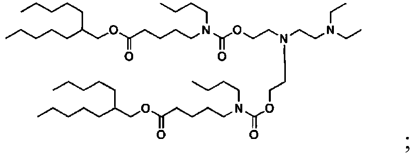
bis(2-pentylheptyl) 6,16-dibutyl-11-(3-(diethylamino)propyl)-7,15-dioxo-8,14-dioxa-6,11,16-triazahenicosanedioate 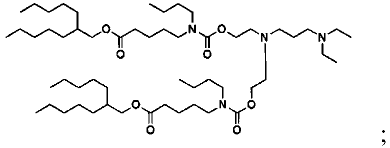
bis(2-hexyloctyl) 6,16-dibutyl-11-(2-(diethylamino)ethyl)-7,15-dioxo-8,14-dioxa-6,11,16-triazahenicosanedioate 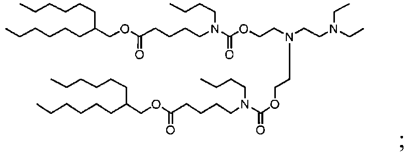
bis(2-hexyloctyl) 6,16-dibutyl-11-(3-(diethylamino)propyl)-7,15-dioxo-8,14-dioxa-6,11,16-triazahenicosanedioate 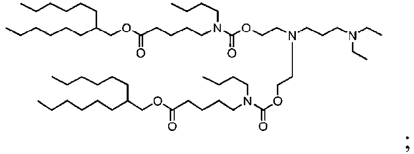
bis(2-pentylheptyl) 12-(2-(diethylamino)ethyl)-7,17-dihexyl-8,16-dioxo-9,15-dioxa-7,12,17-triazatricosanedioate 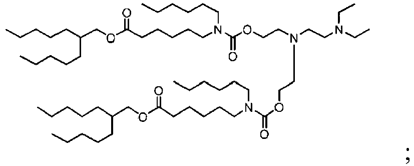
bis(2-pentylheptyl) 12-(3-(diethylamino)propyl)-7,17-dihexyl-8,16-dioxo-9,15-dioxa-7,12,17-triazatricosanedioate 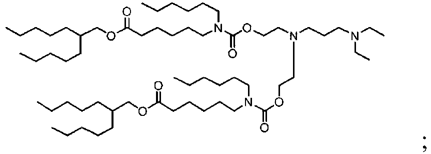
decyl 2-(2-(2-(bis(2-decoxy-2-oxo-ethyl)carbamoyloxy)ethyl-(2-(diethylamino)ethyl)amino)ethoxycarbonyl-(2-decoxy-2-oxo-ethyl)amino)acetate 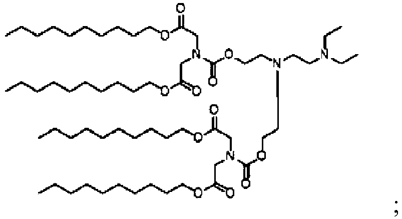
bis(2-pentylheptyl) 11-(2-(diethylamino)ethyl)-7,15-dioxo-6,16-dipentyl-8,14-dioxa-6,11,16-triazahenicosanedioate 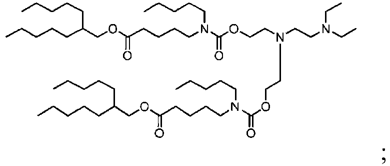
bis(2-pentylheptyl) 11-(3-(diethylamino)propyl)-7,15-dioxo-6,16-dipentyl-8,14-dioxa-6,11,16-triazahenicosanedioate 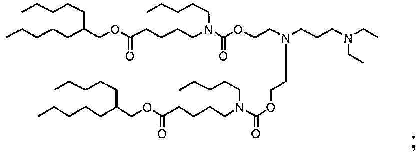
bis(2-pentylheptyl) 11-(2-(diethylamino)ethyl)-6,16-diheptyl-7,15-dioxo-8,14-dioxa-6,11,16-triazahenicosanedioate 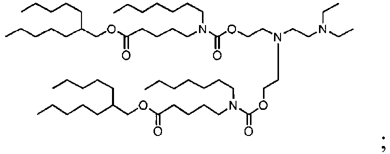
bis(2-pentylheptyl) 11-(3-(diethylamino)propyl)-6,16-diheptyl-7,15-dioxo-8,14-dioxa-6,11,16-triazahenicosanedioate 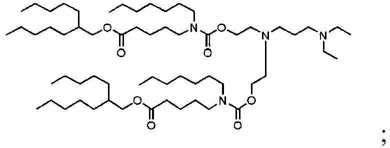
didodecyl 8-(2-(diethylamino)ethyl)-3,13-bis(2-(dodecyloxy)-2-oxoethyl)-4,12-dioxo-5,11-dioxa-3,8,13-triazapentadecanedioate 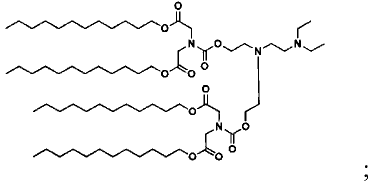
diundecyl 8-(2-(diethylamino)ethyl)-4,12-dioxo-3,13-bis(2-oxo-2-(undecyloxy)ethyl)-5,11-dioxa-3,8,13-triazapentadecanedioate 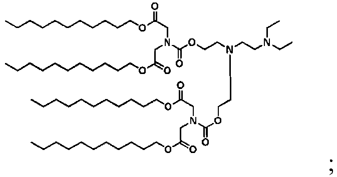
ditridecyl 8-(2-(diethylamino)ethyl)-4,12-dioxo-3,13-bis(2-oxo-2-(tridecyloxy)ethyl)-5,11-dioxa-3,8,13-triazapentadecanedioate 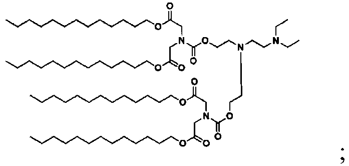
bis(2-pentylheptyl) 11-(3-(diethylamino)propyl)-6,16-dioctyl-7,15-dioxo-8,14-dioxa-6,11,16-triazahenicosanedioate 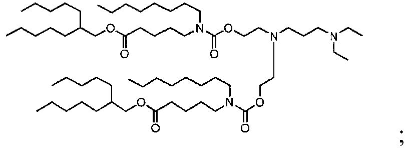
bis(2-pentylheptyl) 11-(4-(diethylamino)butyl)-6,16-dioctyl-7,15-dioxo-8,14-dioxa-6,11,16-triazahenicosanedioate 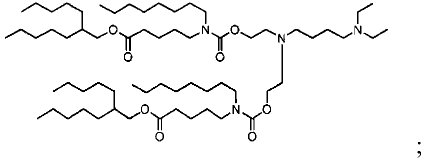
bis(2-hexyloctyl) 11-(3-(diethylamino)propyl)-6,16-dioctyl-7,15-dioxo-8,14-dioxa-6,11,16-triazahenicosanedioate 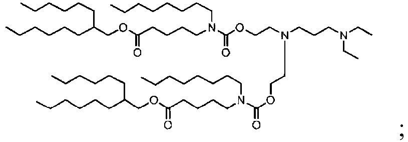
bis(2-hexyloctyl) 11-(4-(diethylamino)butyl)-6,16-dioctyl-7,15-dioxo-8,14-dioxa-6,11,16-triazahenicosanedioate 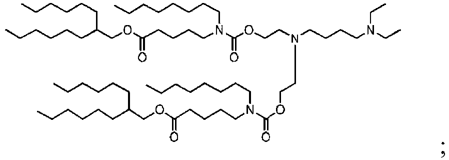
1-heptyl 21-(2-pentylheptyl) 11-(2-(diethylamino)ethyl)-6,16-dioctyl-7,15-dioxo-8,14-dioxa-6,11,16-triazahenicosanedioate 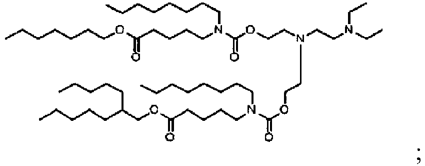
1-heptyl 21-(2-hexyloctyl) 11-(2-(diethylamino)ethyl)-6,16-dioctyl-7,15-dioxo-8,14-dioxa-6,11,16-triazahenicosanedioate 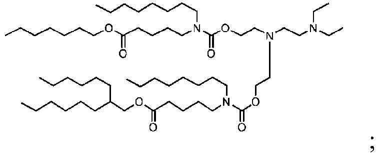
1-octyl 21-(2-pentylheptyl) 11-(2-(diethylamino)ethyl)-6,16-dioctyl-7,15-dioxo-8,14-dioxa-6,11,16-triazahenicosanedioate 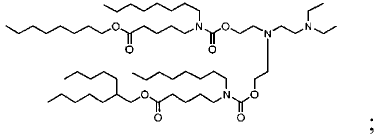
1-(2-hexyloctyl) 21-octyl 11-(2-(diethylamino)ethyl)-6,16-dioctyl-7,15-dioxo-8,14-dioxa-6,11,16-triazahenicosanedioate 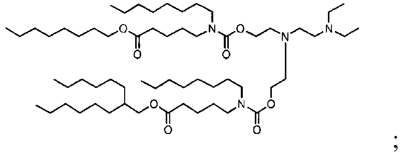
21-heptyl 1-(2-hexyloctyl) 6-decyl-11-(2-(diethylamino)ethyl)-16-octyl-7,15-dioxo-8,14-dioxa-6,11,16-triazahenicosanedioate 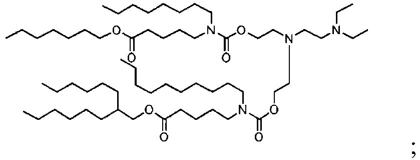
1-(2-hexyloctyl) 21-octyl 6-decyl-11-(2-(diethylamino)ethyl)-16-octyl-7,15-dioxo-8,14-dioxa-6,11,16-triazahenicosanedioate 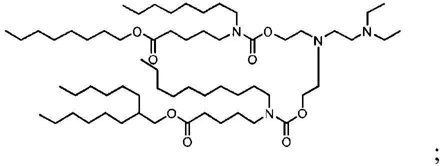
bis(2-pentylheptyl) 12-(2-(diethylamino)ethyl)-7,17-diheptyl-8,16-dioxo-9,15-dioxa-7,12,17-triazatricosanedioate 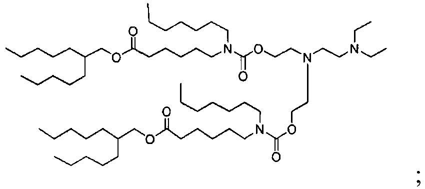
bis(2-pentylheptyl) 12-(2-(diethylamino)ethyl)-8,16-dioxo-7,17-dipentyl-9,15-dioxa-7,12,17-triazatricosanedioate 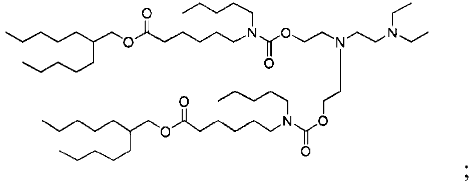
bis(2-hexyloctyl) 7,17-dibutyl-12-(2-(diethylamino)ethyl)-8,16-dioxo-9,15-dioxa-7,12,17-triazatricosanedioate 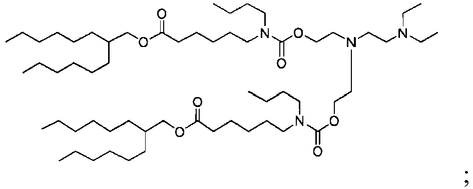
bis(2-pentylheptyl) 12-(3-(diethylamino)propyl)-7,17-diheptyl-8,16-dioxo-9,15-dioxa-7,12,17-triazatricosanedioate 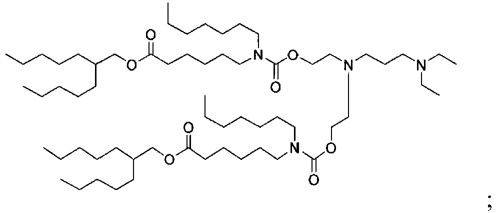
bis(2-pentylheptyl) 12-(3-(diethylamino)propyl)-8,16-dioxo-7,17-dipentyl-9,15-dioxa-7,12,17-triazatricosanedioate 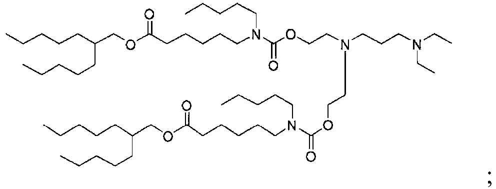
bis(2-pentylheptyl) 12-(2-(diethylamino)ethyl)-7,17-dioctyl-8,16-dioxo-9,15-dioxa-7,12,17-triazatricosanedioate 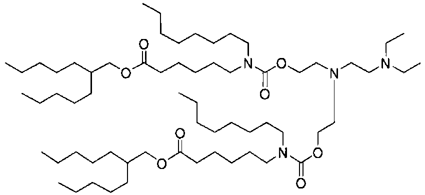
bis(2-pentylheptyl) 12-(3-(diethylamino)propyl)-7,17-dioctyl-8,16-dioxo-9,15-dioxa-7,12,17-triazatricosanedioate

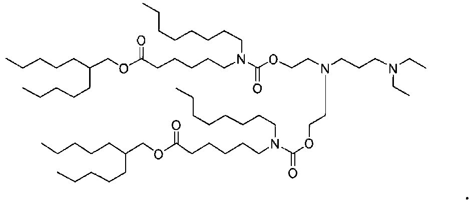

5. A lipid composition comprising:
the compound or a salt thereof according to any one of claims 1 to 4; and
a lipid.

6. The lipid composition according to claim 5,
wherein the lipid is at least one lipid selected from the group consisting of a neutral lipid and a lipid having a nonionic hydrophilic polymer chain.

7. The lipid composition according to claim 5 or 6, further comprising:
a sterol.

8. The lipid composition according to any one of claims 5 to 7, further comprising:
at least one selected from the group consisting of a nucleic acid, a protein, a peptide, and a low-molecular-weight compound.

9. A pharmaceutical composition comprising:
the lipid composition according to any one of claims 5 to 8.

10. A delivery carrier comprising:
the lipid composition according to any one of claims 5 to 8.
